# EUROPEAN PATENT APPLICATION

(11) **EP 2 009 011 A1**
(43) Date of publication of application: **31.12.2008**
(21) Application number: 07741635.2
(22) Date of filing: 13.04.2007
(51) Int. Cl.: C07D 409/06, A61K 31/4535, A61K 31/5377, A61P 3/04, A61P 3/06, A61P 3/10, A61P 9/04, A61P 9/12, A61P 21/00, A61P 43/00, C07D 409/14, C07D 413/14, C07D 417/14, C12N 9/99, C12N 15/09

(54) **NITROGEN-CONTAINING HETEROCYCLIC COMPOUND**

(30) Priority: 14.04.2006 JP 2006112769
(71) Applicant: Takeda Pharmaceutical Company Limited, Osaka-shi, Osaka 541-0045 (JP)
(72) Inventor: FUKATSU, Kohji, Osaka-shi, Osaka 532-8686 (JP); KAMATA, Makoto, Osaka-shi, Osaka 532-8686 (JP); YAMASHITA, Tohru, Osaka-shi, Osaka 532-8686 (JP); ENDO, Satoshi, Osaka-shi, Osaka 532-8686 (JP)
(74) Representative: Dossmann, Gérard
(86) International application number: PCT/JP2007/058199
(87) International publication number: WO 2007/119833

(57) **Abstract**

The object of the present invention is to provide a compound having an ACC inhibitory action, which is useful for the prophylaxis or treatment of obesity, diabetes, hypertension, hyperlipidemia, cardiac failure, diabetic complications, metabolic syndrome, sarcopenia and the like, and has superior properties such as efficacy, duration of activity, specificity, low toxicity and the like.

The present invention provides a compound represented by the following formula 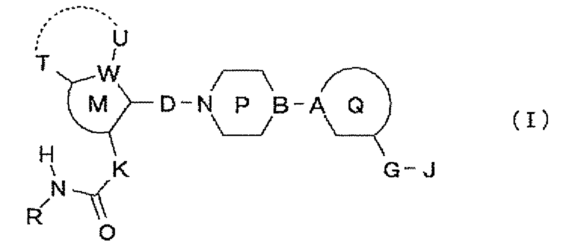 wherein
ring M is a 5- or 6-membered aromatic ring;
W is C or N;
K is an optionally substituted methylene group or an optionally substituted imino group;
R is a hydrogen atom, an optionally substituted hydrocarbon group, an optionally substituted hydroxy group or an optionally substituted heterocyclic group;
T and U are independently a hydrogen atom or a substituent or, T and U form, together with ring M, an optionally substituted bicyclic ring;
D and G are independently a carbonyl group or a sulfonyl group; ring P is an optionally substituted piperidine or an optionally substituted piperazine;
B is CH or N;
ring Q is an optionally substituted monocyclic ring;
A is C, CH or N; and
J is an optionally substituted hydrocarbon group, an optionally substituted heterocyclic group or an optionally substituted amino group,
provided that when the W moiety of ring M is =N- or -N=, then U should be absent,
or a salt thereof.

## Description

### Technical Field

The present invention relates to a nitrogen-containing heterocycle compound having an acetyl-CoA carboxylase (sometimes to be abbreviated as ACC in the present specification) inhibitory action, which is useful for the prophylaxis or treatment of obesity, diabetes, hypertension, hyperlipidemia, cardiac failure, diabetic complications, metabolic syndrome, sarcopenia and the like.

### Background Art

ACC is an enzyme that converts acetyl-CoA to malonyl-CoA, and catalyzes a rate determining reaction in fatty acid metabolism. Malonyl-CoA, which is produced by an ACC catalyst reaction, inhibits fatty acid oxidation in mitochondria based on the feedback inhibition of carnitine palmitoyl transferase-1 (CPT-1). Accordingly, ACC plays a key role in controlling the balance between use of carbohydrate and fatty acid in the liver and skeletal muscle, and further, controlling insulin sensitivity in the liver, skeletal muscle and adipose tissue.

A reduced level of malonyl-CoA by ACC inhibition can promote an increase in fatty acid utilization, decreased secretion of triglyceride (TG)-rich lipoprotein (VLDL) in the liver, regulation of insulin secretion in the pancreas, and further, improvement in the insulin sensitivity in the liver, skeletal muscle and adipose tissue.

In addition, chronic administration of a compound having an ACC inhibitory action can strikingly decrease the TG content of the liver and adipose tissues and selectively decrease body fat in obese test subjects taking low fat diet, by promoting fatty acid utilization and suppressing de novo synthesis of fatty acid.

Accordingly, a compound having an ACC inhibitory action is extremely useful for the prophylaxis or treatment of metabolic syndrome, obesity, hypertension, diabetes, cardiovascular diseases associated with atherosclerosis and the like.

As a nitrogen-containing heterocycle compound, the following compound has been reported.
A compound represented by the formula:

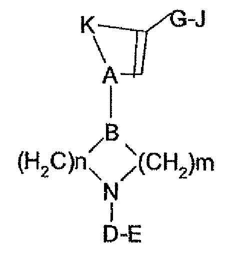

wherein
A-B is N-CH or CH-N; K is (CH₂)ᵣ (r is an integer of 2 to 4); m and n are each an integer of 1 to 3; D is CO or SO₂; E is an optionally substituted bi- to tetra-cyclic ring and the like; G is CO, SO₂ or CR⁷R⁸ (R⁷ and R⁸ are each a hydrogen atom and the like); and J is OR¹, NR²R³, CR⁴R⁵R⁶ (R¹, R², R³, R⁴, R⁵ and R⁶ are each a hydrogen atom and the like),
which has an ACC inhibitory action and is useful as a therapeutic agent for metabolic syndrome, arteriosclerosis, diabetes, obesity and the like (see patent reference 1).
Patent reference 1: WO 03/072197

However, the compound of the present invention is not reported.

### Disclosure of the Invention

### Problems to be Solved by the Invention

There is a demand for the development of a compound having an ACC inhibitory action, which is useful for the prophylaxis or treatment of obesity, diabetes, hypertension, hyperlipidemia, cardiac failure, diabetic complications, metabolic syndrome, sarcopenia and the like, and has superior properties such as efficacy, duration of activity, specificity, low toxicity and the like.

### Means of Solving the Problems

The present inventors have found that a compound represented by the following formula

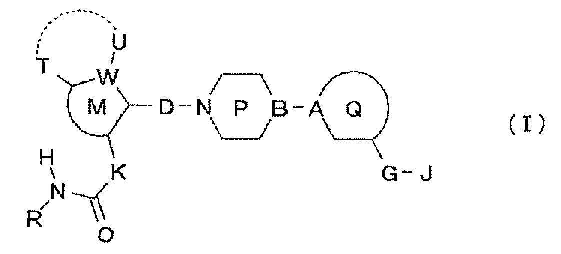

wherein
ring M is a 5- or 6-membered aromatic ring;
W is C or N;
K is an optionally substituted methylene group or an optionally substituted imino group;
R is a hydrogen atom, an optionally substituted hydrocarbon group, an optionally substituted hydroxy group or an optionally substituted heterocyclic group;
T and U are independently a hydrogen atom or a substituent or, T and U form, together with ring M, an optionally substituted bicyclic ring;
D and G are independently a carbonyl group or a sulfonyl group; ring P is an optionally substituted piperidine or an optionally substituted piperazine;
B is CH or N;
ring Q is an optionally substituted monocyclic ring;
A is C, CH or N; and
J is an optionally substituted hydrocarbon group, an optionally substituted heterocyclic group or an optionally substituted amino group,
provided that when the W moiety of ring M is =N- or -N=, then U should be absent,
or a salt thereof [hereinafter sometimes referred to as compound (I)], has a superior ACC inhibitory action and is useful for the prophylaxis or treatment of obesity, diabetes, hypertension, hyperlipidemia, cardiac failure, diabetic complications, metabolic syndrome, sarcopenia and the like.
Based on this finding, the present inventors have conducted intensive studies and completed the present invention. Accordingly, the present invention relates to
[1] the above-mentioned compound (I);
[2] the above-mentioned compound (I) wherein ring M is thiophene, thiazole or pyridine;
[3] the above-mentioned compound (I) wherein K is an imino group;
[4] the above-mentioned compound (I) wherein R is a hydrogen atom or an optionally substituted hydrocarbon group;
[5] the above-mentioned compound (I) wherein T is an optionally substituted phenyl group, and U is a hydrogen atom;
[6] the above-mentioned compound (I) wherein T and U form, together with ring M, an optionally substituted bicyclic ring;
[7] the above-mentioned compound (I) wherein D is a carbonyl group;
[8] the above-mentioned compound (I) wherein G is a carbonyl group;
[9] the above-mentioned compound (I) wherein ring P is piperidine;
[10] the above-mentioned compound (I) wherein ring Q is piperidine, piperazine, morpholine or benzene, each of which is optionally substituted;
[11] the above-mentioned compound (I) wherein A is C or N;
[12] the above-mentioned compound (I) wherein J is an optionally substituted heterocyclic group or an optionally substituted amino group;
[13] the above-mentioned compound (I) which is N,N-diethyl-1'-[(2-{[(ethylamino)carbonyl]amino}-1-benzothiophen-3-yl)carbonyl]-1,4'-bipiperidine-3-carboxamide; N-ethyl-N'-(4-{[3-(morpholin-4-ylcarbonyl)-1,4'-bipiperidin-1'-yl]carbonyl}-2-phenyl-1,3-thiazol-5-yl)urea; N-ethyl-N'-{3-{[(3R)-3-(morpholin-4-ylcarbonyl)-1,4'-bipiperidin-1'-yl]carbonyl)-5-[4-(trifluoromethyl)phenyl]-2-thienyl}urea;
   N-ethyl-N'-(7-methoxy-3-{[3-(piperidin-1-ylcarbonyl)-1,4'-bipiperidin-1'-yl]carbonyl}-1-benzothien-2-yl)urea; or
   N-ethyl-1'-[(2-{[(ethylamino)carbonyl]amino}-6-methylthieno[2,3-b]pyridin-3-yl)carbonyl]-N-isopropyl-1,4'-bipiperidine-3-carboxamide;
   or a salt thereof;
[14] a prodrug of the above-mentioned compound (I);
[15] an acetyl-CoA carboxylase inhibitor comprising the above-mentioned compound (I) or a prodrug thereof;
[16] a pharmaceutical agent comprising the above-mentioned compound (I) or a prodrug thereof;
[17] the pharmaceutical agent of the above-mentioned [16], which is an agent for the prophylaxis or treatment of obesity, diabetes, hypertension, hyperlipidemia, cardiac failure, diabetic complications, metabolic syndrome or sarcopenia;
[18] use of the above-mentioned compound (I) or a prodrug thereof for the production of an acetyl-CoA carboxylase inhibitor;
[19] use of the above-mentioned compound (I) or a prodrug thereof for the production of an agent for the prophylaxis or treatment of obesity, diabetes, hypertension, hyperlipidemia, cardiac failure, diabetic complications, metabolic syndrome or sarcopenia;
[20] a method of inhibiting acetyl-CoA carboxylase in a mammal, which comprises administering the above-mentioned compound (I) or a prodrug thereof to the mammal;
[21] a method for the prophylaxis or treatment of obesity, diabetes, hypertension, hyperlipidemia, cardiac failure, diabetic complications, metabolic syndrome or sarcopenia in a mammal, which comprises administering the above-mentioned compound (I) or a prodrug thereof to the mammal;
   and the like.

The definition of each symbol in the formula (I) is described in detail in the following.
The "halogen atom" in the present specification means, unless otherwise specified, fluorine, chlorine, bromine or iodine.
Examples of the "hydrocarbon group" of the "optionally substituted hydrocarbon group" for J include a C₁₋₁₀ alkyl group, a C₂₋₁₀ alkenyl group, a C₂₋₁₀ alkynyl group, a C₃₋₁₀ cycloalkyl group, a C₃₋₁₀ cycloalkenyl group, a C₄₋₁₀ cycloalkadienyl group, a C₆₋₁₄ aromatic hydrocarbon group, a C₇₋₁₃ aralkyl group, a C₈₋₁₃ aromatic hydrocarbon-alkenyl group, a C₃₋₁₀ cycloalkyl-C₁₋₆ alkyl group and the like.

Examples of the C₁₋₁₀ alkyl group include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, heptyl, octyl, nonyl, decyl and the like.
Examples of the C₂₋₁₀ alkenyl group include ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, 5-hexenyl, 1-heptenyl, 1-octenyl and the like.
Examples of the C₂₋₁₀ alkynyl group include ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, 1-heptynyl, 1-octynyl and the like.

Examples of the C₃₋₁₀ cycloalkyl group include cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl, bicyclo[3.2.2]nonyl, bicyclo[3.3.1]nonyl, bicyclo[4.2.1]nonyl, bicyclo[4.3.1]decyl, adamantyl and the like.
Examples of the C₃₋₁₀ cycloalkenyl group include 2-cyclopenten-1-yl, 3-cyclopenten-1-yl, 2-cyclohexen-1-yl, 3-cyclohexen-1-yl and the like.
Examples of the C₄₋₁₀ cycloalkadienyl group include 2,4-cyclopentadien-1-yl, 2,4-cyclohexadien-1-yl, 2,5-cyclohexadien-1-yl and the like.
Examples of the C₆₋₁₄ aromatic hydrocarbon group include phenyl, naphthyl, anthryl, phenanthryl, acenaphthyl, biphenylyl and the like. Of these, phenyl, 1-naphthyl, 2-naphthyl and the like are preferable.
Examples of the C₇₋₁₃ aralkyl group include benzyl, phenethyl, naphthylmethyl, biphenylylmethyl and the like.
Examples of the C₈₋₁₃ aromatic hydrocarbon-alkenyl group include styryl and the like.
Examples of the C₃₋₁₀ cycloalkyl-C₁₋₆ alkyl group include cyclohexylmethyl and the like.

The above-mentioned C₁₋₁₀ alkyl group, C₂₋₁₀ alkenyl group and C₂₋₁₀ alkynyl group optionally have 1 to 3 substituents at substitutable positions.
Examples of the substituent include
(1) a C₃₋₁₀ cycloalkyl group (e.g., cyclopropyl, cyclohexyl);
(2) a C₆₋₁₄ aromatic hydrocarbon group (e.g., phenyl, naphthyl) optionally substituted by 1 to 3 substituents selected from a C₁₋₆ alkyl group (e.g., methyl, ethyl) optionally substituted by 1 to 3 halogen atoms, a hydroxy group, a C₁₋₆ alkoxy group (e.g., methoxy, ethoxy), a carboxyl group and a halogen atom;
(3) an aromatic heterocyclic group (e.g., thienyl, furyl, pyridyl, oxazolyl, thiazolyl, tetrazolyl, oxadiazolyl, pyrazinyl, quinolyl, indolyl) optionally substituted by 1 to 3 substituents selected from a C₁₋₆ alkyl group (e.g., methyl, ethyl) optionally substituted by 1 to 3 halogen atoms, a hydroxy group, a C₁₋₆ alkoxy group (e.g., methoxy, ethoxy), a carboxyl group and a halogen atom;
(4) a non-aromatic heterocyclic group (e.g., tetrahydrofuryl, morpholinyl, thiomorpholinyl, piperidinyl, pyrrolidinyl, piperazinyl, dioxolyl, dioxolanyl, 1,3-dihydro-2-benzofuranyl, thiazolidinyl) optionally substituted by 1 to 3 substituents selected from a C₁₋₆ alkyl group (e.g., methyl, ethyl) optionally substituted by 1 to 3 halogen atoms, a hydroxy group, a C₁₋₆ alkoxy group (e.g., methoxy, ethoxy), an oxo group, a carboxyl group and a halogen atom;
(5) an amino group optionally mono- or di-substituted by substituent(s) selected from a C₁₋₆ alkyl group (e.g., methyl, ethyl), a C₁₋₆ alkyl-carbonyl group (e.g., acetyl, isobutanoyl, isopentanoyl), a C₁₋₆ alkoxy-carbonyl group (e.g., methoxycarbonyl, ethoxycarbonyl, propoxycarbonyl, tert-butoxycarbonyl) and a carbamoyl group optionally mono- or di-substituted by C₁₋₆ alkyl group(s) (e.g., methylcarbamoyl, ethylcarbamoyl);
(6) a C₁₋₆ alkylsulfonylamino group (e.g., methylsulfonylamino);
(7) an amidino group;
(8) a C₁₋₆ alkyl-carbonyl group (e.g., acetyl, isobutanoyl, isopentanoyl) optionally substituted by 1 to 3 halogen atoms;
(9) a C₁₋₆ alkoxy-carbonyl group (e.g., methoxycarbonyl, ethoxycarbonyl, propoxycarbonyl, tert-butoxycarbonyl) optionally substituted by 1 to 3 halogen atoms;
(10) a C₁₋₆ alkylsulfonyl group (e.g., methylsulfonyl) optionally substituted by 1 to 3 halogen atoms;
(11) a carbamoyl group optionally mono- or di-substituted by C₁₋₆ alkyl group(s) (e.g., methyl, ethyl) optionally substituted by 1 to 3 halogen atoms;
(12) a thiocarbamoyl group optionally mono- or di-substituted by C₁₋₆ alkyl group(s) (e.g., methyl, ethyl) optionally substituted by 1 to 3 halogen atoms;
(13) a sulfamoyl group optionally mono- or di-substituted by C₁₋₆ alkyl group(s) (e.g., methyl, ethyl) optionally substituted by 1 to 3 halogen atoms;
(14) a carboxyl group;
(15) a hydroxy group;
(16) a C₁₋₆ alkoxy group (e.g., methoxy, ethoxy) optionally substituted by 1 to 3 halogen atoms;
(17) a C₂₋₆ alkenyloxy group (e.g., ethenyloxy) optionally substituted by 1 to 3 halogen atoms;
(18) a C₃₋₁₀ cycloalkyloxy group (e.g., cyclohexyloxy);
(19) a C₇₋₁₃ aralkyloxy group (e.g., benzyloxy);
(20) a C₆₋₁₄ aromatic hydrocarbon-oxy group (e.g., phenyloxy, naphthyloxy);
(21) a C₁₋₆ alkyl-carbonyloxy group (e.g., acetyloxy, tert-butylcarbonyloxy);
(22) a thiol group;
(23) a C₁₋₆ alkylthio group (e.g., methylthio, ethylthio) optionally substituted by 1 to 3 halogen atoms;
(24) a C₇₋₁₃ aralkylthio group (e.g., benzylthio);
(25) a C₆₋₁₄ aromatic hydrocarbon-thio group (e.g., phenylthio, naphthylthio);
(26) a sulfo group;
(27) a cyano group;
(28) an azido group;
(29) a nitro group;
(30) a nitroso group;
(31) a halogen atom;
(32) a C₁₋₆ alkylsulfinyl group (e.g., methylsulfinyl);
(33) an oxo group; and the like.

The C₃₋₁₀ cycloalkyl group, C₃₋₁₀ cycloalkenyl group, C₄₋₁₀ cycloalkadienyl group, C₆₋₁₄ aromatic hydrocarbon group, C₇₋₁₃ aralkyl group, C₈₋₁₃ aromatic hydrocarbon-alkenyl group and C₃₋₁₀ cycloalkyl-C₁₋₆ alkyl group which are exemplified as the above-mentioned "hydrocarbon group" optionally have 1 to 3 substituents at substitutable positions.
Examples of the substituent include
(1) the groups exemplified as the substituents for the above-mentioned C₁₋₁₀ alkyl group and the like;
(2) a C₁₋₆ alkyl group (e.g., methyl, ethyl) optionally substituted by 1 to 3 substituents selected from a halogen atom, a carboxyl group, a C₁₋₆ alkoxy-carbonyl group (e.g., methoxycarbonyl, ethoxycarbonyl) and a carbamoyl group;
(3) a C₂₋₆ alkenyl group (e.g., ethenyl, 1-propenyl) optionally substituted by 1 to 3 substituents selected from a halogen atom, a carboxyl group, a C₁₋₆ alkoxy-carbonyl group (e.g., methoxycarbonyl, ethoxycarbonyl) and a carbamoyl group;
(4) a C₇₋₁₃ aralkyl group (e.g., benzyl) optionally substituted by 1 to 3 substituents selected from a C₁₋₆ alkyl group optionally substituted by 1 to 3 halogen atoms, a hydroxy group, a C₁₋₆ alkoxy group and a halogen atom; and the like.

Examples of the heterocyclic group" of the "optionally substituted heterocyclic group" for J include an "aromatic heterocyclic group" and a "non-aromatic heterocyclic group",
Examples of the aromatic heterocyclic group include a 5-to 7-membered monocyclic aromatic heterocyclic group containing, as a ring-constituting atom besides carbon atoms, 1 to 4 hetero atoms selected from an oxygen atom, a sulfur atom and a nitrogen atom, and a fused aromatic heterocyclic group. Examples of the fused aromatic heterocyclic group include a group drived from a fused ring wherein the 5- to 7-membered monocyclic aromatic heterocyclic group is condensed with 1 or 2 rings selected from a 5- or 6-membered aromatic heterocycle containing 1 or 2 nitrogen atoms (e.g., pyrrole, imidazole, pyrazole, pyrazine, pyridine, pyrimidine), a 5-membered aromatic heterocycle containing one sulfur atom (e.g., thiophene) and a benzene ring, and the like.
Preferable examples of the aromatic heterocyclic group include
monocyclic aromatic heterocyclic groups such as furyl (e.g., 2-furyl, 3-furyl), thienyl (e.g., 2-thienyl, 3-thienyl), pyridyl (e.g., 2-pyridyl, 3-pyridyl, 4-pyridyl), pyrimidinyl (e.g., 2-pyrimidinyl, 4-pyrimidinyl, 5-pyrimidinyl, 6-pyrimidinyl), pyridazinyl (e.g., 3-pyridazinyl, 4-pyridazinyl), pyrazinyl (e.g., 2-pyrazinyl), pyrrolyl (e.g., 1-pyrrolyl, 2-pyrrolyl, 3-pyrrolyl), imidazolyl (e.g., 1-imidazolyl, 2-imidazolyl, 4-imidazolyl, 5-imidazolyl), pyrazolyl (e.g., 1-pyrazolyl, 3-pyrazolyl, 4-pyrazolyl), thiazolyl (e.g., 2-thiazolyl, 4-thiazolyl, 5-thiazolyl), isothiazolyl (e.g., 4-isothiazolyl), oxazolyl (e.g., 2-oxazolyl, 4-oxazolyl, 5-oxazolyl), isoxazolyl, oxadiazolyl (e.g., 1,2,4-oxadiazol-5-yl, 1,3,4-oxadiazol-2-yl), thiadiazolyl (e.g., 1,3,4-thiadiazol-2-yl), triazolyl (e.g., 1,2,4-triazol-1-yl, 1,2,4-txiazol-3-yl, 1,2,3-triazol-1-yl, 1,2,3-triazol-2-yl, 1,2,3-triazol-4-yl), tetrazolyl (e.g., tetrazol-1-yl, tetrazol-5-yl), triazinyl (e.g., 1,2,4-triazol-1-yl, 1,2,4-triazol-3-yl) and the like;
fused aromatic heterocyclic groups such as quinolyl (e.g., 2-quinolyl, 3-quinolyl, 4-quinolyl, 6-quinolyl), isoquinolyl (e.g., 3-isoquinolyl), quinazolyl (e.g., 2-quinazolyl, 4-quinazolyl), quinoxalyl (e.g., 2-quinoxalyl, 6-quinoxalyl), benzofuryl (e.g., 2-benzofuryl, 3-benzofuryl), benzothienyl (e.g., 2-benzothienyl, 3-benzothienyl), benzoxazolyl (e.g., 2-benzoxazolyl), benzisoxazolyl (e.g., 7-benzisoxazolyl), benzothiazolyl (e.g., 2-benzothiazolyl), benzimidazolyl (e.g., benzimidazol-1-yl, benzimidazol-2-yl, benzimidazol-5-yl), benzotriazolyl (e.g., 1H-1,2,3-benzotriazol-5-yl), indolyl (e.g., indol-1-yl, indol-2-yl, indol-3-yl, indol-5-yl), indazolyl (e.g., 1H-indazol-3-yl), pyrrolopyrazinyl (e.g., 1H-pyrrolo[2,3-b]pyrazin-2-yl, 1H-pyrrolo[2,5-b]pyrazin-5-yl), imidazopyridinyl (e.g., 1H-imidazo[4,5-b]pyridin-2-yl, 1H-imidazo[4,5-c]pyridin-2-yl, 2H-imidazo[1,2-a]pyridin-3-yl), imidazopyrazinyl (e.g., 1H-imidazo[4,5-b]pyrazin-2-yl), pyrazolopyridinyl (e.g., 1H-pyrazolo[4,3-c]pyridin-3-yl), pyrazolothienyl (e.g., 2H-pyrazolo[3,4-b]thiophen-2-yl), pyrazolotriazinyl (e.g., pyrazolo[5,1-c][1,2,4]triazin-3-yl), thienopyridinyl (e.g., thieno[2,3-b]pyridin-3-yl) and the like; and the like.

Examples of the above-mentioned non-aromatic heterocyclic group include a 5- to 7-membered monocyclic non-aromatic heterocyclic group containing, as a ring-constituting atom besides carbon atoms, 1 to 4 hetero atoms selected from an oxygen atom, a sulfur atom and a nitrogen atom, and a fused non-aromatic heterocyclic group. Examples of the fused non-aromatic heterocyclic group include a group drived from a fused ring wherein the 5- to 7-membered monocyclic non-aromatic heterocyclic group is condensed with 1 or 2 rings selected from a 5- or 6-membered aromatic heterocycle containing 1 or 2 nitrogen atoms (e.g., pyrrole, imidazole, pyrazole, pyrazine, pyridine, pyrimidine), a 5-membered aromatic heterocycle containing one sulfur atom (e.g., thiophene) and a benzene ring, a group wherein the above-mentioned group is partially saturated, and the like.
Preferable examples of the non-aromatic heterocyclic group include
monocyclic non-aromatic heterocyclic groups such as pyrrolidinyl (e.g., 1-pyrrolidinyl), piperidinyl (e.g., piperidino), morpholinyl (e.g., morpholino), thiomorpholinyl (e.g., thiomorpholino), piperazinyl (e.g., 1-piperazinyl), hexamethyleniminyl (e.g., hexamethylenimin-1-yl), oxazolidinyl (e.g., oxazolidin-3-yl), thiazolidinyl (e.g., thiazolidin-3-yl), imidazolidinyl (e.g., imidazolidin-3-yl), dioxolyl (e.g., 1,3-dioxol-4-yl), dioxolanyl (e.g., 1,3-dioxolan-4-yl), dihydrooxadiazolyl (e.g., 4,5-dihydro-1,2,4-oxadiazol-3-yl), 2-thioxo-1,3-oxazolidin-5-yl, tetrahydropyranyl (e.g., 4-tetrahydropyranyl) and the like;
fused non-aromatic heterocyclic groups such as dihydroisoindolyl (e.g., 1,3-dihydro-2H-isoindol-2-yl), 4,5,6,7-tetrahydro-1-benzofuranyl (e.g., 4,5,6,7-tetrahydro-1-benzofuran-3-yl), 4,5,6,7-tetrahydro-1-benzothienyl (e.g., 4,5,6,7-tetrahydro-1-benzothiophen-3-yl), indanyl (e.g., indan-5-yl), chromenyl (e.g., 4H-chromen-2-yl, 2H-chromen-3-yl), dihydroisoquinolinyl (e.g., 1,2-dihydroisoquinolin-4-yl), tetrahydroisoquinolinyl (e.g., 1,2,3,4-tetrahydroisoquinolin-4-yl), dihydrophthalazinyl (e.g., 1,4-dihydrophthalazin-4-yl), pyrazolidinyl (e.g., pyrazolidin-1-yl), tetrahydroquinolinyl (e.g., 1,2,3,4-tetrahydroquinolin-4-yl) and the like;
and the like.
The "heterocyclic group" of the "optionally substituted heterocyclic group" for J optionally has 1 to 3 substituents at substitutable positions. Examples of the substituent include those exemplified as the substituents which the C₃₋₁₀ cycloalkyl group exemplified as the "hydrocarbon group" of the above-mentioned "optionally substituted hydrocarbon group" for J optionally has.
The "optionally substituted heterocyclic group" for J is preferably a nitrogen-containing non-aromatic heterocyclic group (e.g., pyrrolidinyl, imidazolidinyl, pyrazolidinyl, piperidinyl, piperazinyl, morpholinyl, thiomorpholinyl, tetrahydroquinolinyl, tetrahydroisoquinolinyl) optionally substituted by 1 or 2 substituents selected from a C₁₋₆ alkyl group and a C₃₋₁₀ cycloalkyl group, each of which is optionally substituted by 1 to 3 halogen atoms, or the like.

Examples of the "optionally substituted amino group" for J include an amino group optionally substituted by 1 or 2 substituents selected from a C₁₋₁₀ alkyl group, a C₂₋₁₀ alkenyl group, a C₃₋₁₀ cycloalkyl group, a C₃₋₁₀ cycloalkenyl group, a C₆₋₁₄ aromatic hydrocarbon group, a C₇₋₁₃ aralkyl group, a C₈₋₁₃ aromatic hydrocarbon-alkenyl group and the like, each of which is optionally substituted.
Examples of the C₁₋₁₀ alkyl group, C₂₋₁₀ alkenyl group, C₃₋₁₀ cycloalkyl group, C₃₋₁₀ cycloalkenyl group, C₆₋₁₄ aromatic hydrocarbon group, C₇₋₁₃ aralkyl group and C₈₋₁₃ aromatic hydrocarbon-alkenyl group include those exemplified as the "hydrocarbon group" of the above-mentioned "optionally substituted hydrocarbon group" for J.

The C₁₋₁₀ alkyl group, C₂₋₁₀ alkenyl group, C₃₋₁₀ cycloalkyl group, C₃₋₁₀ cycloalkenyl group, C₆₋₁₄ aromatic hydrocarbon group, C₇₋₁₃ aralkyl group and C₈₋₁₃ aromatic hydrocarbon-alkenyl group optionally have 1 to 3 substituents at substitutable positions. Examples of the substituent include
a halogen atom;
a C₁₋₆ alkoxy-carbonyl group (e.g., methoxycarbonyl, ethoxycarbonyl, tert-butoxycarbonyl);
a C₁₋₆ alkyl-carbonyl group;
a cyano group;
a carbamoyl group optionally mono- or di-substituted by C₁₋₁₀ alkyl group(s) (e.g., methyl, ethyl, propyl, isopropyl,
neopentyl);
a hydroxy group;
a carboxyl group;
and the like.

The "optionally substituted amino group" for J is preferably an amino group optionally mono- or di-substituted by substituent(s) selected from a C₁₋₆ alkyl group (e.g., methyl, ethyl, propyl, isopropyl) and a C₃₋₁₀ cycloalkyl group (e.g., cyclopentyl, cyclohexyl, adamantyl), each of which is optionally substituted by 1 to 3 halogen atoms, or the like.

J is preferably an "optionally substituted amino group" or an "optionally substituted heterocyclic group", more preferably
1) an amino group optionally mono- or di-substituted by substituent(s) selected from a C₁₋₆ alkyl group and a C₃₋₁₀ cycloalkyl group, each of which is optionally substituted by 1 to 3 halogen atoms;
2) a nitrogen-containing non-aromatic heterocyclic group (e.g., pyrrolidinyl, imidazolidinyl, pyrazolidinyl, piperidinyl, piperazinyl, morpholinyl, thiomorpholinyl, tetrahydroquinolinyl, tetrahydroisoquinolinyl) optionally substituted by 1 or 2 substituents selected from a C₁₋₆ alkyl group and a C₃₋₁₀ cycloalkyl group, each of which is optionally substituted by 1 to 3 halogen atoms,
   or the like.

Examples of the "5- or 6-membered aromatic ring" for ring M include a 5- or 6-membered aromatic hydrocarbon, a 5- or 6-membered aromatic heterocycle and the like. The 5- or 6-membered aromatic hydrocarbon is preferably a 5- or 6-membered ring (e.g., a benzene ring), from among C₆₋₁₄ aromatic hydrocarbons corresponding to the C₆₋₁₄ aromatic hydrocarbon group exemplified as the "hydrocarbon group" of the "optionally substituted hydrocarbon group" for J, or the like. Examples of the 5- or 6-membered aromatic heterocycle include a 5- or 6-membered ring, from among aromatic heterocycles corresponding to the "aromatic heterocyclic group"exemplified as the "heterocyclic group" of the "optionally substituted heterocyclic group" for J. Of these, furan, thiophene, thiazole, oxazole, imidazole, triazole, pyrazole, pyrimidine, pyridine and the like are preferable, and thiophene, thiazole, pyridine and the like are more preferable, and thiophene is most preferable.

Examples of the "substituent" which the "optionally substituted methylene group or optionally substituted imino group" for K optionally has include those similar to the "C₁₋₁₀ alkyl group, C₂₋₁₀ alkenyl group, C₃₋₁₀ cycloalkyl group, C₃₋₁₀ cycloalkenyl group, C₆₋₁₄ aromatic hydrocarbon group, C₇₋₁₃ aralkyl group and C₈₋₁₃ aromatic hydrocarbon-alkenyl group, each of which is optionally substituted" exemplified as the substituent of the "optionally substituted amino group" for J.
K is preferably an imino group (-NH-).

Examples of the "optionally substituted hydrocarbon group" and "optionally substituted heterocyclic group" for R include those exemplified as the above-mentioned "optionally substituted hydrocarbon group" and "optionally substituted heterocyclic group" for J.
Examples of the "substituent" which the "optionally substituted hydroxy group" for R optionally has include those similar to the "C₁₋₁₀ alkyl group, C₂₋₁₀ alkenyl group, C₃₋₁₀ cycloalkyl group, C₃₋₁₀ cycloalkenyl group, C₆₋₁₄ aromatic hydrocarbon group, C₇₋₁₃ aralkyl group and C₈₋₁₃ aromatic hydrocarbon-alkenyl group, each of which is optionally substituted" exemplified as the substituent of the above-mentioned "optionally substituted amino group" for J.
R is preferably a hydrogen atom, an optionally substituted hydrocarbon group (preferably C₁₋₆ alkyl) or the like.

Examples of the "substituent" for T or U include those exemplified as the substituent which the C₃₋₁₀ cycloalkyl group exemplified as the "hydrocarbon group" of the above-mentioned "optionally substituted hydrocarbon group" for J optionally has. The substituent is preferably
(1) a C₆₋₁₄ aromatic hydrocarbon group (preferably phenyl) optionally substituted by 1 to 3 substituents selected from a C₁₋₆ alkyl group optionally substituted by 1 to 3 halogen atoms, a hydroxy group, a C₁₋₆ alkoxy group, a carboxyl group, a halogen atom and a C₁₋₆ alkylsulfonyl group;
(2) an aromatic heterocyclic group (preferably pyridyl) optionally substituted by 1 to 3 substituents selected from a C₁₋₆ alkyl group optionally substituted by 1 to 3 halogen atoms, a hydroxy group, a C₁₋₆ alkoxy group, a carboxyl group and a halogen atom;
(3) a C₁₋₆ alkyl group optionally substituted by 1 to 3 substituents selected from a halogen atom, a carboxyl group, a C₁₋₆ alkoxy-carbonyl group and a carbamoyl group;
(4) a C₁₋₆ alkyl-carbonyl group optionally substituted by 1 to 3 halogen atoms;
   or the like.

Examples of the "bicyclic ring" of the "optionally substituted bicyclic ring" formed by T and U together with ring M include
(i) a bicyclic ring containing a 5- or 6-membered aromatic ring, from among rings corresponding to the "fused aromatic heterocyclic group" exemplified as the "heterocyclic group" of the above-mentioned "optionally substituted heterocyclic group" for J,
(ii) a bicyclic ring containing a 5- or 6-membered aromatic ring, from among rings corresponding to the "non-aromatic heterocyclic group" exemplified as the "heterocyclic group" of the above-mentioned "optionally substituted heterocyclic group" for J,
(iii) a bicyclic ring wherein a ring corresponding to the group selected from the C₃₋₁₀ cycloalkyl group, C₃₋₁₀ cycloalkenyl group, C₄₋₁₀ cycloalkadienyl group and C₆₋₁₄ aromatic hydrocarbon group exemplified as the above-mentioned "optionally substituted hydrocarbon group" for J, is condensed with a 5- or 6-membered aromatic ring,
   and the like. Examples of the "5- or 6-membered aromatic ring" include those exemplified as the above-mentioned ring M.
   Examples of the "bicyclic ring" include quinoline, isoquinoline, quinazoline, quinoxaline, benzofuran, benzothiophene, benzoxazole, benzisoxazole, benzothiazole, benzimidazole, benzotriazole (e.g., 1H-1,2,3-benzotriazole), indole, indazole, pyrrolopyrazine (e.g., 1H-pyrrolo[2,3-b]pyrazine, 1H-pyrrolo[2,3-b]pyrazine), imidazopyridine (e.g., 1H-imidazo[4,5-b]pyridine, 1H-imidazo[4,5-c]pyridine, 2H-imidazo[1,2-a]pyridine), imidazopyrazine (e.g., 1H-imidazo[4,5-b]pyrazine), pyrazolopyridine (e.g., 1H-pyrazolo[4,3-c]pyridine), thienopyridine (e.g., thieno[2,3-b]pyridine), pyrazolothiophene (e.g., 2H-pyrazolo[3,4-b]thiophene), pyrazolotriazine (e.g., pyrazolo[5,1-c][1,2,4]triazine), 4,5,6,7-tetrahydro-1-benzofuran, 4,5,6,7-tetrahydro-1-benzothiophene, indane, chromene, dihydroisoquinoline (e.g., 1,2-dihydroisoquinoline), tetrahydroisoquinoline (e.g., 1,2,3,4-tetrahydroisoquinoline), dihydrophthalazine (e.g., 1,4-dihydrophthalazine), pyrazolidine, tetrahydroquinoline (e.g., 1,2,3,4-tetrahydroquinoline), naphthalene, inden and the like, and benzothiophene, 4,5,6,7-tetrahydrobenzothiophene, thienopyridine and the like are preferable.
   The "substituent which the above-mentioned "optionally substituted bicyclic ring" optuinally has include those exemplified as the substituent which the C₃₋₁₀ cycloalkyl group exemplified as the "hydrocarbon group" of the above-mentioned "optionally substituted hydrocarbon group" for J optionally has.
   The substituent is preferably oxo, a C₁₋₆ alkyl group, a C₁₋₆ alkoxy group or the like.

Preferably T and U are independently
(1) a hydrogen atom;
(2) a C₆₋₁₄ aromatic hydrocarbon group (preferably phenyl) optionally substituted by substituent(s) (preferably 1 to 3 substituents selected from an a C₁₋₆ alkyl group optionally substituted by 1 to 3 halogen atoms, a hydroxy group, a C₁₋₆ alkoxy group, a carboxyl group, a halogen atom and a C₁₋₆ alkylsulfonyl group);
(3) an aromatic heterocyclic group (preferably pyridyl) optionally substituted by substituent(s) (preferably 1 to 3 substituents selected from an a C₁₋₆ alkyl group optionally substituted by 1 to 3 halogen atoms, a hydroxy group, a C₁₋₆ alkoxy group, a carboxyl group and a halogen atom); or
(4) a C₁₋₆ alkyl group optionally substituted by substituent(s) (preferably 1 to 3 substituents selected from a halogen atom, a carboxyl group, a C₁₋₆ alkoxy-carbonyl group and a carbamoyl group); or
   T and U form, together with ring M, a bicyclic ring (preferably benzothiophene, 4,5,6,7-tetrahydrobenzothiophene, thienopyridine) optionally substituted by substituent(s) (preferably 1 to 3 substituents selected from oxo, a C₁₋₆ alkyl group and a C₁₋₆ alkoxy group).
   Preferable combination of T and U is
   1) T is a C₆₋₁₄ aromatic hydrocarbon group (preferably phenyl) optionally substituted by substituent(s) (preferably 1 to 3 substituents selected from an a C₁₋₆ alkyl group optionally substituted by 1 to 3 halogen atoms, a hydroxy group, a C₁₋₆ alkoxy group, a carboxyl group, a halogen atom and a C₁₋₆ alkylsulfonyl group), and U is a hydrogen atom or C₁₋₆ alkyl (preferably a hydrogen atom);
   2) T and U form, together with ring M, a bicyclic ring (preferably benzothiophene, 4,5,6,7-tetrahydrobenzothiophene, thienopyridine) optionally substituted by substituent(s) (preferably 1, to 3 substituents selected from oxo, a C₁₋₆ alkyl group and a C₁₋₆ alkoxy group);
      or the like.
      Provided that when W moiety of ring M is =N- or -N= (e.g., ring M is 1,3-thiazole), then U shloud be absent.

D and G is preferably each a carbonyl group.

The "piperidine" or "piperazine" of the optionally substituted piperidine or optionally substituted piperazine" for ring P optionally further has 1 to 3 substituents, besides group D and group Q ring, at substitutable positions. Examples of the "substituents" include those exemplified as the substituents which the C₃₋₁₀ cycloalkyl group exemplified as the "hydrocarbon group" of the above-mentioned "optionally substituted hydrocarbon group" for J optionally has.
Ring P is preferably piperidine.

B is preferably CH.

Examples of the "monocyclic ring" of the "optionally substituted monocyclic ring" for ring Q include a monocyclic ring, from among rings corresponding to the C₃₋₁₀ cycloalkyl group, C₃₋₁₀ cycloalkenyl group, C₄₋₁₀ cycloalkadienyl group and C₆₋₁₄ aromatic hydrocarbon group exemplified as the above-mentioned "optionally substituted hydrocarbon group" for J, a ring corresponding to the monocyclic aromatic heterocyclic group and monocyclic non-aromatic heterocyclic group exemplified as the above-mentioned "optionally substituted heterocyclic group" for J, and the like.
The "monocyclic ring" is preferably piperidine, piperazine, morpholine, benzene or the like.
The monocyclic ring" of the "optionally substituted monocyclic ring" for ring Q optionally further has 1 to 3 substituents, besides group P ring and group G, at substitutable positions. Examples of the "substituent" include those exemplified as the substituents which the C₃₋₁₀ cycloalkyl group exemplified as the "hydrocarbon group" of the above-mentioned "optionally substituted hydrocarbon group" for J optionally has (e.g., a C₁₋₆ alkyl-carbonyl group optionally substituted by 1 to 3 halogen atoms, a C₁₋₆ alkyl group). The number of the substituents is, for example, 1 to 3.
Ring Q is preferably piperidine, piperazine, morpholine or benzene, each of which is optionally substituted by substituent(s) (e.g., 1 to 3 substituents selected from a C₁₋₆ alkyl-carbonyl group optionally substituted by 1 to 3 halogen atoms, and a C₁₋₆ alkyl group).

A is preferably C or N, more preferably N.

Preferable examples of compound (I) include the following compounds:
(Compound A)
Compound (I) wherein
ring M is thiophene, thiazole and the like;
K is an imino group;
R is a hydrogen atom or an optionally substituted hydrocarbon group (e.g., C₁₋₆ alkyl);
T is a C₆₋₁₄ aromatic hydrocarbon group (preferably phenyl), and,
U is a hydrogen atom or C₁₋₆ alkyl, or T and U form, together with ring M, a bicyclic ring (e.g., benzothiophene, 4,5,6,7-tetrahydrobenzothiophene) optionally substituted by substituent(s) (e.g., oxo);
D is a carbonyl group;
G is a carbonyl group;
ring P is piperidine;
B is CH;
ring Q is piperidine, piperazine, morpholine or benzene, each of which is optionally substituted by a C₁₋₆ alkyl-carbonyl group optionally substituted by 1 to 3 halogen atoms;
A is C or N, and
J is an optionally substituted heterocyclic group (e.g., morpholino) or an optionally substituted amino group (e.g., an amino group optionally mono- or di-substituted by C₁₋₆ alkyl group(s)).
(Compound B)
Compound (I) wherein
ring M is thiophene, thiazole or pyridine;
K is an imino group;
R is a hydrogen atom or an optionally substituted hydrocarbon group (e.g., C₁₋₆ alkyl);
T is a phenyl group optionally substituted by substituent(s) (e.g., 1 to 3 substituents selected from a C₁₋₆ alkyl group optionally substituted by 1 to 3 halogen atoms, a hydroxy group, a C₁₋₆ alkoxy group, a carboxyl group, a halogen atom and a C₁₋₆ alkylsulfonyl group), and U is a hydrogen atom;
D is a carbonyl group;
G is a carbonyl group;
ring P is piperidine;
B is CH;
ring Q is piperidine, piperazine, morpholine or benzene, each of which is optionally substituted by substituent(s) (e.g., 1 to 3 substituents selected from a C₁₋₆ alkyl-carbonyl group optionally substituted by 1 to 3 halogen atoms, and a C₁₋₆ alkyl group);
A is C or N, and
J is an optionally substituted heterocyclic group (e.g., a nitrogen-containing non-aromatic heterocyclic group (e.g., pyrrolidinyl, imidazolidinyl, pyrazolidinyl, piperidinyl, piperazinyl, morpholinyl, thiomorpholinyl, tetrahydroquinolinyl, tetrahydroisoquinolinyl) optionally substituted by 1 or 2 substituents selected from a C₁₋₆ alkyl group and a C₃₋₁₀ cycloalkyl group, each of which is optionally substituted by 1 to 3 halogen atoms), or an optionally substituted amino group (e.g., an amino group optionally mono- or di-substituted by substituent(s) selected from a C₁₋₆ alkyl group and a C₃₋₁₀ cycloalkyl group, each of which is optionally substituted by 1 to 3 halogen atoms).
(Compound C)
Compound (I) wherein
ring M is thiophene;
K is an imino group;
R is a hydrogen atom or an optionally substituted hydrocarbon group (e.g., C₁₋₆ alkyl);
T and U form, together with ring M, a bicyclic ring (e.g., benzothiophene, 4,5,6,7-tetrahydrobenzothiophene, thienopyridine) optionally substituted by substituent(s) (e.g., 1 to 3 substituents selected from oxo, a C₁₋₆ alkyl group and a C₁₋₆ alkoxy group);
D is a carbonyl group;
G is a carbonyl group;
ring P is piperidine;
B is CH;
ring Q is piperidine, piperazine, morpholine or benzene, each of which is optionally substituted by substituent(s) (e.g., 1 to 3 substituents selected from a C₁₋₆ alkyl-carbonyl group optionally substituted by 1 to 3 halogen atoms, and a C₁₋₆ alkyl group);
A is C or N, and
J is an optionally substituted heterocyclic group (e.g., a nitrogen-containing non-aromatic heterocyclic group (e.g., pyrrolidinyl, imidazolidinyl, pyrazolidinyl, piperidinyl, piperazinyl, morpholinyl, thiomorpholinyl, tetrahydroquinolinyl, tetrahydroisoquinolinyl) optionally substituted by 1 or 2 substituents selected from a C₁₋₆ alkyl group and a C₃₋₁₀ cycloalkyl group, each of which is optionally substituted by 1 to 3 halogen atoms), or an optionally substituted amino group (e.g., an amino group optionally mono- or di-substituted by substituent(s) selected from a C₁₋₆ alkyl group and a C₃₋₁₀ cycloalkyl group, each of which is optionally substituted by 1 to 3 halogen atoms).
(Compound D)
N,N-diethyl-1'-[(2-{[(ethylamino)carbonyl]amino}-1-benzothiophen-3-yl)carbonyl]-1,4'-bipiperidine-3-carboxamide;
N-ethyl-N'-(4-{[3-(morpholin-4-ylcarbonyl)-1,4'-bipiperidin-1'-yl]carbonyl}-2-phenyl-1,3-thiazol-5-yl)urea;
N-ethyl-N'-{3-{[(3R)-3-(morpholin-4-ylcarbonyl)-1,4'-bipiperdin-1'-yl]caxbonyl}-5-[4-(trifluoromethyl)phenyl]-2-thienyl}urea;
N-ethyl-N'-(7-methoxy-3-{[3-(piperidin-1-ylcarbonyl)-1,4'-bipiperidin-1'-yl]carbonyl}-1-benzothien-2-yl)urea; or N-ethyl-1'-[(2-{[(ethylamino)carbonyl]amino}-6-methylthieno[2,3-b]pyridin-3-yl)carbonyl]-N-isopropyl-1,4'-bipiperidine-3-carboxamide;
or a salt thereof.

As a salt of the compound represented by the formula (I), a pharmacologically acceptable salt is preferable. Examples of such salt include salts with inorganic base, salts with organic base, salts with inorganic acid, salts with organic acid, salts with basic or acidic amino acid and the like.
Preferable examples of salts with inorganic base include alkali metal salts such as sodium salt, potassium salt and the like; alkaline earth metal salts such as calcium salt, magnesium salt and the like; aluminum salt: ammonium salt and the like.
Preferable examples of salts with organic base include salts with trimethylamine, triethylamine, pyridine, picoline, ethanolamine, diethanolamine, triethanolamine, tromethamine[tris(hydroxymethyl)methylamine], tert-butylamine, cyclohexylamine, benzylamine, dicyclohexylamine or N,N-dibenzylethylenediamine.
Preferable examples of salts with inorganic acid include salts with hydrochloric acid, hydrobromic acid, nitric acid, sulfuric acid or phosphoric acid.
Preferable examples of salts with organic acid include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid or p-toluenesulfonic acid.
Preferable examples of salts with basic amino acid include salts with arginine, lysine or ornithine.
Preferable examples of salts with acidic amino acid include salts with aspartic acid or glutamic acid.
Of the above-mentioned salts, salts with inorganic acid and salts with organic acid are preferable, and salts with hydrochloride, trifluoroacetate or fumarate, and the like is preferable.

A prodrug of the compound (I) means a compound which is converted to the compound (I) with a reaction due to an enzyme, an gastric acid, etc. under the physiological condition in the living body, that is, a compound which is converted to the compound (I) by oxidation, reduction, hydrolysis, etc. according to an enzyme; a compound which is converted to the compound (I) by hydrolysis etc. due to gastric acid, etc.
Examples of the prodrug of compound (I) include a compound obtained by subjecting an amino group in compound (I) to an acylation, alkylation or phosphorylation (e.g., a compound obtained by subjecting an amino group in compound (I) to an eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation or tert-butylation); a compound obtained by subjecting a hydroxy group in compound (I) to an acylation, alkylation, phosphorylation or boration (e.g., a compound obtained by subjecting a hydroxy group in compound (I) to an acetylation, palmitoylation, propanoylation, pivaloylation, succinylation, fumarylation, alanylation, dimethylaminomethylcarbonylation); a compound obtained by subjecting a carboxyl group in compound (I) to an esterification or amidation (e.g., a compound obtained by subjecting a carboxyl group in compound (I) to an ethyl esterification, phenyl esterification, carboxymethyl esterification, dimethylaminomethyl esterification, pivaloyloxymethyl esterification, ethoxycarbonyloxyethyl esterification, phthalidyl esterification, (5-methyl-2-oxo-1,3-dioxolen-4-yl)methyl esterification, cyclohexyloxycarbonylethyl esterification or methyl amidation etc.) and the like. These compounds can be produced from compound (I) according to a method known per *se*.
A prodrug for compound (I) may also be one which is converted into compound (I) under a physiological condition, such as those described in IYAKUHIN no KAIHATSU, Development of Pharmaceuticals, Vol. 7, Design of Molecules, p. 163-198, Published by HIROKAWA SHOTEN, 1990.
In addition, compound (I) may be labeled with an isotope (e.g., ³H, ¹⁴C, ³⁵S, ¹²⁵I) and the like.
Moreover, compound (I) may be an anhydride or a hydrate.

Compound (I) or a prodrug thereof (hereinafter sometimes to be abbreviated simply as the compound of the present invention) has low toxicity, and can be used as an agent for the prophylaxis or treatment of various diseases mentioned below in a mammal (e.g., human, mouse, rat, rabbit, dog, cat, bovine, horse, swine, monkey) directly or in the form of a pharmaceutical composition by admixing with a pharmacologically acceptable carrier and the like.
Here, examples of the pharmacologically acceptable carrier include various organic or inorganic carrier substances conventionally used as preparation materials, which are added as excipient, lubricant, binder or disintegrant for solid dosage forms; as solvent, solubilizing agents, suspending agent, isotonicity agent, buffer or soothing agent for liquid preparation, and the like. Where necessary, preparation additives such as preservative, antioxidant, colorant, sweetener and the like can also be used.

Preferable examples of the excipient include lactose, sucrose, D-mannitol, D-sorbitol, starch, pregelatinized starch, dextrin, crystalline cellulose, low-substituted hydroxypropylcellulose, sodium carboxymethylcellulose, gum arabic, pullulan, light anhydrous silicic acid, synthetic aluminum silicate and magnesium aluminometasilicate.
Preferable examples of the lubricant include magnesium stearate, calcium stearate, talc and colloidal silica.
Preferable examples of the binder include pregelatinized starch, sucrose, gelatin, gum arabic, methylcellulose, carboxymethylcellulose, sodium carboxymethylcellulose, crystalline cellulose, sucrose, D-mannitol, trehalose, dextrin, pullulan, hydroxypropylcellulose, hydroxypropylmethylcellulose and polyvinylpyrrolidone.
Preferable examples of the disintegrant include lactose, sucrose, starch, carboxymethylcellulose, calcium carboxymethylcellulose, sodium croscarmellose, sodium carboxymethylstarch, light anhydrous silicic acid and low-substituted hydroxypropylcellulose.

Preferable examples of the solvent include water for injection, physiological brine, Ringer's solution, alcohol, propylene glycol, polyethylene glycol, sesame oil, corn oil, olive oil and cottonseed oil.
Preferable examples of the solubilizing agent include polyethylene glycol, propylene glycol, D-mannitol, trehalose, benzyl benzoate, ethanol, trisaminomethane, cholesterol, triethanolamine, sodium carbonate, sodium citrate, sodium salicylate and sodium acetate.
Preferable examples of the suspending agent include surfactants such as stearyltriethanolamine, sodium lauryl sulfate, lauryl aminopropionic acid, lecithin, benzalkonium chloride, benzethonium chloride, glyceryl monostearate and the like; hydrophilic polymers such as polyvinyl alcohol, polyvinylpyrrolidone, sodium carboxymethylcellulose, methylcellulose, hydroxymethylcellulose, hydroxyethylcellulose, hydroxypropylcellulose and the like; polysorbates and polyoxyethylene hydrogenated castor oil.

Preferable examples of the isotonicity agent include sodium chloride, glycerol, D-mannitol, D-sorbitol and glucose.
Preferable examples of the buffer include buffers such as phosphate, acetate, carbonate and citrate.
Preferable examples of the soothing agent include benzyl alcohol.
Preferable examples of the preservative include paraoxybenzoates, chlorobutanol, benzyl alcohol, phenethyl alcohol, dehydroacetic acid and sorbic acid.
Preferable examples of the antioxidant include sulfite and ascorbate.
Preferable examples of the colorant include aqueous food tar colors (e.g., food colors such as Food Red No. 2 and No. 3, Food Yellow No. 4 and No. 5, Food Blue No. 1 and No. 2, etc.), water insoluble lake dye (e.g., aluminum salt of the above-mentioned aqueous food tar color) and natural dye (e.g., p-carotene, chlorophyll, red iron oxide).
Preferable examples of the sweetening agent include sodium saccharin, dipotassium glycyrrhizinate, aspartame and stevia.

Examples of the dosage form of the above-mentioned pharmaceutical composition include oral preparations such as tablets (inclusive of sugar-coated tablets, film-coated tablets, sublingual tablets, orally disintegrating tablets), capsules (inclusive of soft capsules, microcapsules), granules, powders, troches, syrups, emulsions, suspensions, films (e.g., orally disintegrable films) and the like; and parenteral agents such as injections (e.g., subcutaneous injections, intravenous injections, intramuscular injections, intraperitoneal injections, drip infusions), external preparations (e.g., dermal preparations, ointments), suppository (e.g., rectal suppositorys, vaginal suppositorys), pellets, nasal preparations, pulmonary preparations (inhalants), eye drop sand the like. These may be administered safely orally or parenterally (e.g., topically, rectally, intravenous administrately).
These preparations may be release control preparations (e.g., sustained-release microcapsule) such as immediate-release preparation, sustained-release preparation and the like.
A pharmaceutical composition can be produced by a method conventionally used in the technical field of pharmaceutical preparation, for example, the method described in the Japanese Pharmacopoeia and the like. Specific production method of the preparation is explained in the followings.
While the content of the compound of the present invention in the pharmaceutical composition varies depending on the dosage form, dose of the compound of the present invention, and the like, it is, for example, about 0.1 to 100 wt%.

During production of an oral preparation, coating may be applied as necessary for the purpose of masking of taste, enteric property or durability.
Examples of the coating base to be used for coating include sugar coating base, aqueous film coating base, enteric film coating base, sustained-release film coating base and the like.

As the sugar coating base, sucrose is used. Moreover, one or more kinds selected from talc, precipitated calcium carbonate, gelatin, gum arabic, pullulan, carnauba wax and the like may be used in combination.
Examples of the aqueous film coating base include cellulose polymers such as hydroxypropyl cellulose, hydroxypropylmethyl cellulose, hydroxyethyl cellulose, methylhydroxyethyl cellulose etc.; synthetic polymers such as polyvinylacetal diethylaminoacetate, aminoalkyl methacrylate copolymer E [Eudragit E (trade name), Rohm Pharma Corp.], polyvinylpyrrolidone etc.; and polysaccharides such as pullulan etc.
Examples of the enteric film coating base include cellulose polymers such as hydroxypropylmethyl cellulose phthalate, hydroxypropylmethyl cellulose acetate succinate, carboxymethylethyl cellulose, cellulose acetate phthalate etc.; acrylic polymers such as methacrylic acid copolymer L [Eudragit L (trade name), Rohm Pharma Corp. ], methacrylic acid copolymer LD [Eudragit L-30D55 (trade name), Rohm Pharma Corp. ], methacrylic acid copolymer S [Eudragit S (trade name), Rohm Pharma Corp.] etc.; and naturally occurring substances such as shellac etc.
Examples of the sustained-release film coating base include cellulose polymers such as ethyl cellulose etc.; acrylic polymers such as aminoalkyl methacrylate copolymer RS [Eudragit RS (trade name), Rohm Pharma Corp.], ethyl acrylate-methyl methacrylate copolymer suspension [Eudragit NE (trade name), Rohm Pharma Corp.] and the like.
The aforementioned coating bases may be used after mixing with two or more kinds thereof at appropriate ratios. For coating, for example, a light shielding agent such as titanium oxide, diiron trioxide and the like can be used.

The compound of the present invention shows low toxicity (e.g., acute toxicity, chronic toxicity, genetic toxicity, reproductive toxicity, cardiotoxicity, carcinogenicity and the like) and a few side effects. Therefore, it can be used as an agent for the prophylaxis or treatment of or a diagnostic of various diseases in a mammal (e.g., human, bovine, horse, dog, cat, monkey, mouse, rat etc., specially human).
The compound of the present invention has a superior ACC (acetyl-CoA carboxylase) inhibitory action. Examples of ACC include liver, adipose tissue or pancreas-specific isozyme (ACC1); and muscle specific isozyme (ACC2). The compound of the present invention particularly has a selective inhibitory action on ACC2 and the like.

The compound of the present invention can be used as an agent for the prophylaxis or treatment of obesity, diabetes (e.g., type 1 diabetes, type 2 diabetes, gestational diabetes, obese diabetes), hyperlipidemia (e.g., hypertriglyceridemia, hypercholesterolemia, hypoHDL-emia, postprandial hyperlipemia), hypertension, cardiac failure, diabetic complications [e.g., neuropathy, nephropathy, retinopathy, cataract, macroangiopathy, osteopenia, hyperosmolar diabetic coma, infections (e.g., respiratory infection, urinary tract infection, gastrointestinal infection, dermal soft tissue infections, inferior limb infection), diabetic gangrene, xerostomia hypacusis, hypacusis, cerebrovascular disorder, peripheral blood circulation disorder], metabolic syndrome (pathology having three or more selected from hypertriglyceridemia (TG), low HDL cholesterol (HDL-C), hypertension, abdomen obesity and impaired glucose tolerance), sarcopenia and the like.

For diagnostic criteria of diabetes, Japan Diabetes Society reported new diagnostic criteria.
According to this report, diabetes is a condition showing any of a fasting blood glucose level (glucose concentration of intravenous plasma) of not less than 126 mg/dl, a 75 g oral glucose tolerance test (75 g OGTT) 2 hr level (glucose concentration of intravenous plasma) of not less than 200 mg/dl, and a non-fasting blood glucose level (glucose concentration of intravenous plasma) of not less than 200 mg/dl. A condition not falling under the above-mentioned diabetes and different from "a condition showing a fasting blood glucose level (glucose concentration of intravenous plasma) of less than 110 mg/dl or a 75 g oral glucose tolerance test (75 g OGTT) 2 hr level (glucose concentration of intravenous plasma) of less than 140 mg/dl" (normal type) is called a borderline type".

In addition, ADA (American Diabetes Association) reported new diagnostic criteria of diabetes and WHO.
According to these reports, diabetes is a condition showing a fasting blood glucose level (glucose concentration of intravenous plasma) of not less than 126 mg/dl and a 75 g oral glucose tolerance test 2 hr level (glucose concentration of intravenous plasma) of not less than 200 mg/dl.

According to the above-mentioned reports from ADA and WHO, impaired glucose tolerance is a condition showing a 75 g oral glucose tolerance test 2 hr level (glucose concentration of intravenous plasma) of not less than 140 mg/dl and less than 200 mg/dl. According to the report of ADA, a condition showing a fasting blood glucose level (glucose concentration of intravenous plasma) of not less than 100 mg/dl and less than 126 mg/dl is called IFG (Impaired Fasting Glucose). On the other hand, WHO defines the IFG (Impaired Fasting Glucose) to be a condition showing a fasting blood glucose level (glucose concentration of intravenous plasma) of not less than 110 mg/dl and less than 126 mg/dl, and calls it IFG (Impaired Fasting Glycaemia).

The compound of the present invention can be also used as an agent for the prophylaxis or treatment of diabetes, borderline type, impaired glucose tolerance, IFG (Impaired Fasting Glucose) and IFG (Impaired Fasting Glycemia), as determined according to the above-mentioned new diagnostic criteria. Moreover, the compound of the present invention can prevent progress of borderline type, impaired glucose tolerance, IFG (Impaired Fasting Glucose) or IFG (Impaired Fasting Glycemia) into diabetes.

The compound of the present invention can also be used as an agent for the prophylaxis or treatment of diabetic complications, osteoporosis, cachexia (e.g., carcinocachexia, tuberculous cachexia, diabetic cachexia, hemopathic cachexia, endocrinopathic cachexia, infectious cachexia or cachexia induced by acquired immunodeficiency syndrome), fatty liver, polycystic ovary syndrome, renal disease (e.g., diabetic nephropathy, glomerulonephritis, glomerulosclerosis, nephrosissyndrome, hypertensive nephrosclerosis, terminal renal disorder), muscular dystrophy, myocardial infarction, angina pectoris, cerebrovascular disorder (e.g., cerebral infarction, cerebral apoplexy), Alzheimer's disease, Parkinson's disease, anxiety, dementia, insulin resistance syndrome, syndrome X, hyperinsulinemia, sensory abnormality in hyperinsulinemia, tumor (e.g., leukemia, breast cancer, prostate cancer, skin cancer), irritable bowel syndrome, acute or chronic diarrhea, inflammatory disease (e.g., arteriosclerosis (e.g., atherosclerosis), chronic rheumatoid arthritis, spondylitis deformans, osteoarthritis, lumbago, gout, postoperative or posttraumatic inflammation, swelling, neuralgia, pharyngolaryngitis, cystitis, hepatitis (including nonalcoholic steatohepatitis), pneumonia, pancreatitis, enteritis, inflammatory bowel disease (including inflammatory colitis), ulcerative colitis, stomach mucous membrane injury (including stomach mucous membrane injury caused by aspirin)), small intestine mucous membrane injury, malabsorption, testis dysfunction, visceral obesity syndrome or sarcopenia.
The compound of the present invention can also be used for secondary prevention or suppression of progression of the above-mentioned various diseases (e.g., cardiovascular events such as myocardial infarction and the like).

While the dose of the compound of the present invention varies depending on the subject of administration, administration route, target disease, symptom and the like, for example, for oral administration to an adult diabetic patient, it is generally about 0.01 to 100 mg/kg body weight, preferably 0.05 to 30 mg/kg body weight, more preferably 0.1 to 10 mg/kg body weight for one dose, which is desirably administered once to 3 times a day.

With the aim of enhancing the action of the compound of the present invention or decreasing the dose of the compound and the like, the compound can be used in combination with pharmaceutical agents such as therapeutic agents for diabetes, therapeutic agents for diabetic complications, anti-hyperlipidemia agents, antihypertensive agents, antiobesity agents, diuretics, antithrombotic agents and the like (hereinafter to be abbreviated as concomitant drug). The time of administration of the compound of the present invention and that of the concomitant drug are not limited, and they may be administered simultaneously or in a staggered manner to the administration subject. In addition, the compound of the present invention and the concomitant drug may be administered as two kinds of preparations containing respective active ingredients or a single preparation containing both active ingredients.
The dose of the concomitant drug can be appropriately determined based on the dose employed clinically. In addition, the mixing ratio of the compound of the present invention and the concomitant drug can be appropriately determined according to the administration subject, administration route, target disease, condition, combination, and the like. For example, when the administration subject is a human, the concomitant drug may be used in an amount of 0.01 to 100 parts by weight per 1 part by weight of the compound of the present invention.

Examples of the therapeutic agents for diabetes include insulin preparations (e.g., animal insulin preparations extracted from pancreas of bovine or swine; human insulin preparations genetically synthesized using *Escherichia coli* or yeast; zinc insulin; protamine zinc insulin; fragment or derivative of insulin (e.g., INS-1), oral insulin preparation), insulin sensitizers (e.g., pioglitazone or a salt thereof (preferably hydrochloride), rosiglitazone or a salt thereof (preferably maleate), Tesaglitazar, Ragaglitazar, Muraglitazar, Edaglitazone, Metaglidasen, Naveglitazar, AMG-131, THR-0921), α-glucosidase inhibitors (e.g., voglibose, acarbose, miglitol, emiglitate), biguanides (e.g., metformin, buformin or a salt thereof (e.g., hydrochloride, fumarate, succinate)), insulin secretagogues [sulfonylurea (e.g., tolbutamide, glibenclamide, gliclazide, chlorpropamide, tolazamide, acetohexamide, glyclopyramide, glimepiride, glipizide, glybuzole), repaglinide, nateglinide, mitiglinide or a calcium salt hydrate thereof], dipeptidyl peptidase IV inhibitors (e.g., Vildagliptin, Sitagliptin, Saxagliptin, T-6666, TS-021), β3 agonists (e.g., AJ-9677), GPR40 agonists, GLP-1 receptor agonists [e.g., GLP-1, GLP-1MR agent, NN-2211, AC-2993 (exendin-4), BIM-51077, Aib(8,35)hGLP-1(7,37)NH₂, CJC-1131], amylin agonists (e.g., pramlintide), phosphotyrosine phosphatase inhibitors (e.g., sodium vanadate), gluconeogenesis inhibitors (e.g., glycogen phosphorylase inhibitors, glucose-6-phosphatase inhibitors, glucagon antagonists), SGLUT (sodium-glucose cotransporter) inhibitors (e.g., T-1095), 11β-hydroxysteroid dehydrogenase inhibitors (e.g., BVT-3498), adiponectin or agonist thereof, IKK inhibitors (e.g., AS-2868), leptin resistance improving drugs, somatostatin receptor agonists, glucokinase activators (e.g., Ro-28-1675), GIP (Glucose-dependent insulinotropic peptide) and the like.

Examples of the therapeutic agent for diabetic complications include aldose reductase inhibitors (e.g., tolrestat, epalrestat, zenarestat, zopolrestat, minalrestat, fidarestat, CT-112), neurotrophic factors and increasing drugs thereof (e.g., NGF, NT-3, BDNF, neurotrophin (e.g., 4-(4-chlorophenyl)-2-(2-methyl-1-imidazalyl)-5-[3-(2-methylphenoxy)propyl]oxazole)) described in WO01/14372, nerve regeneration promoters (e.g., Y-128), PKC inhibitors (e.g., ruboxistaurin mesylate), AGE inhibitors (e.g., ALT946, pimagedine, pyratoxanthine, N-phenacylthiazolium bromide (ALT766), ALT-711, EXO-226, pyridorin, pyridoxamine), active oxygen scavengers (e.g., thioctic acid), cerebral vasodilators (e.g., tiapuride, mexiletine), somatostatin receptor agonists (e.g., BIM23190) and apoptosis signal regulating kinase-1 (ASK-1) inhibitor.
Examples of the anti-hyperlipidemia agent include statin compounds (e.g., pravastatin, simvastatin, lovastatin, atorvastatin, fluvastatin, rosuvastatin, pitavastatin or a salt thereof (e.g., sodium salt, calcium salt)), squalene synthase inhibitors (e.g., compounds described in WO97/10224, for example, N-[[(3R,5S)-1-(3-acetoxy-2,2-dimethylpropyl)-7-chloro-5-(2,3-dimethoxyphenyl)-2-oxo-1,2,3,5-tetrahydro-4,1-benxoxazepin-3-yl]acetyl]piperidine-4-acetic acid), fibrate compounds (e.g., bezafibrate, clofibrate, simfibrate, clinofibrate), ACAT inhibitors (e.g., avasimibe, eflucimibe), anion exchange resins (e.g., colestyramine), probucol, nicotinic acid drugs (e.g., nicomol, niceritrol), ethyl icosapentate and phytosterols (e.g., soysterol, γ-oryzanol).

Examples of the antihypertensive agent include angiotensin converting enzyme inhibitors (e.g., captopril, enalapril, delapril), angiotensin II antagonists (e.g., candesartan cilexetil, losartan, eprosartan, valsartan, telmisartan, irbesartan, tasosartan, 1-[[2'-(2,5-dihydro-5-oxo-4H-1,2,4-oxadiazol-3-yl)biphenyl-4-yl]methyl]-2-ethoxy-1H-benzimidazole-7-carboxylic acid), calcium antagonists (e.g., manidipine, nifedipine, amlodipine, efonidipine, nicardipine), potassium channel openers (e.g., levcromakalim, L-27152, AL 0671, NIP-121) and clonidine.
Examples of the antiobesity agent include central nervous system antiobesity drugs (e.g., dexfenfluramine, fenfluramine, phentermine, sibutramine, amfepramone, dexamphetamine, mazindol, phenylpropanolamine, clobenzorex; MCH receptor antagonists (e.g., SB-568849; SNAP-7941; compounds contained in WO01/82925 and WO01/87834); neuropeptide Y antagonists (e.g., CP-422935); cannabinoid receptor antagonists (e.g., SR-141716, SR-147778); ghrelin antagonist; 11β-hydroxysteroid dehydrogenase inhibitors (e.g., BVT-3498)), pancreatic lipase inhibitors (e.g., orlistat, cetilistat), β3 agonists (e.g., AJ-9677, AZ40140), anorectic peptides (e.g., leptin, CNTF (ciliary neurotrophic factor)), cholecystokinin agonists (e.g., lintitript, FPL-15849) and feeding deterrents (e.g., P-57).

Examples of the diuretics include xanthine derivatives (e.g., theobromine sodium salicylate, theobromine calcium salicylate), thiazide preparations (e.g., ethiazide, cyclopenthiazide, trichloromethiazide, hydrochlorothiazide, hydroflumethiazide, benzylhydrochlorothiazide, penflutizide, polythiazide, methyclothiazide), antialdosterone preparations (e.g., spironolactone, triamterene), carbonic anhydrase inhibitors (e.g., acetazolamide), chlorobenzenesulfonamide agents (e.g., chlortalidone, mefruside, indapamide), azosemide, isosorbide, ethacrynic acid, piretanide, bumetanide and furosemide.

Examples of the antithrombotic agent include heparin (e.g., heparin sodium, heparin calcium, dalteparin sodium), warfarin (e.g., warfarin potassium etc.), anti-thrombin drugs (e.g., aragatroban), thrombolytic agents (e.g., urokinase, tisokinase, alteplase, nateplase, monteplase, pamiteplase) and platelet aggregation inhibitors (e.g., ticlopidine hydrochloride, cilostazol, ethyl icosapentate, beraprost sodium, sarpogrelate hydrochloride).

The production method of compound (I) is explained in the following. Compound (I) can be produced by, for example, the [Production Method] to be described in detail in the following or a method according thereto.
In the following [Production Method], the compound used as a starting compound may be each in the form of a salt. Examples of the salt include those exemplified as the salt of the compound represented by the formula (I).
When alkylation reaction, hydrolysis, amination reaction, esterification reaction, amidation reaction, esterification reaction, etherification reaction, oxidation reaction, reduction reaction and the like are to be performed in the following Production Method, these reactions are performed according to a method known per se. Examples of such method include the methods described in ORGANIC FUNCTIONAL GROUP PREPARATIONS, 2nd ed., ACADEMIC PRESS, INC., 1989; Comprehensive Organic Transformations, VCH Publishers Inc., 1989 and the like, and the like.

### [Production Method]

Compound (I) can be produced, for example, according to the following Reaction Schemes 1 and 2.

### <Reaction Scheme 1>

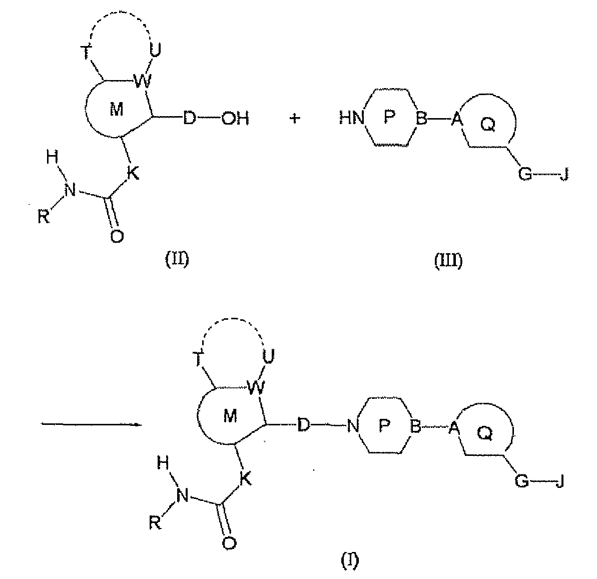

wherein the symbols in the formula are as defined above.
This reaction is carried out by the following "method using a dehydration-condensation agent" or "method using a reactive derivative of carboxylic acid or sulfonic acid" or the like.

### i) Method using a dehydration-condensation agent

Compound (III), 1 to 5 equivalents of compound (II), and 1 to 2 equivalents of a dehydration-condensation agent are reacted in an inert solvent. Where necessary, the reaction can be carried out in the presence of 1 to 1.5 equivalents of 1-hydroxybenzotriazole (HOBt), a catalytic amount to 5 equivalents of a base, and the like.
Examples of the above-mentioned "dehydration-condensation agent" include dicyclohexylcarbodiimide (DCC), 1-ethyl-3-(3-dimethylaminopropyl)carbodiimide hydrochloride (EDC-HCl) and the like. Of these, EDC-HCl is preferable.
Examples of the above-mentioned "inert solvent," include nitrile solvents, amide solvents, halogenated hydrocarbon solvents, ether solvents and the like. Two or more solvents may be used in a mixture at an appropriate ratio.
As the nitrile solvents, for example, acetonitrile and propionitrile can be used. Of these, acetonitrile is preferable.
As the amide solvents, for example, N,N-dimethylformamide (DMF), N,N-dimethylacetamide and N-methylpyrrolidone can be used. Of these, DMF is preferable.
As the halogenated hydrocarbon solvents, for example, dichloromethane, chloroform, 1,2-dichloroethane and carbon tetrachloride can be used. Of these, dichloromethane is preferable.
As the ether solvents, for example, diethyl ether, tetrahydrofuran (THF), 1,4-dioxane and 1,2-dimethoxyethane can be used. Of these, THF is preferable.
Examples of the above-mentioned "base" include
1) strong bases such as hydrides of alkali metal or alkaline earth metal (e.g., lithium hydride, sodium hydride, potassium hydride, calcium hydride), amides of alkali metal or alkaline earth metal (e.g., lithium amide, sodium amide, lithium diisopropylamide, lithium dicyclohexylamide, lithium hexamethyldisilazide, sodium hexamethyldisilazide, potassium hexamethyldisilazide), lower (C₁₋₆ )alkoxides of alkali metal or alkaline earth metal (e.g., sodium methoxide, sodium ethoxide, potassium tert-butoxide) and the like;
2) inorganic bases such as hydroxides of alkali metal or alkaline earth metal (e.g., sodium hydroxide, potassium hydroxide, lithium hydroxide, barium hydroxide), carbonates of alkali metal or alkaline earth metal (e.g., sodium carbonate, potassium carbonate, cesium carbonate), alkali metal hydrogencarbonates (e.g., sodium hydrogen carbonate, potassium hydrogen carbonate) and the like; and
3) organic bases such as amines (e.g., triethylamine, diisopropylethylamine, N-methylmorpholine and the like); basic heterocyclic compounds (e.g., pyridine, 4-dimethylaminopyridine, DBU (1,8-diazabicyclo[5.4.0]undec-7-ene), DBN (1,5-diazabicyclo[4.3.0]non-5-ene), imidazole, 2,6-lutidine and the like) and the like
   and the like.
   Of these bases, triethylamine, 4-dimethylaminopyridine and the like are preferable.
   The reaction temperature is generally room temperature (the room temperature means 1 to 30°C in the specification). The reaction time is, for example, 1 to 24 hr.

### ii) Method using a reactive derivative of carboxylic acid or sulfonic acid

A reactive derivative of compound (II) and 1 to 5 equivalents (preferably 1 to 3 equivalents) of compound (III) are reacted in an inert solvent. Where necessary, the reaction can be carried out in the presence of 1 to 10 equivalents, preferably 1 to 3 equivalents of a base.
Examples of the "reactive derivative" of compound (II) include acid halides (e.g., acid chloride, acid bromide), mixed acid anhydrides (e.g., acid anhydrides with a C₁₋₆ alkylcarboxylic acid, a C₆₋₁₀ aryl-carboxylic acid or a C₁₋₆ alkylcarbonic acid), activated esters (e.g., esters with a phenol optionally having substituent(s), HOBt or N-hydroxysuccinimide) and the like.
Examples of the "substituent" of the above-mentioned "phenol optionally having substituent(s)" include a halogen atom, a nitro group, an optionally halogenated C₁₋₆ alkyl group and an optionally halogenated C₁₋₆ alkoxy group. The number of the substituents is, for example, 1 to 5.
Examples of the "optionally halogenated C₁₋₆ alkyl group" include a C₁₋₆ alkyl group optionally having 1 to 5, preferably 1 to 3 halogen atoms (e.g., methyl, ethyl, propyl, isopropyl, butyl, isobutyl, sec-butyl, tert-butyl, pentyl, hexyl).
Specific examples thereof include methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl and 6,6,6-trifluorohexyl.
Examples of the "optionally halogenated C₁₋₆ alkoxy group" include a C₁₋₆ alkoxy group optionally having 1 to 5, preferably 1 to 3 halogen atoms (e.g., methoxy, ethoxy, propoxy, butoxy, pentyloxy, hexyloxy). Specific examples thereof include methoxy, difluoromethoxy, trifluoromethoxy, ethoxy, 2,2,2-trifluoroethoxy, propoxy, isopropoxy, butoxy, 4,4,4-trifluorobutoxy, isobutoxy, sec-butoxy, pentyloxy, isopentyloxy and hexyloxy.
Specific examples of the "phenol optionally having substituent(s)" include phenol, pentachlorophenol, pentafluorophenol and p-nitrophenol.
The reactive derivative is preferably an acid halide.
Examples of the above-mentioned "inert solvent" include ether solvents, halogenated hydrocarbon solvents, aromatic solvents, aliphatic hydrocarbon solvents, nitrile solvents, amide solvents, ketone solvents, sulfoxide solvents and water. Two or more solvents may be used in a mixture at an appropriate ratio. Of these, acetonitrile, THF, dichloromethane, chloroform and the like are preferable.
As the ether solvents, halogenated hydrocarbon solvents, nitrile solvents and amide solvents, those exemplified for the above-mentioned "method using a dehydration-condensation agent" can be used.
As the aromatic solvents, for example, benzene, toluene, xylene and pyridine can be used.
As the aliphatic hydrocarbon solvents, for example, hexane, pentane and cyclohexane can be used.
As the ketone solvents, for example, acetone and methyl ethyl ketone can be used.
As the sulfoxide solvents, for example, dimethyl sulfoxide (DMSO) can be used.
As the above-mentioned "base", those similar to the base for the above-mentioned "method using a dehydration-condensation agent" can be used, and sodium hydride, potassium carbonate, sodium carbonate, sodium hydroxide, potassium hydroxide, sodium hydrogen carbonate, potassium hydrogen carbonate, triethylamine, pyridine and the like are preferable.
The reaction temperature is generally -20°C to 50°C, preferably room temperature.
The reaction time is generally 5 min to 40 hr, preferably 30 min to 18 hr.
In the above-mentioned production method, compound (III) used as a starting material compound can be produced according to method known per se, for example, the method described in WO03/72197 or a method analogous thereto. In addition, compound (II) can be produced according to a method known per se.

### <Reaction Scheme 2>

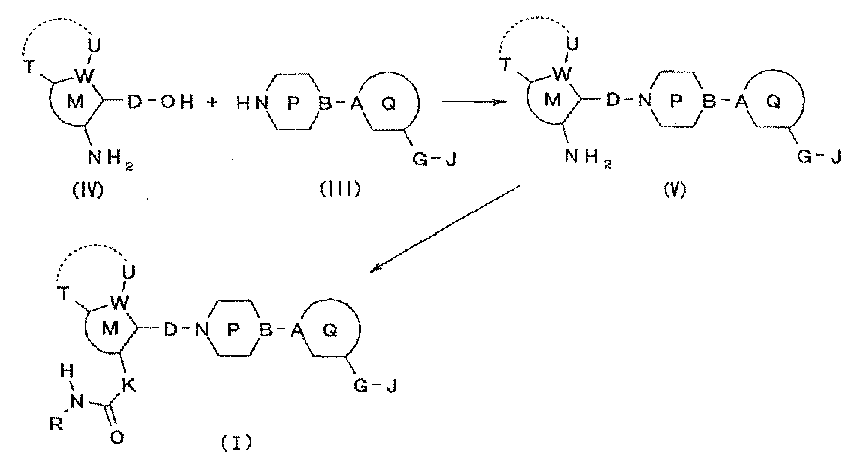

wherein the symbols in the formula are as defined above.
Compound (V) can be produced from compound (IV) and compound (III) in the same manner as in the above-mentioned Reaction Scheme 1.
Compound (IV) used as a starting material compound can be produced according to a method known per se.
Compound (I) wherein K is an optionally substituted imino group can be also produced by reacting compound (V) with an isocyanate in the presence of a base, if desired.
The amount of the isocyanate to be used is about 1 to about 5 mol, preferably about 1 to about 2 mol, per 1 mol of compound (V).
Examples of the "base" include strong bases, inorganic bases and basic heterocyclic compounds exemplified for the above-mentioned Reaction Scheme 1. The amount of the base to be used is about 1 to about 5 mol, preferably about 1 to about 3 mol, per 1 mol of compound (V).
This reaction is advantageously carried out without solvent or in a solvent inert to the reaction. While the solvent is not particularly limited as long as the reaction proceeds, and preferable examples of the solvent include ether solvents, aliphatic hydrocarbon solvents, amide solvents, halogenated hydrocarbon solvents, nitrile solvents, ketone solvents, sulfoxide solvents, aromatic solvents, water and the like, and a mixture of two or more solvent, and the like.
As the ether solvents, aliphatic hydrocarbon solvents, amide solvents, halogenated hydrocarbon solvents, nitrile solvents, ketone solvents, sulfoxide solvents and aromatic solvents, those exemplified for the above-mentioned Reaction Scheme 1 can be used.
The reaction temperature is about -20°C to about 200°C, preferably about 0°C to about 150°C. The reaction time is generally about 5 min to about 48 hr, preferably about 10 min to about 24 hr.

Compound (I) wherein K is an optionally substituted imino group can be also produced by condensing compound (V) with an amine represented by the formula:RNH₂ and an activating carbonyl compound [e.g, phosgene, bis(trichloromethyl) carbonate, N,N'-carbonyldiimidazole, isobutyl chlorocarbonate°].
The amount of the amine represented by the formula RNH₂ to be used is about 1 to about 3 mol, preferably about 1 to about 2 mol, per 1 mol of compound (V).
The amount of the activating carbonyl compound to be used is about 1 to about 10 mol, preferably about 1 to about 5 mol, per 1 mol of compound (V).
This reaction can be carried out in the presence of a base, if desired. Examples of the "base" include strong bases, inorganic bases and basic heterocyclic compounds exemplified for the above-mentioned Reaction Scheme 1. The amount of the base to be used is about 1 to about 10 mol, preferably about 1 to about 5 mol, per 1 mol of compound (V).
This reaction is advantageously carried out without solvent or in a solvent inert to the reaction. While the solvent is not particularly limited as long as the reaction proceeds, and preferable examples of the solvent include ether solvents, aliphatic hydrocarbon solvents, amide solvents, halogenated hydrocarbon solvents, nitrile solvents, ketone solvents, sulfoxide solvents, aromatic solvents and the like, and a mixture of two or more solvent, and the like.
As the ether solvents, aliphatic hydrocarbon solvents, amide solvents, halogenated hydrocarbon solvents, nitrile solvents, ketone solvents, sulfoxide solvents and aromatic solvents, those exemplified for the above-mentioned Reaction Scheme 1 can be used.
The reaction temperature is about -20°C to about 200°C, preferably about -10°C to about 100°C. The reaction time is generally about 5 min to about 48 hr, preferably about 10 min to about 24 hr.

In compound (I) thus obtained, a functional group within a molecule can also be converted to a desired functional group by a combination of chemical reactions known per *se.* Examples of the chemical reaction here include oxidation reaction, reduction reaction, alkylation reaction, hydrolysis reaction, amination reaction, esterification reaction, aryl coupling reaction, deprotection reaction and the like.

In the above-mentioned production methods, when the starting compound has an amino group, a carboxyl group, a hydroxy group or a carbonyl group as a substituent, a protecting group generally used in peptide chemistry and the like may be introduced into these groups. By removing the protecting group as necessary after the reaction, the object compound can be obtained.
Examples of the amino-protecting group include a formyl group, a C₁₋₆ alkyl-carbonyl group (e.g., acetyl, propionyl), a C₁₋₆ alkoxy-carbonyl group (e.g., methoxycarbonyl, ethoxycarbonyl, tert-butoxycarbonyl), a benzoyl group, a C₇₋₁₀ aralkyl-carbonyl group (e.g., benzylcarbanyl), a C₇₋₁₄ aralkyloxy-carbonyl group (e.g., benzyloxycarbonyl, 9-fluorenylmethoxycarbonyl), a trityl group, a phthaloyl group, a N,N-dimethylaminomethylene group, a substituted silyl group (e.g., trimethylsilyl, triethylsilyl, dimethylphenylsilyl, tert-butyldimethylsilyl, tert-butyldiethylsilyl), a C₂₋₆ alkenyl group (e.g., 1-allyl) and the like. These groups are optionally substituted by 1 to 3 substituents selected from a halogen atom, a C₁₋₆ alkoxy group (e.g., methoxy, ethoxy, propoxy) and a nitro group.
Examples of the carboxy-protecting group include a C₁₋₆ alkyl group (e.g., methyl, ethyl, propyl, isopropyl, butyl, tert-butyl), a C₇₋₁₁ aralkyl group (e.g. benzyl), a phenyl group, a trityl group, a substituted silyl group (e.g., trimethylsilyl, triethylsilyl, dimethylphenylsilyl, tert-butyldimethylsilyl, tert-butyldiethylsilyl), a C₂₋₆ alkenyl group (e.g., 1-ally) and the like. These groups are optionally substituted by 1 to 3 substituents selected from a halogen atom, a C₁₋₆ alkoxy group (e.g., methoxy, ethoxy, propoxy) and a nitro group.
Examples of the hydroxyl-protecting group include a C₁₋₆ alkyl group (e.g., methyl, ethyl, propyl, isopropyl, butyl, tert-butyl), a phenyl group, a trityl group, a C₇₋₁₀ aralkyl group (e.g., benzyl), a formyl group, a C₁₋₆ alkyl-carbonyl group (e.g., acetyl, propionyl), a benzoyl group, a C₇₋₁₀ aralkyl-carbonyl group (e.g., benzylcarbonyl), a 2-tetrahydropyranyl group, a 2-tetrahydrofuranyl group, a substituted silyl (e.g., trimethylsilyl, triethylsilyl, dimethylphenylsilyl, tert-butyldimethylsilyl, tert-butyldiethylsilyl), a C₂₋₆ alkenyl group (e.g., 1-allyl) and the like. These groups are optionally substituted by 1 to 3 substituents selected from a halogen atom, a C₁₋₆ alkyl group (e.g., methyl, ethyl, n-propyl), aC₁₋₆ alkoxy group (e.g., methoxy, ethoxy, propoxy) and a nitro group.
Examples of the carbonyl-protecting group include a cyclic acetal (e.g., 1,3-dioxane), a non-cyclic acetal (e.g., di-C₁₋₆ alkylacetals) and the like.
The method for removal of the above-mentioned protecting groups may be a method known *per se*, for example, a method according to the method described in Protective Groups in Organic Synthesis, John Wiley and Sons (1980), and the like. Specifically, a method using an acid, a base, ultraviolet rays, hydrazine, phenylhydrazine, sodium N-methyldithiocarbamate, tetrabutylammonium fluoride, palladium acetate, trialkylsilyl halide (e.g., trimethylsilyl iodide, trimethylsilyl bromide) and the like, a reduction method and the like.

Compound (I) obtained by the above-mentioned production methods can be isolated and purified by a known means, for example, solvent extraction, liquid conversion, phase transfer, crystallization, recrystallization, chromatography and the like.

When compound (I) contains an optical isomer, a stereoisomer, a regioisomer or a rotamer, these are also encompassed in compound (I), and can be obtained as a single product according to synthesis and separation methods known per se. For example, when compound (I) has an optical isomer, an optical isomer resolved from this compound is also encompassed in compound (I).
The optical isomer can be produced by a method known per se.

### Compound (I) may be a crystal.

Crystals of compound (I) (hereinafter sometimes to be abbreviated as the crystals of the present invention) can be produced by crystallization according to crystallization methods known per se.

In the present specification, the melting point means that measured using, for example, a micromelting point apparatus (Yanako, MP-500D or Buchi, B-545) or a DSC (differential scanning calorimetry) device (SEIKO, EXSTAR6000) and the like.
In general, the melting points vary depending on the measurement apparatuses, the measurement conditions and the like. The crystal in the present specification may show different values from the melting point described in the present specification, as long as they are within each of a general error range.
The crystal of the present invention is superior in physicochemical properties (e.g., melting point, solubility, stability etc.) and biological properties (e.g., pharmacokinetics (absorption, distribution, metabolism, excretion), efficacy expression etc.), and thus it is extremely useful as a medicament.

### Example

The present invention is explained in detail in the following by referring to Reference Examples, Examples, Preparation Examples and Experimental Examples, which are not to be construed as limitative. In addition, the present invention may be modified without departing from the scope of invention.
The abbreviations in Reference Examples and Examples indicate the following meanings.
s: singlet,
d: doublet,
t: triplet,
q: quartet,
m: multiplet,
br: broad,
J: coupling constant,
HOBt: 1-hydroxy-1H-benzotriazole,
DMF: N,N-dimethylformamide,
FBC·HCl: 1-ethyl-3-(3-dimethylaminopropyl)carbodiimide hydrochloride,
AcOEt: ethyl acetate,
THF: tetrahydrofuran.
In Reference Examples and Examples, "%" represents weight% unless otherwise stated.

### Reference Example 1 1-benzyl 4-tert-butyl 2-(morpholin-4-ylcarbonyl)piperazine-1,4-dicarboxylate

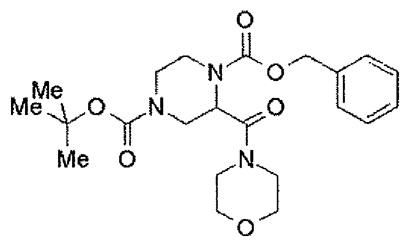

To a solution of 1-[(benzyloxy)carbonyl]-4-(tert-butoxycarbonyl)piperazine-2-carboxylic acid (2.95 g, 8.10 mmol), morpholine (0.847 ml, 9.71 mmol) and HOBt (1.24 g, 8.10 mmol) in DMF (10 ml) was added EDC·HCl (1.55 g, 8.10 mmol) under ice-cooling, and the mixture was stirred at room temperature for 16 hr. The reaction mixture was dissolved in ethyl acetate, and the solution was washed with 0.5N hydrochloric acid, aqueous potassium carbonate solution and saturated brine, and dried over anhydrous sodium sulfate. The solvent was evaporated under reduced pressure, and the obtained residue was passed through NH-silica gel column chromatography (eluent; ethyl acetate) to give the title compound (3.51 g, quantitative) as an oil. The compound was used for Reference Example 2 without purification.

### Reference Example 2 benzyl 2-(morpholin-4-ylcarbonyl)piperazine-1-carboxylate

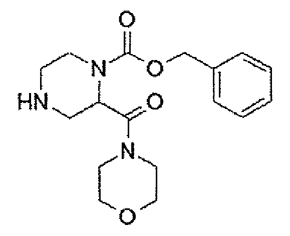

To 1-benzyl 4-tert-butyl 2-(morpholin-4-ylcarbonyl)piperazine-1,4-dicarboxylate obtained in Reference Example 1 (3.51 g, 8.10 mmol) was added 4N HCl-AcOEt (40 ml), and the solvent was evaporated under reduced pressure after 2 hr. The residue was dissolved in water, and the solution was basified with potassium carbonate, and extracted with ethyl acetate. The extract was washed with saturated brine, and dried over anhydrous sodium sulfate. The solvent was evaporated under reduced pressure to give the title compound (2.51 g, yield 92.9%) as an oil. The compound was used for Reference Example 3 without purification.

### Reference Example 3 benzyl 4-[1-(tert-butoxycarbonyl)piperidin-4-yl]-2-(morpholin-4-ylcarbonyl)piperazine-1-carboxylate

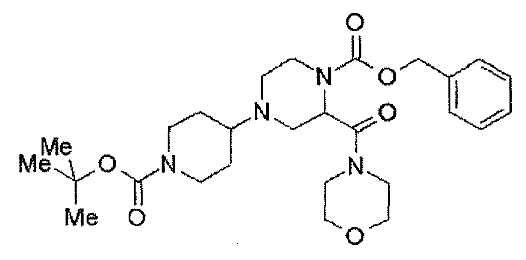

To a solution of benzyl 2-(morpholin-4-ylcarbonyl)piperazine-1-carboxylate obtained in Reference Example 2 (2.51 g, 7.53 mmol), tert-butyl 4-oxopiperidine-1-carboxylate (1.53 g, 7.53 mmol) and acetic acid (0.431 ml, 7.53 mmol) in THF (30 ml) was added sodium triacetoxyborohydride (2.39 g, 11.3 mmol), and the mixture was stirred at room temperature for 16 hr. The solvent was evaporated under reduced pressure. Ethyl acetate was added to the residue, and the mixture was washed with aqueous potassium carbonate solution and saturated brine, and dried over anhydrous sodium sulfate. The solvent was evaporated under reduced pressure, and the obtained residue was purified by silica gel column chromatography (eluent; hexane-ethyl acetate=1:1 to ethyl acetate) to give the title compound (1.73 g, yield 44.5%) as an oil. EI(pos) 517.2 [M+H]⁺

### Reference Example 4 tert-butyl 4-[3-(morpholin-4-ylcarbonyl)piperazin-1-yl]piperidine-1-carboxylate

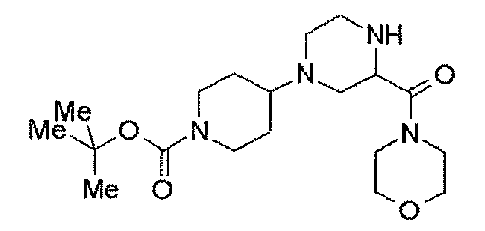

To benzyl 4-[1-(tert-butoxycarbonyl)piperidin-4-yl]-2-(morpholin-4-ylcarbonyl)piperazine-1-carboxylate obtained in Reference Example 3 (1.72 g, 3.32 mmol) and 10% palladium carbon (1 g) was added THF (20 ml), and the mixture was stirred at room temperature for 16 hr under a hydrogen atmosphere. The reaction mixture was filtered through celite, and the solvent was evaporated under reduced pressure to give the title compound (1.27 g, quantitative) as an oil. The compound was used for Reference Example 5 without purification.

Reference Example 5 tert-butyl 4-[3-(morpholin-4-ylcarbonyl)-4-(trifluoroacetyl)piperazin-1-yl]piperidine-1-carboxylate

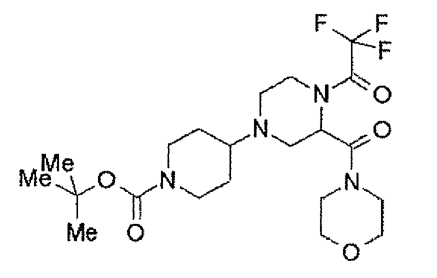

tert-Butyl 4-[3-(morpholin-4-ylcarbonyl)piperazin-1-yl]piperidine-1-carboxylate obtained in Reference Example 4 (1.27 g, 3.32 mmol) was dissolved in THF (20 ml), and triethylamine (0.693 ml, 4.98 mmol) and trifluoroacetic acid anhydride (0.563 ml, 3.99 mmol) were added under ice-cooling. The mixture was stirred at room temperature for 3 hr, and the solvent was evaporated under reduced pressure. Ethyl acetate was added to the residue, and the mixture was washed with aqueous sodium hydrogen carbonate solution and saturated brine, and dried over anhydrous sodium sulfate. The solvent was evaporated under reduced pressure to give the title compound (1.31 g, yield 82.4%) as an oil.
EI(pos) 479.2 [M+H]⁺

### Reference Example 6 4-{[4-(piperidin-4-yl)-1-(trifluoroacetyl)piperazin-2-yl]carbonyl}morpholine dihydrochloride

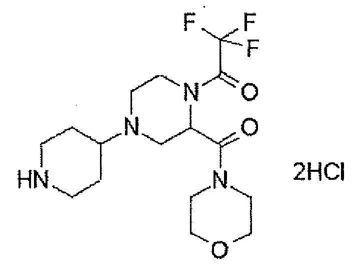

To tert-butyl 4-[3-(morpholin-4-ylcarbonyl)-4-(trifluoroacetyl)piperazin-1-yl]piperidine-1-carboxylate obtained in Reference Example 5 (1.31 g, 2.74 mmol) was added 4N hydrogen chloride-ethyl acetate (15 ml). The reaction mixture was stirred for 2 hr, and the solvent was evaporated under reduced pressure to give an oil (1.22 g, yield 98.2%).

### Reference Example 7 3-({4-[3-(morpholin-4-ylcarbonyl)-4-(trifluoroacetyl)piperazin-1-yl]piperidin-1-yl}carbonyl)-1-benzothiophen-2-amine

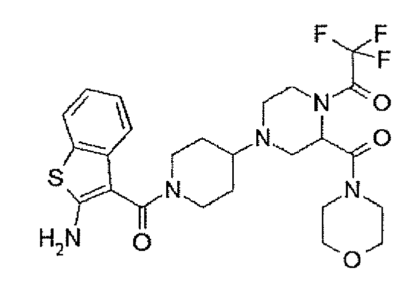

In the same manner as in Reference Example 1 and using 4-{[4-(piperidin-4-yl)-1-(trifluoroacetyl)piperazin-2-yl]carbonyl}morpholine dihydrochloride obtained in Reference Example 6 (500 mg, 1.11 mol) and 2-amino-1-benzathiophene-3-carboxylic acid (214 mg, 1.11 mmol), the title compound (407 mg, yield 66.5%) was obtained.
EI(pos) 554.1 [M+H]⁺

### Reference Example 8 benzyl 2-[(diethylamino)carbonyl]morpholine-4-carboxylate

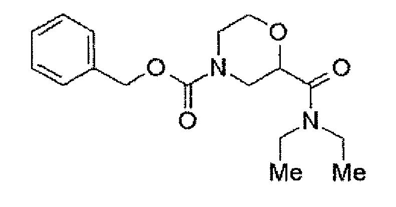

In the same manner as in Reference Example 1 and using 4-[(benzyloxy)carbonyl]morpholine-2-carboxylic acid (4.35 g, 16.4 mmol) and diethylamine (1.87 ml, 18.0 mmol) and triturating with diisopropyl ether, the title compound (4.68 g, yield 89.2%) was obtained. The compound was used for Reference Example 9 without purification.

### Reference Example 9 N,N-diethylmorpholine-2-carboxamide

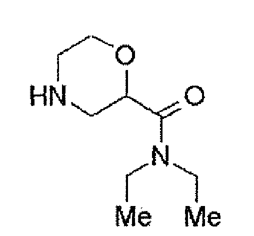

In the same manner as in Reference Example 4 and using benzyl 2-[(diethylamino)carbonyl]morpholine-4-carboxylate obtained in Reference Example 8 (4.68 g, 14.6 mmol), the title compound (2.39 g, yield 87.9%) was obtained. The compound was used for Reference Example 10 without purification.

### Reference Example 10 benzyl 4-{12-[(diethylamino)carbonyl]morpholin-4-yl}piperidine-1-carboxylate hydrochloride

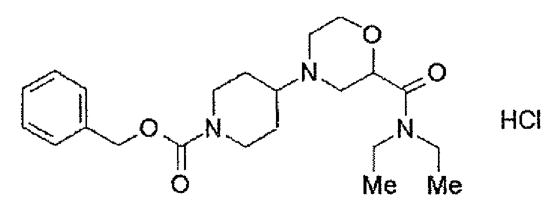

In the same manner as in Reference Example 3 and using N,N-diethylmorpholine-2-carboxamide obtained in Reference Example 9 (2.39 g, 12.8 mmol) and benzyl 4-oxopiperidine-1-carboxylate (3.29 g, 14.1 mmol), adding 4N hydrogen chloride-ethyl acetate (3.2 ml) to the obtained oil and triturating with diisopropyl ether, the title compound (5.65 g, quantitative) was obtained.

### Reference Example 11 N,N-diethyl-4-(piperidin-4-yl)morpholine-2-carboxamide hydrochloride

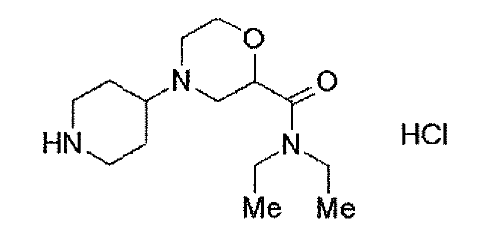

In the same manner as in Reference Example 4 and using benzyl 4-{2-[(diethylamino)carbonyl]morpholin-4-yl}piperidine-1-carboxylate hydrochloride obtained in Reference Example 10 (5.50 g, 12.5 mmol), the title compound (3.32 g, yield 87.0%) was obtained. The compound was used for Reference Example 12 without purification.

### Reference Example 12 4-{1-[(2-amino-1-benzothiophen-3-yl)carbonyl]piperidin-4-yl}-N,N-diethylmorpholine-2-carboxamide

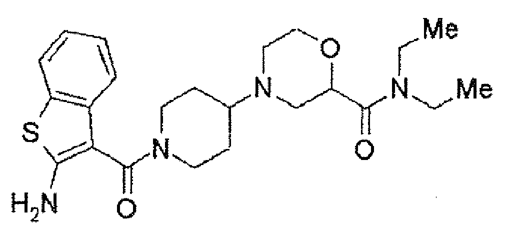

In the same manner as in Reference Example 1 and using N,N-diethyl-4-(piperidin-4-yl)morpholine-2-carboxamide hydrochloride obtained in Reference Example 11 (480 mg, 1.57 mmol) and 2-amino-1-benzothiophene-3-carboxylic acid (303 mg, 1.57 mmol) the title compound (393 mg, yield 56.3%) was obtained.
EI(pos) 445.1 [M+H]⁺

Reference Example 13 N,N-diethyl-3-(pyridin-4-yl)benzamide

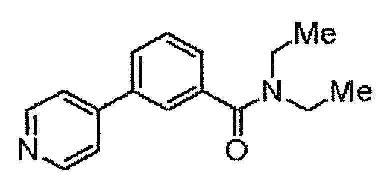

To a solution of 3-bromo-N,N-diethylbenzamide (3.00 g, 11.7 mmol), pyridin-4-ylboronic acid (2.88 g, 23.4 mmol) and 2N aqueous sodium carbonate solution (11.7 ml) in tetrahydrofuran (120 ml) was added tetrakistriphenylphosphinepalladium (406 mg, 0.351 mmol) under a nitrogen atmosphere, and the mixture was heated under reflux for 1 day. The mixture was allowed to cool to room temperature, and ethyl acetate was added. The aqueous layer was separated, and dried over anhydrous sodium sulfate. The solvent was evaporated under reduced pressure, and the obtained residue was purified by silica gel column chromatography (eluent; hexane-ethyl acetate=1:1 to ethyl acetate) to give the title compound (2.05 g, yield 68.8%) as an oil.
EI(pos) 255.2 [M+H]⁺

### Reference Example 14 tert-butyl 4-{3-[(diethylamino)carbonyl]phenyl}piperidine-1-carboxylate

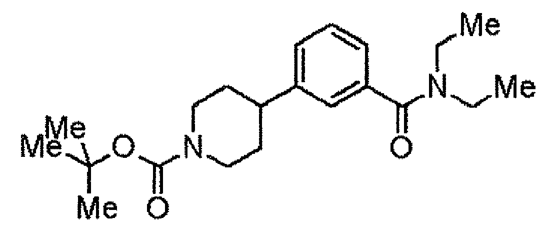

A suspension of N,N-diethyl-3-(pyridin-4-yl)benzamide obtained in Reference Example 13 (1.73 g, 6.80 mmol) and rhodium carbon (700 mg) in acetic acid (40 ml) was stirred at 80°C for 5 hr under 0.5 MPa of hydrogen atmosphere. After completion of the reaction, rhodium carbon was removed by filtration, and the filtrate was concentrated under reduced pressure. To a solution of the obtained residue and potassium carbonate (2.82 g, 20.4 mmol) in tetrahydrofuran (50 ml) and water (50 ml) was added di-tert-butyl dicarbonate (1.56 ml, 6.80 mmol), and the mixture was stirred for 16 hr. Ethyl acetate was added, and the aqueous layer was separated, washed with 0.5N hydrochloric acid, aqueous potassium carbonate solution and saturated brine, and dried over anhydrous sodium sulfate. The solvent was evaporated under reduced pressure, and the obtained residue was passed through silica gel column chromatography (eluent; hexane:ethyl acetate=3:1 to 1:1) to give the title compound (1.23 g. yield 50.2%) as an oil.
EI(pos) 361.0 [M+H]⁺

### Reference Example 15 N,N-diethyl-3-(piperidin-4-yl)benzamide hydrochloride

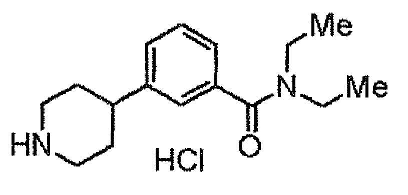

In the same manner as in Reference Example 6 and using tert-butyl 4-{3-[(diethylamino)carbonyl]phenyl}piperidine-1-carboxylate obtained in Reference Example 14 (1.23 g, 3.41 mmol), the title compound (906 mg, yield 89.7%) was obtained. The compound was used for Reference Example 16 without purification.

### Reference Example 16 3-{1-[(2-amino-1-benzothiophen-3-yl)carbonyl]piperidin-4-yl}-N,N-diethylbenzamide

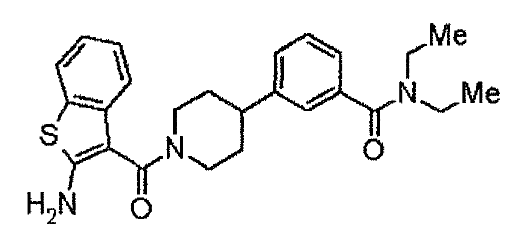

In the same manner as in Reference Example 1 and using N,N-diethyl-3-(piperidin-4-yl)benzamide hydrochloride obtained in Reference Example 15 (206 mg, 0.694 mmol) and 2-amino-1-benzothiophene-3-carboxylic acid (134 mg, 0.694 mmol), the title compound (230 mg, yield 76.2%) was obtained.
EI(pos) 436.0 [M+H]⁺

### Reference Example 17 1'-[(2-amino-1-benzothiophen-3-yl)carbonyl]-N,N-diethyl-1,4'-bipiperidine-3-carboxamide

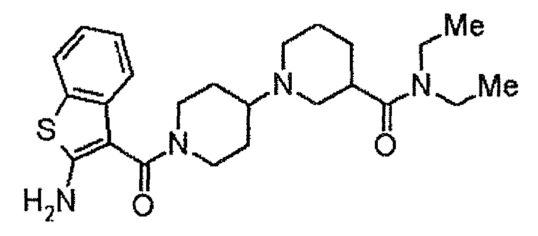

In the same manner as in Reference Example 1 and using N,N-diethyl-1,4'-bipiperidine-3-carboxamide dihydrochloride (220 mg, 0.646 mmol) and 2-amino-1-benzothiophene-3-carboxylic acid (124 mg, 0.646 mmol), the title compound (198 mg, yield 69.1%) was obtained.
EI(pos) 443.0 [M+H]⁺

### Reference Example 18 3-{[3-(morpholin-4-ylcarbonyl)-1,4'-bipiperidin-1'-yl]carbonyl}-1-benzothiophen-2-amine

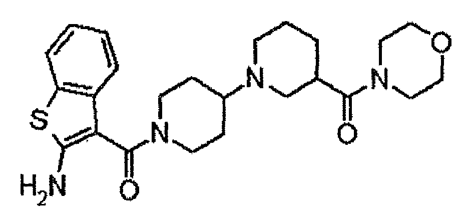

In the same manner as in Reference Example 1 and using 3-(morpholin-4-ylcarbonyl)-1,4'-bipiperidine dihydrochloride (600 mg, 1.69 mmol) and 2-amino-1-benzothiophene-3-carboxylic acid (327 mg, 1.69 mmol), the title compound (533 mg, yield 65.0%) was obtained. The compound was used for the next reaction without purification.

### Reference Example 19 2-amino-7-oxo-4,5,6,7-tetrahydro-1-benzothiophene-3-carboxylic acid

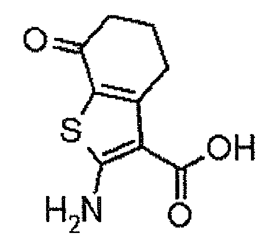

To ethyl 2-amino-7-oxo-4,5,6,7-tetrahydro-1-benzothiophene-3-carboxylate (885 mg, 3.70 mmol) were added ethanol (9 mL) and 2N aqueous sodium hydroxide solution (3.70 mL, 7.40 mmol), and the mixture was heated at 80°C for 3 hr. The solvent was evaporated under reduced pressure, and the residue was dissolved in water. 1N Hydrochloric acid was added, and the mixture was extracted with ethyl acetate. The extract was washed with saturated brine, and dried over anhydrous sodium sulfate. The solvent was evaporated under reduced pressure, and the obtained residue was purified by silica gel column chromatography (eluent; ethyl acetate) and triturated with diisopropyl ether to give the title compound (233 mg, yield 29.8%) was obtained.
¹H NMR (DMSO-d₆) δ1.98 (2H, m), 2.37 (2H, t, J = 6.3 Hz), 2.93 (2H, t, J = 6.0 Hz), 8.24 (2H, s), 12.53 (1H, s).

### Reference Example 20 2-amino-3-{[3-(morpholin-4-ylcarbonyl)-1,4'-bipiperidin-1'-yl]carbonyl}-5,6-dihydro-1-benzothiophen-7(4H)-one

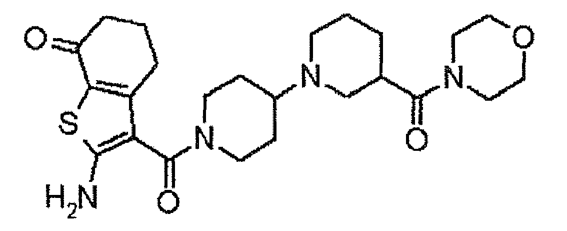

In the same manner as in Reference Example 1 and using 3-(morpholin-4-ylcarbonyl)-1,4'-bipiperidine dihydrochloride (200 mg, 0.564 mmol) and 2-amino-7-oxo-4,5,6,7-tetrahydro-1-benzothiophene-3-carboxylic acid obtained in Reference Example 19 (119 mg, 0.564 mmol), the title compound (20 mg, yield 7.5%) was obtained.
EI(pos) 475.1 [M+H]⁺

### Reference Example 21 methyl 2-[(tert-butoxycarbonyl)amino]-5-phenylthiophene-3-carboxylate

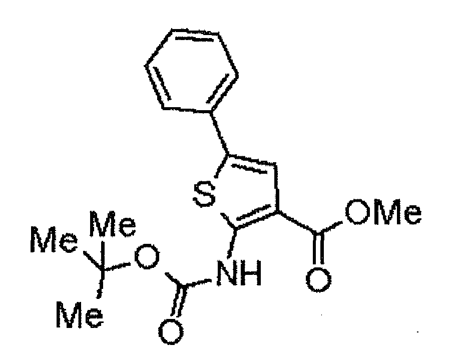

To methyl 2-amino-5-phenylthiophene-3-carboxylate (10.3 g, 44.2 mmol) in a mixed solvent of tetrahydrofuran-tert-butanol (50 ml-200 ml) were added di-tert-butyl bicarbonate (12.2 ml, 53.0 mmol) and 4-dimethylaminopyridine (270 mg, 2.21 mmol), and the mixture was stirred at room temperature for 14 hr. The reaction mixture was concentrated under reduced pressure, and the obtained white solid was washed with ethyl acetate to give the title compound (9.18 g, yield 62.3%) was obtained. ¹H NMR (CDCl₃) δ1.55 (9H, s), 3.89 (3H, s), 7.23-7.28 (1H, m), 7.33-7.38 (3H, m), 7.55-7.58 (2H, m), 10.06 (1H, brs).

### Reference Example 22 2-[(tert-butoxycarbonyl)amino]-5-phenylthiophene-3-carboxylic acid

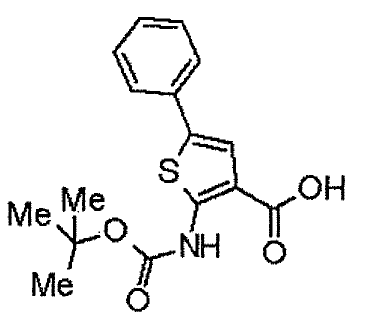

In the same manner as in Reference Example 19 and using methyl 2-[(tert-butoxycarbonyl)amino]-5-phenylthiophene-3-carboxylate obtained in Reference Example 21 (2.00 g, 6.00 mmol), the title compound (1.73 g, yield 90.0%) was obtained.
¹H NMR (CDCl₃) δ1.58 (9H, s), 7.27-7.30 (1H, m), 7.35-7.40 (2H, m), 7.43 (1H, d, J = 0.8 Hz), 7.56-7.59 (2H, m), 9.88 (1H, brs).

### Reference Example 23 tert-butyl [3-({3-[(diethylamino)carbonyl]-1,4'-bipiperidin-1'-yl}carbonyl)-5-phenyl-2-thienyl]carbamate

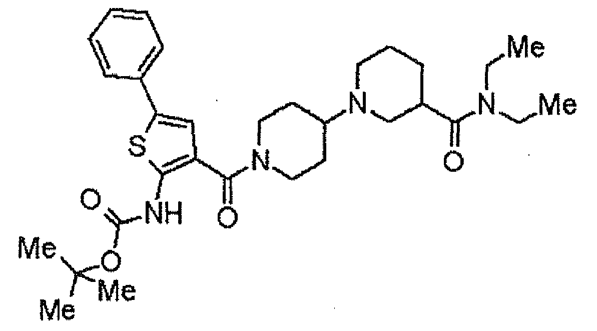

In the same manner as in Reference Example 1 and using N,N-diethyl-1,4'-bipiperidine-3-carboxamide dihydrochloride (218 mg, 0.641 mmol) and 2-[(tert-butoxycarbonyl)amino]-5-phenylthiophene-3-carboxylic acid obtained in Reference Example 22 (205 mg, 0.641 mmol), the title compound (295 mg, yield 80.9%) was obtained.
EI(pos) 569.1 [M+H]⁺

### Reference Example 24 1'-[(2-amino-5-phenyl-3'-thienyl)carbonyl]-N,N-diethyl-1,4'-bipiperidine-3-carboxamide

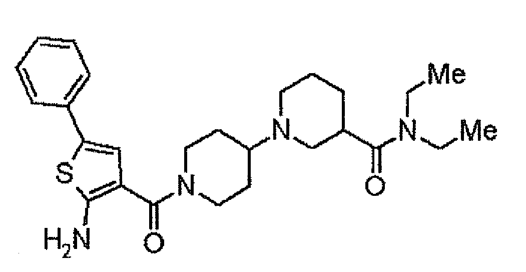

tert-Butyl [3-({3-[(diethylamino)carbonyl]-1,4'-bipiperidin-1'-yl}carbonyl)-5-phenyl-2-thienyl]carbamate obtained in Reference Example 23 (284 mg, 0.499 mmol) was dissolved in trifluoroacetic acid (2.5 mL), and the solution was concentrated under reduced pressure after 1 hr. The residue was dissolved in ethyl acetate, and the solution was washed with aqueous potassium carbonate solution and saturated brine. The solvent was evaporated under reduced pressure, and the residue was purified by NH-silica gel column chromatography (eluent; ethyl acetate) to give the title compound (221 mg, yield 94.4%) as an oil.
EI(pos) 469.0 [M+H]⁺

### Reference Example 25 tert-butyl (3-{[3-(morpholin-4-ylcarbonyl)-1,4'-bipiperidin-1'-yl]carbonyl}-5-phenyl-2-thienyl)carbamate

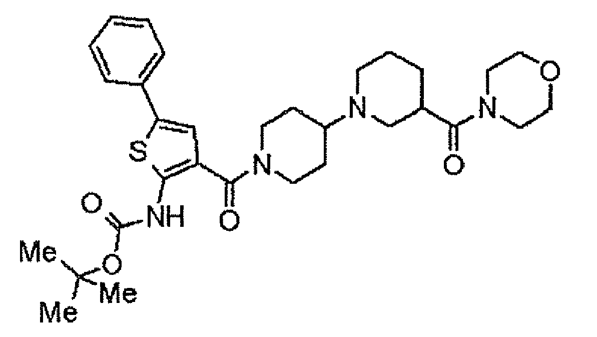

In the same manner as in Reference Example 1 and using 3-(morpholin-4-ylcarbonyl)-1,4'-bipiperidine dihydrochloride (500 mg, 1.41 mmol) and 2-[(tert-butoxycarbonyl)amino]-5-phenylthiophene-3-carboxylic acid obtained in Reference Example 22 (451 mg, 1.41 mmol), the title compound (698 mg, yield 84.9%) was obtained.
EI(pos) 583.1 [M+H]⁺

### Reference Example 26 3-{[3-(morpholin-4-ylcarbonyl)-1,4'-bipiperidin-1'-yl]carbonyl}-5-phenylthiophen-2-amine

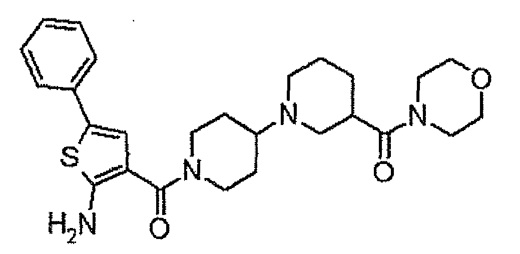

In the same manner as in Reference Example 13 and using tert-butyl (3-{[3-(morpholin-4-ylcarbonyl)-1,4'-bipiperidin-1'-yl]carbonyl}-5-phenyl-2-thienyl)carbamate obtained in Reference Example 25 (697 mg, 1.20 mmol), the title compound (491 mg, yield 85.2%) was obtained.
EI(pos) 483.0 [M+H]⁺

### Reference Example 27 5-amino-2-phenyl-1,3-thiazole-4-carboxylic acid

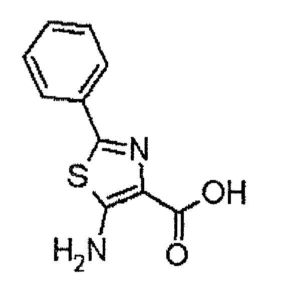

In the same manner as in Reference Example 19 and using ethyl 5-amino-2-phenyl-1,3-thiazole-4-carboxylate (480 mg, 1.93 mmol), the title compound (338 mg, yield 79.3%) was obtained.
¹H NMR (DMSO-d₆) δ7.35-7.46 (3H, m), 7.50 (2H, s), 7.73 (2H, m), 12.17 (1H, s).

### Reference Example 28 4-{[3-(morpholin-4-ylcarbonyl)-1,4'-bipiperidin-1'-yl]carbonyl}-2-phenyl-1,3-thiazole-5-amine

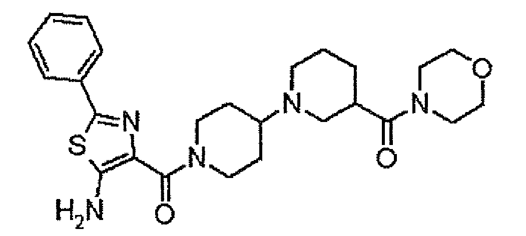

In the same manner as in Reference Example 1 and using 3-(morpholin-4-ylcarbonyl)-1,4'-bipiperidine dihydrochloride (331 mg, 0.935 mmol) and 5-amino-2-phenyl-1,3-thiazole-4-carboxylic acid obtained in Reference Example 27 (206 mg,0.935 mmol), the title compound (340 mg, yield 75.2%) was obtained.
EI(pos) 484.0 [M+H]⁺

### Reference Example 29 ethyl 2-(acetylamino)-6-bromo-7-oxo-4,5,6,7-tetrahydro-1-benzothiophene-3-carboxylate

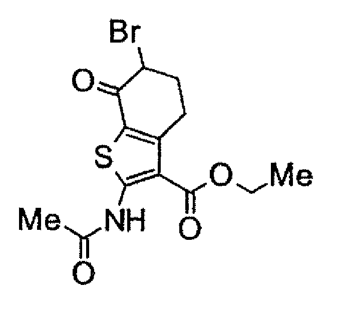

To a solution of ethyl 2-(acetylamino)-7-oxo-4,5,6,7-tetrahydro-1-benzothiophene-3-oarboxylate (8.00 g, 28.4 mmol) in chloroform (80 mL) was added dropwise a solution of bromine (1.5 mL, 29.8 mmol) in chloroform (15 mL) over 20 min while heating under reflux, and the mixture was stirred at the same temperature for 1.5 hr. The solution was washed threetimes with saturated brine, and dried over anhydrous magnesium sulfate.
The solvent was evaporated under reduced pressure, and the obtained residue was purified by silica gel column chromatography (eluent; ethyl acetate:hexane=1:1 to 1:0) to give the title compound (9.07 g, yield 89%) as an oil.

### Reference Example 30 ethyl 2-(acetylamino)-7-hydroxy-1-benzothiophene-3-carboxylate

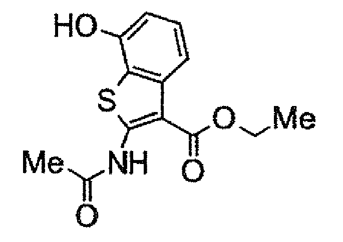

To a solution of ethyl 2-(acetylamino)-6-bromo-7-oxo-4,5,6,7-tetrahydro-1-benzothiophene-3-carboxylate obtained in Reference Example 29 (8.11 g, 22.6 mmol) in DMF (50 mL) was added lithium carbonate (2.50 g, 33.8 mmol), and the mixture was stirred at 90°C for 5 hr. Lithium carbonate (2.50 g, 33.8 mmol) was added again, and the mixture was stirred at 90°C for 3 hr. The reaction mixture was diluted with ethyl acetate, washed threetimes with saturated brine, and dried over anhydrous magnesium sulfate. The solvent was evaporated under reduced pressure, and the obtained solid was collected by filtration, and washed with hexane-ethyl acetate to give the title compound (2.63 g, yield 41%).
¹H NMR (DMSO-d₆) δ1.51 (3H, t, J = 7.2 Hz), 2.36 (3H, s), 4.48 (2H, q, J = 7.2 Hz), 5.35 (1H, s), 6.72 (1H, d, J = 8.5 Hz), 7.30 (1H, m), 7.89 (1H, d, J = 8.1 Hz), 11.77 (1H, br).

### Reference Example 31 ethyl 2-(acetylamino)-7-methoxy-1-benzothiophene-3-carboxylate

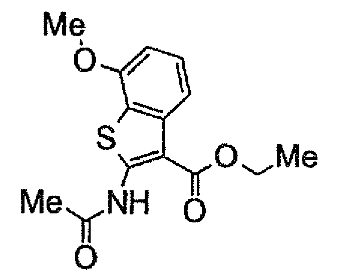

To a suspension of ethyl 2-(acetylamino)-7-hydroxy-1-benzothiophene-3-carboxylate obtained in Reference Example 30 (10.0 g, 35.8 mmol) and potassium carbonate (7.42 g, 53.7 mmol) in DMF (90 mL) was added methyl iodide (2.67 my, 43.0 mmol), and the mixture was stirred at room temperature for 5 hr. The reaction mixture was diluted with ethyl acetate, washed with water and saturated brine, and dried over anhydrous sodium sulfate. The solution was passed through basic silica gel, and the obtained residue was recrystallized from ethyl acetate to give the title compound (8.10 g, yield 77%) as a solid.
¹H NMR (DMSO-d₆) δ1.42 (3H, t, J = 7.2 Hz), 2.32 (3H, s), 3.95 (3H, s), 4.42 (2H, q, J = 7.2 Hz), 6.92 (1H, d, J = 7.5 Hz), 7.39 (1H, dd, J = 7.5, 8.1 Hz), 7.82 (1H, d, J = 8.1 Hz), 11.37 (1H, s).

### Reference Example 32 2-amino-7-methoxy-1-benzothiophene-3-carboxylic acid

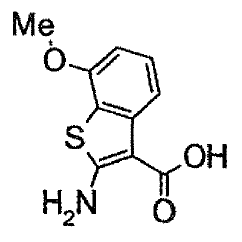

To a solution of ethyl 2-(acetylamino)-7-methoxy-1-benzothiophene-3-carboxylate obtained in Reference Example 31 (2.03 g, 6.92 mmol) in ethanol (35 mL) was added 8N aqueous sodium hydroxide solution (8.65 mL, 69.2 mmol), and the mixture was stirred at 90°C for 1 hr. The reaction mixture was concentrated under reduced pressure, and the residue was diluted with water. 1N Hydrochloric acid (70 mL) was added, and the mixture was extracted with ethyl acetate. The extract was washed with water and saturated brine, and dried over anhydrous sodium sulfate. The obtained residue was triturated with diisopropyl ether to give the title compound (1.27 g, yield 82%) as a solid.
¹H NMR (DMSO-d₆) δ3.87 (3H, s), 6.69 (1H, d, J = 7.5 Hz), 7.17-7.22 (1H, m), 7.60 (1H, d, J = 8.1 Hz), 7.92 (2H, br), 12.23 (1H, br).

### Reference Example 33 5-bromo-2-[(tert-butoxycarbonyl)amino]thiophene-3-carboxylic acid

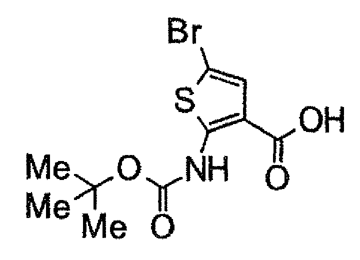

In the same manner as in Reference Example 19 and using methyl 5-bromo-2-[(tert-butoxycarbonyl)amino]thiophene-3-carboxylate (2.00 g, 5.95 mmol), the title compound (1.05 g, yield 55%) was obtained as a yellow solid.
¹H NMR (DMSO-d₆) δ1.50 (9H, s), 7.20 (1H, s), 10.20 (1H, br).

### Reference Example 34 tert-butyl (5-bromo-3-{[(3R)-3-(morpholin-4-ylcarbonyl)-1,4'-bipiperidin-1'-yl]carbonyl}-2-thienyl)carbamate

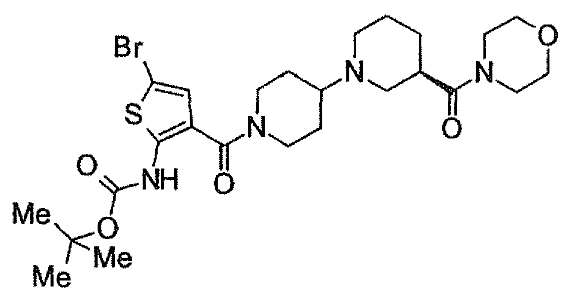

In the same manner as in Reference Example 1 and using (3R)-3-(morpholin-4-ylcarbonyl)-1,4'-bipiperidine dihydrochloride (1.70 g, 4.80 mmol) and 5-bromo-2-[(tert-butoxycarbonyl)amino]thiophene-3-carboxylic acid obtained in Reference Example 33 (1.55 g, 4.80 mmol), the title compound (2.76 g, yield 98.3%) was obtained.
EI(pos) 586.9 [M+H]⁺

### Reference Example 35 tert-butyl {3-{[(3R)-3-(morpholin-4-ylcarbonyl)-1,4'-bipiperadin-1'-yl]carbonyl}-5-[4-(trifluoromethyl)phenyl]-2-thienyl}carbamate

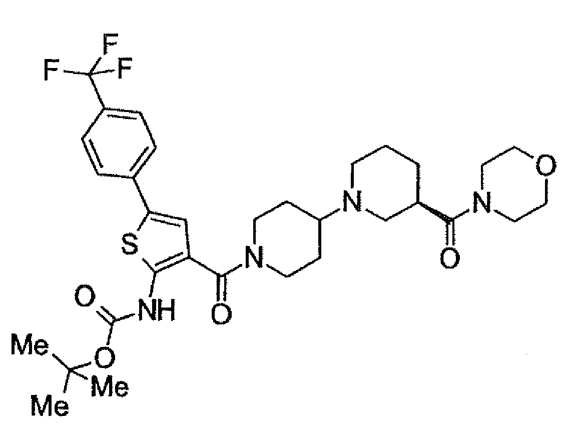

To a solution of tert-butyl (5-bromo-3-{[(3R)-3-(morpholin-4-ylcarbonyl)-1,4'-bipiperidin-1'-yl]carbonyl}-2-thienyl)carbamate obtained in Reference Example 34 (400 mg, 0.683 mmol), [4-(trifluoromethyl)phenyl]boronic acid (259 mg, 1.37 mmol) and 2N aqueous sodium carbonate solution (0.683 mL) in N,N-dimethylformamide (3 mL) was added 1,1'-bis(diphenylphosphino)ferrocenepalladium dichloride (27.9 mg, 0.034 mmol), and the mixture was stirred at 90°C for 16 hr under a nitrogen atmosphere. After completion of the reaction, the mixture was diluted with ethyl acetate, washed threetimes with saturated brine, and dried over anhydrous sodium sulfate. The solvent was evaporated under reduced pressure, and the obtained residue was purified twice by silica gel column chromatography (eluent; ethyl acetate:methanol=1:0 to 4:1) and basic silica gel column chromatography (eluent; hexane:ethyl acetate=3:1 to 0:1) to give the title compound (137 mg, yield 31%) was obtained. EI(pos) 651.1 [M+H]⁺

### Reference Example 36 3-{[(3R)-3-(morpholin-4-ylcarbonyl)-1,4'-bipiperidin-1'-yl]carbonyl}-5-[4-(trifluoromethyl)phenyl]thiophen-2-amine

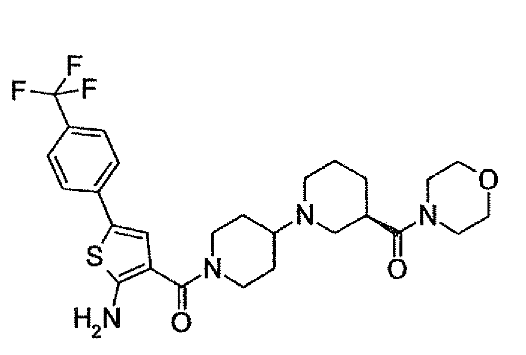

In the same manner as in Reference Example 24 and using tert-butyl {3-{[(3R)-3-(morpholin-4-ylcarbonyl)-1,4'-bipiperidin-1'-yl]carbonyl}-5-[4-(trifluoromethyl)phenyl]-2-thienyl}carbamate obtained in Reference Example 35 (136 mg, 0.209 mmol), the title compound (94.6 mg, yield 82.3%) was obtained.
EI(pos) 551.1 [M+H]⁺

### Reference Example 37 tert-butyl (6-chloro-4-{[3-(morpholin-4-ylcarbonyl)-1,4'-bipiperidin-1'-yl]carbonyl}pyridin-3-yl)carbamate

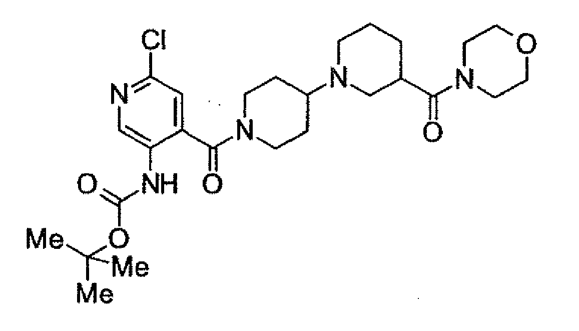

In the same manner as in Reference Example 1 and using 5-[(tert-butoxycarbonyl)amino]-2-chloroisonicotinic acid (569 mg, 2.09 mmol) and 3-(morpholin-4-ylcarbonyl)-1,4'-bipiperidine dihydrochloride (704 mg, 1.99 mmol) and triturating with diisopropyl ether and hexane, the title compound (685 mg, yield 64%) was obtained.
EI(pos) 536.1 [M+H]⁺

### Reference Example 38 tert-butyl (6-[4-(methylsulfonyl)phenyl]-4-{[3-(morpholin-4-ylcarbonyl)-1,4'-bipiperidin-1'-yl]carbonyl}pyridin-3-yl)carbamate

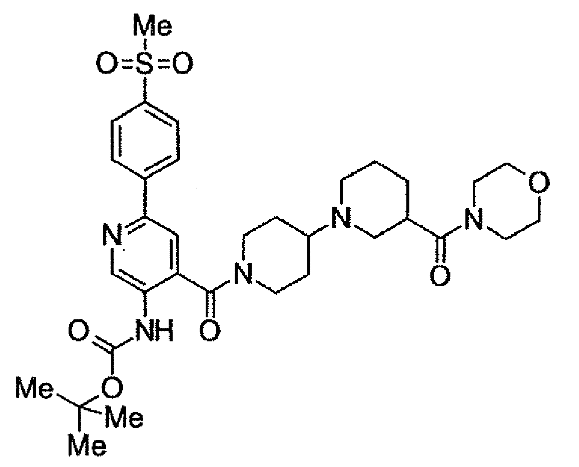

In the same manner as in Reference Example 13 and using tert-butyl (6-chloro-4-{[3-(morpholin-4-ylcarbonyl)-1,4'-bipiperidin-1'-yl]carbonyl}pyridin-3-yl)carbamate obtained in Reference Example 37 (200 mg, 0.373 mmol) and [4-(methylsulfonyl)phenyl]boronic acid (149 mg, 0.746 mmol) and triturating with diisopropyl ether, the title compound (182 mg, yield 74%) was obtained.
EI(pos) 656.3 [M+H]⁺

### Reference Example 39 6-[4-(methylsulfonyl)phenyl]-4-{[3-(morpholin-4-ylcarbonyl)-1,4'-bipiperidin-1'-yl]carbonyl}pyridine-3-amine

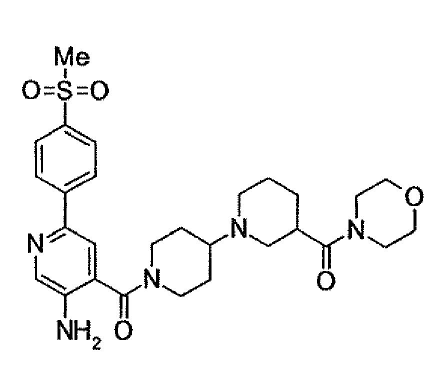

In the same manner as in Reference Example 24 and using tert-butyl (6-[4-(methylsulfonyl)phenyl]-4-{[3-(morpholin-4-ylcarbonyl)-1,4'-bipiperidin-1'-yl]carbonyl}pyridin-3-yl)carbamate obtained in Reference Example 38 (178 mg, 0.271 mmol) and triturating with diisopropyl ether, the title compound (121 mg, yield 80%) was obtained.
EI(pos) 556.0 [M+H]⁺

### Reference Example 40 1'-benzyl-1,4'-bipiperidine-3-carboxylic acid dihydrochloride

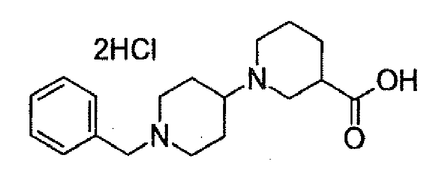

Ethyl 1'-benzyl-1,4'-bipiperidine-3-carboxylate (71.1 g, 0.215 mol) was dissolved in concentrated hydrochloric acid (500 mL), and the solution was refluxed with stirring for 16 hr. After completion of the reaction, the mixture was concentrated under reduced pressure, and triturated with tetrahydrofuran.
The obtained precipitate was collected by filtration, and washed with diisopropyl ether to give the title compound (76.7 mg, yield 95%) as a powder. The compound was used for Reference Example 41 without purification.

### Reference Example 41 1'-benzyl-3-(pyrrolidin-1-ylcarbonyl)-1,4'-bipiperidine

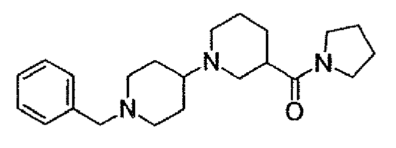

In the same manner as in Reference Example 1 and using 1'-benzyl-1,4'-bipiperidine-3-carboxylic acid dihydrochloride obtained in Reference Example 40 (2.00 g, 5.33 mmol) and pyrrolidine (0.677 mL, 7.99 mmol), the title compound (1.69 g, yield 90%) was obtained. The compound was used for Reference Example 42 without purification.

### Reference Example 42 3-(pyrrolidin-1-ylcarbonyl)-1,4'-bipiperidine dihydrochloride

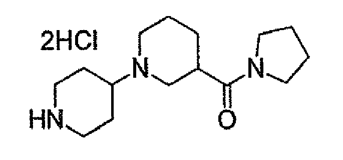

To 1'-benzyl-3-(pyrrolidin-1-ylcarbonyl)-1,4'-bipiperidine obtained in Reference Example 41 (1.69 g, 4.75 mmol), 4N hydrogen chloride-ethyl acetate (3.57 mL, 14.3 mmol) and 10% palladium carbon (1 g) was added methanol (25 mL), and the mixture was stirred at room temperature for 16 hr under a hydrogen atmosphere. The reaction mixture was filtered through celite, and the solvent was evaporated under reduced pressure to give the title compound (1.61 g, quantitative) as an oil. The compound was used for Reference Example 43 without purification.

Reference Example 43 3-{[3-(pyrrolidin-1-ylcarbonyl)-1,4'-bipiperidin-1'-yl]carbonyl}-1-benzothiophen-2-amine

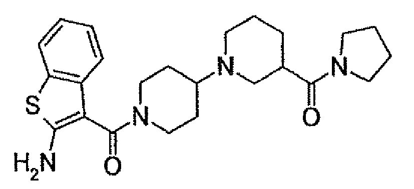

In the same manner as in Reference Example 1 and using 3-(pyrrolidin-1-ylcarbonyl)-1,4'-bipiperidine dihydrochloride obtained in Reference Example 42 (554 mg, 1.64 mmol) and 2-amino-1-benzothiophene-3-carboxylic acid (288 mg, 1.49 mmol) and triturating with diisopropyl ether, the title compound (357 mg, yield 54%) was obtained.
EI(pos) 441.0 [M+H]⁺

### Reference Example 44 1'-benzyl-3-(piperidin-1-ylcarbonyl)-1,4'-bipiperidine

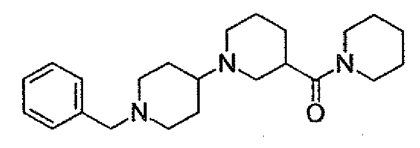

In the same manner as in Reference Example 1 and using 1'-benzyl-1,4'-bipiperidine-3-carboxylic acid dihydrochloride obtained in Reference Example 40 (2.00 g, 5.33 mmol) and piperidine (0.79 mL, 7.99 mmol), the title compound (1.97 g, quantitative) was obtained. The compound was used for Reference Example 45 without purification.

### Reference Example 45 3-(piperidin-1-ylcarbonyl)-1,4'-bipiperidine dihydrochloride

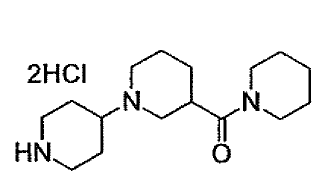

To 1'-benzyl-3-(piperidin-1-ylcarbonyl)-1,4'-bipiperidine obtained in Reference Example 44 (1.97 g, 5.33 mmol), 4N hydrogen chloride-ethyl acetate (4.0 mL, 16.0 mmol) and 10% palladium carbon (1 g) was added methanol (27 mL, and the mixture was stirred at room temperature for 16 hr under a hydrogen atmosphere. The reaction mixture was filtered through celite, and the solvent was evaporated under reduced pressure to give the title compound (1.88 g, quantitative) as an oil. The compound was used for Reference Example 46 without purification.

### Reference Example 46 3-{[3-(piperidin-1-ylcarbonyl)-1,4'-bipiperidin-1'-yl]carbonyl]-1-benzothiophen-2-amine

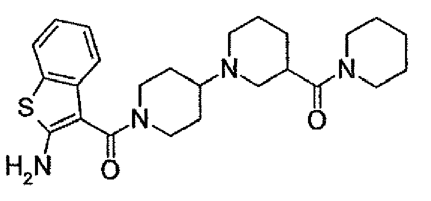

In the same manner as in Reference Example 1 and using 3-(piperidin-1-ylcarbonyl)-1,4'-bipiperidine dihydrochloride obtained in Reference Example 45 (575 mg, 1.63 mmol) and 2-amino-1-benzothiophene-3-carboxylic acid (287 mg, 1.48 mmol) and triturating with diisopropyl ether, the title compound (316 mg, yield 47%) was obtained.
EI(pos) 455.1 [M+H]⁺

### Reference Example 47 7-methoxy-3-{[3-(piperidin-1-ylcarbonyl)-1,4'-bipiperidin-1'-yl]carbonyl}-1-benzothiophen-2-amine

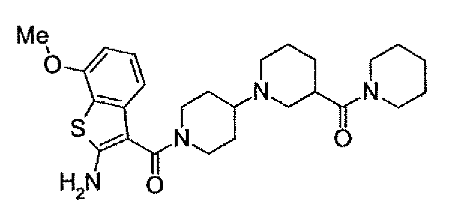

In the same manner as in Reference Example 1 and using 3-(piperidin-1-ylcarbonyl)-1,4'-bipiperidine dihydrochloride obtained in Reference Example 45 (418 mg, 1.19 mmol) and 2-amino-7-methoxy-1-benzothiophene-3-carboxylic acid obtained in Reference Example 32 (265 mg, 1.19 mmol) and triturating with diisopropyl ether and hexane, the title compound (341 mg, yield 59%) was obtained.
EI(pos) 485.1 [M+H]⁺

### Reference Example 48 benzyl 3-{[ethyl(isopropyl)amino]carbonyl}piperidine-1-carboxylate

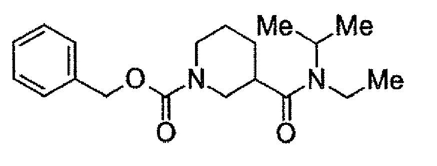

To a solution of 1-[(benzyloxy)carbonyl]piperidine-3-carboxylic acid (3.00 g, 11.4 mmol) and DMF (2 drops) in THF (60 mL) was added oxalyl chloride (1.47 mL, 17.1 mmol) under ice-cooling, and the mixture was concentrated under reduced pressure after 1 hr. The obtained residue was dissolved in THF (60 mL), ethylisopropylamine (4.14 mL, 34.2 mmol) was added under ice-cooling, and the mixture was stirred for 30 min. The solvent was evaporated under reduced pressure, ethyl acetate was added to the residue, and the mixture was washed with 1N hydrochloric acid, 10% potassium carbonate and saturated brine, and dried over anhydrous sodium sulfate. The solution was purified by asic silica gel to give the title compound (3.79 g, quantitative). The compound was used for Reference Example 49 without purification.

### Reference Example 49 tert-butyl 3-{[ethyl(isopropyl)amino]carbonyl}-1,4'-bipiperidine-1'-carboxylate

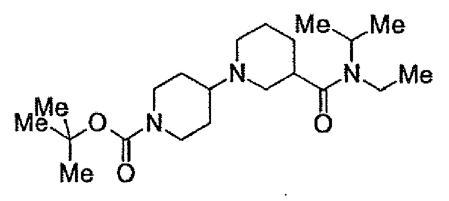

A suspension of benzyl 3-{[ethyl(isopropyl)amino]carbonyl}piperidine-1-carboxylate obtained in Reference Example 48 (3.79 g, 11.4 mmol) and 10% palladium carbon (2 g) in THF (60 mL) was stirred for 16 hr under a hydrogen atmosphere. The catalyst was filtered off, and the filtrate was concentrated under reduced pressure. To a solution of the obtained oil and tert-butyl 4-oxopiperidine-1-carboxylate (2.27 g, 11.4 mmol) in THF (60 mL) was added sodium triacetoxyborohydride (3.62 g, 17.1 mmol), and the mixture was stirred at room temperature for 16 hr. The solvent was evaporated under reduced pressure, and ethyl acetate was added to the residue. The mixture was washed with 10% potassium carbonate and saturated brine, and dried over anhydrous sodium sulfate. The solution was passed through basic silica gel, and purified by silica gel column chromatography (eluent; ethyl acetate:methanol=1:0 to 9:1) to give the title compound (1.71 g, yield 39.2%).
EI(pos) 382.2 [M+H]⁺

### Reference Example 50 N-ethyl-N-isopropyl-1,4'-bipiperidine-3-carboxamide dihydrochloride

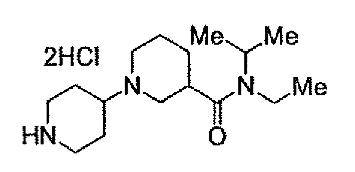

To tert-butyl 3-{[ethyl(isopropyl)amino]carbonyl}-1,4'-bipiperidine-1'-carboxylate obtained in Reference Example 49 (1.70 g, 4.46 mmol) was added 4N hydrogen chloride-ethyl acetate (20 mL), and the mixture was concentrated under reduced pressure after 1 hr to give the title compound (1.58 g, quantitative). The compound was used for Reference Example 51 without purification.

### Reference Example 51 1'-[(2-amino-7-methoxy-1-benzothien-3-yl)carbonyl]-N-ethyl-N-isopropyl-1,4'-bipiperidine-3-carboxamide

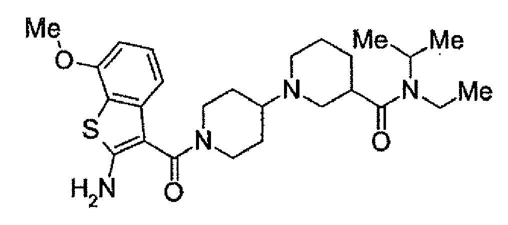

In the same manner as in Reference Example 1 and using N-ethyl-N-isopropyl-1,4'-bipiperidine-3-carboxamide dihydrochloride obtained in Reference Example 50 (381 mg, 1.08 mmol) and 2-amino-7-methoxy-1-benzothiophene-3-carboxylic acid obtained in Reference Example 32 (240 mg, 1.08 mmol) and triturating with diisopropyl ether and hexane, the title compound (381 mg, yield 73%) was obtained.
EI(pos) 487.5 [M+H]⁺

### Reference Example 52 ethyl 3-amino-6-methylthieno[2,3-b]pyridine-2-carboxylate

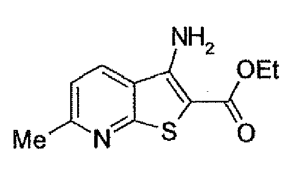

To a solution of 2-chloro-6-methylnicotinonitrile (44.0 g, 0.289 mol) and ethyl thioglycolate (35.0 mL, 0.318 mol) in DMF (500 mL) was added sodium ethoxide (21.7 g, 0.318 mol), and the mixture was stirred at room temperature for 30 min. Sodium ethoxide (5.00 g, 73.5 mmol) was added again, and the mixture was stirred at room temperature for 30 min. Water was added to the reaction mixture, and the precipitated solid was collected by filtration, washed with water, and dried to give the title compound (66.4 g, yield 97%) as a yellow solid. ¹H NMR (CDCl₃) δ1.39 (3H, t, J = 7.2 Hz), 2.68 (3H, s), 4.36 (2H, q, J = 7.2 Hz), 5.89 (2H, br), 7.16 (1H, d, J = 8.3 Hz), 7.81 (1H, d, J = 8.3 Hz).

### Reference Example 53 ethyl 3-bromo-6-methylthieno[2,3-b]pyridine-2-carboxylate

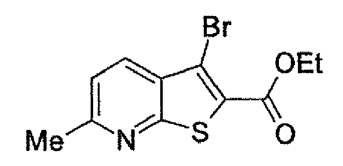

Ethyl 3-amino-6-methylthieno[2,3-b]pyridine-2-carboxylate Reference obtained in Example 52 (36.1 g, 0.153 mol) was added to the mixture of copper(II) bromide (37.5 g, 0.168 mol) and tert-butyl nitrite (23.6 mL, 0.199 mol) in acetonitrile (350 mL) over 2 hr under water-cooling, and the mixture was stirred for 1 hr. 1N Hydrochloric acid (700 mL) was slowly added to the reaction mixture, and the resulting precipitate was collected by filtration, and washed with water. The solid was dissolved in THF, and the solution was diluted with ethyl acetate, washed with 1N hydrochloric acid and saturated brine, and dried over anhydrous sodium sulfate (solution A). The previous filtrate was extracted with ethyl acetate, and the extract was washed with saturated brine, and dried over anhydrous sodium sulfate. The residue was subjected to basic silica gel column chromatography (eluent; ethyl acetate) to give crude product (about 5 g). The produce was combined with solution A, and the mixture was again subjected to basic silica gel column chromatography (ethyl acetate), and crystallized from hexane to give the title compound (30.5 g, yield 66%) as a pale-yellow solid.
¹H NMR (CDCl₃) δ1.35 (3H, t, J = 7.1 Hz), 2.67 (3H, s), 4.39 (2H, q, J = 7.1 Hz), 7.54 (1H, d, J = 8.3 Hz), 8.20 (1H, d, J = 8.3 Hz).

### Reference Example 54 3-bromo-6-methylthieno[2,3-b]pyridine-2-carboxylic acid

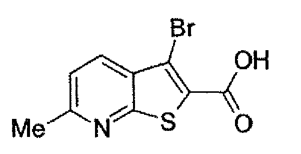

To a solution of ethyl 3-bromo-6-methylthieno[2,3-b]pyridine-2-carboxylate obtained in Reference Example 53 (79.2 g, 0.264 mol) in ethanol (250 mL) was added 2N aqueous sodium hydroxide solution (264 mL, 0.527 mol), and the mixture was stirred at room temperature for 16 hr. The reaction mixture was diluted with water (1 L), and adjusted with 1N hydrochloric acid (530 mL) to pH 5 to 6, and the precipitated solid was collected by filtration, and washed with water. The obtained solid was suspended in acetone, collected by filtration, washed successively with acetone and diisopropyl ether to give the title compound (68.5 g, yield 95%). ¹H NMR (CDCl₃) δ2.66 (3H, s), 7.52 (1H, d, J = 8.5 Hz), 8.18 (1H, d, J = 8.3 Hz), 14.06 (1H, br).

### Reference Example 55 tert-butyl (3-bromo-6-methylthieno[2,3-b]pyridin-2-yl)carbamate

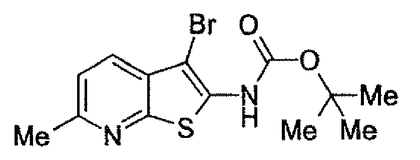

A solution of 3-bromo-6-methylthieno[2,3-b]pyridine-2-carboxylic acid obtained in Reference Example 54 (15.0 g, 55.2 mmol), diphenylphosphoryl azide (13.1 mL, 60.6 mmol), triethylamine (11.6 mL, 82.8 mmol) in tert-butanol (100 mL) was heated at 90°C for 15 hr. The reaction mixture was diluted with ethyl acetate, and the solution was washed successively with saturated aqueous sodium hydrogen carbonate solution and saturated brine, and dried anhydrous magnesium sulfate. The solvent was evaporated under reduced pressure, and the obtained residue was purified by silica gel column chromatography (ethyl acetate:hexane=1:19 to 3:7) to give the title compound (16.3 g, yield 86%) as a white solid. ¹H NMR (DMSO-d₆) δ1.51 (9H, s), 2.58 (3H, s), 7.32 (1H, d, J = 8.4 Hz), 7.78 (1H, d, J = 8.4 Hz), 10.12 (1H, s).

### Reference Example 56 2-[(tert-butoxycarbonyl)amino]-6-methylthieno[2,3-b]pyridine-3-carboxylic acid

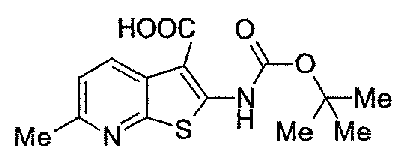

To a solution of tert-butyl (3-bromo-6-methylthieno[2,3-b]pyridin-2-yl)carbamate obtained in Reference Example 55 (9.07 g, 26.4 mmol) in dry THF (88 mL) added dropwise 1.6 M n-butyllithium hexane solution (38 mL, 60.7 mmol) at -78°C under a nitrogen atmosphere, and the mixture was stirred at the same temperature for 1 hr. Dry carbon dioxide gas vaporized from solid carbon dioxide was bubbled at 0-10°C. The reaction mixture was diluted with water and ethyl acetate, 1N hydrochloric acid was added, and the precipitated solid was collected by filtration. The obtained solid was purified by repeatedly suspending and filtering using water and acetonitrile/diethyl ether (1:1) successively to give the title compound (6.51 g, yield 80%) as white crystals. melting point 162-163°C
EI(pos) 309 [M+H]⁺
¹H NMR (DMSO-d₆) δ1.53 (9H, s), 2.50 (3H, s), 7.29 (1H, d, J = 8.3 Hz), 8.37 (1H, d, J = 8.3 Hz), 11.26 (1H, br).

### Reference Example 57 tert-butyl {3-[(3-{[ethyl(isopropyl)amino]carbonyl}-1,4'-bipiperidin-1'-yl)carbonyl]-6-methylthieno[2,3-b]pyridin-2-yl)carbamate

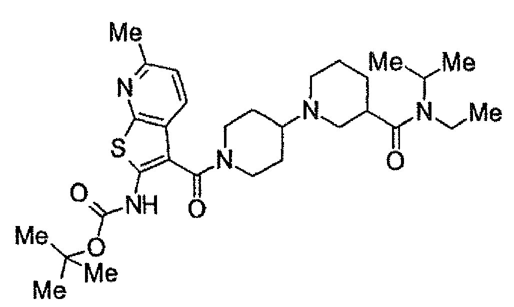

In the same manner as in Reference Example 1 and using N-ethyl-N-isopropyl-1,4'-bipiperidine-3-carboxamide dihydrochloride obtained in Reference Example 50 (335 mg, 0.945 mmol) and 2-[(tert-butoxycarbonyl)amino]-6-methylthieno[2,3-b]pyridine-3-carboxylic acid obtained in Reference Example 56 (292 mg, 0.945 mmol), the title compound (192 mg, yield 36%) was obtained as an oil.
EI(pos) 572.1 [M+H]⁺

### Reference Example 58 1'-[(2-amino-6-methylthieno[2,3-b]pyridin-3-yl)carbonyl]-N-ethyl-N-isopropyl-1,4'-bipiperidine-3-carboxamide

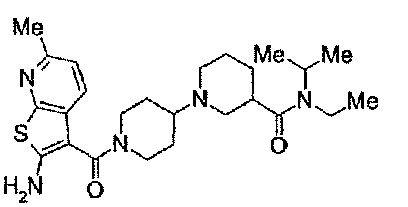

In the same manner as in Reference Example 24 and using tert-butyl {3-[(3-{[ethyl(isopropyl)amino]carbonyl}-1,4'-bipiperidin-1'-yl)carbonyl]-6-methylthieno[2,3-b]pyridin-2-yl}carbamate obtained in Reference Example 57 (192 mg, 0.336 mmol), the title compound (158 mg, yield 99%) was obtained as an oil.
EI(pos) 472.1 [M+H]⁺

### Reference Example 59 tert-butyl 3-{[(2R,6S)-2,6-dimethylpiperidin-1-yl]carbonyl}-1,4'-bipiperidine-1'-carboxylate

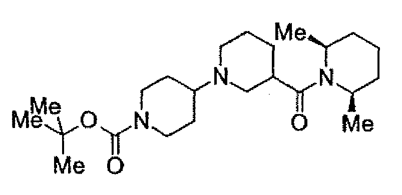

In the same manner as in Reference Examples 48 and 49 and using 1-[(benzyloxy)carbonyl]piperidine-3-carboxylic acid (3.00 g, 11.4 mmol), the title compound (3.12 g, yield 67%) was obtained.
EI(pos) 408.2 [M+H]⁺

### Reference Example 60 3-{[(2R,6S)-2,6-dimethylpiperidin-1-yl]carbonyl}-1,4'-bipiperidine dihydrochloride

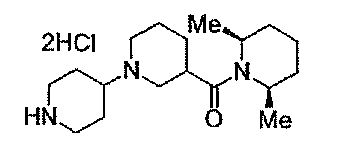

In the same manner as in Reference Example 50 and using tert-butyl 3-{[(2R,6S)-2,6-dimethylpiperidin-1-yl]carbonyl}-1,4'-bipiperidine-1'-carboxylate obtained in Reference Example 59 (3.12 g, 7.65 mmol), the title compound (2.91 g, quantitative) was obtained. The compound was used for the next step without purification.

### Reference Example 61 3-[(3-{[(2R,6S)-2,6-dimethylpiperidin-1-yl]carbonyl)-1,4'-bipiperidin-1'-yl) carbonyl]-1-benzothiophen-2-amine

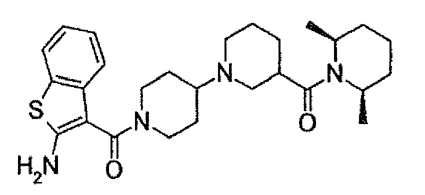

In the same manner as in Reference Example 1 and using 3-{[(2R,6S)-2,6-dimethylpiperidin-1-yl]carbonyl}-1,4'-bipiperidine dihydrochloride obtained in Reference Example 60 (260 mg, 0.683 mmol) and 2-amino-1-benzothiophene-3-carboxylic acid (132 mg, 0.683 mmol) and triturating with diisopropyl ether and hexane, the title compound (253 mg, yield 77%) was obtained.
EI(pos) 483.1 [M+H]⁺

### Reference Example 62 1-(tert-butoxycarbonyl)-3-methylpiperidine-3-carboxylic acid

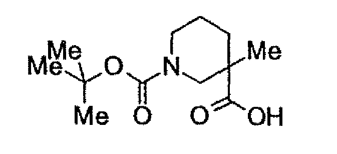

In the same manner as in Reference Example 19 and using 1-tert-butyl 3-ethyl 3-methylpiperidine-1,3-dicarboxylate (4.56 g, 16.8 mmol) and crystallizing from hexane, the title compound (3.69 mg, yield 90%) was obtained. The compound was used for Reference Example 62 without purification.

### Reference Example 62 tert-butyl 3-[(diethylamino)carbonyl]-3-methylpiperidine-1-carboxylate

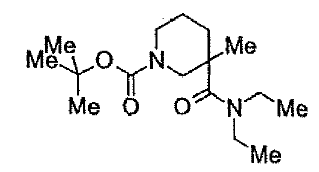

In the same manner as in Reference Example 1 and using 1-(tert-butoxycarbonyl)-3-methylpiperidine-3-carboxylic acid obtained in Reference Example 61 (2.00 g, 8.22 mmol) and diethylamine (1.28 mL, 12.3 mmol), the title compound (1.63 g, yield 67%) was obtained as an oil.
EI(pos) 199.2 [M-Boc+H]⁺

### Reference Example 63 N,N-diethyl-3-methylpiperidine-3-carboxamide hydrochloride

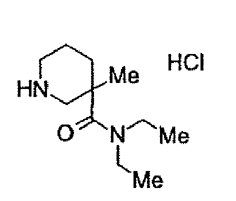

In the same manner as in Reference Example 6 and using tert-butyl 3-[(diethylamino)carbonyl]-3-methylpiperidine-1-carboxylate obtained in Reference Example 62 (1.63 g, 5.46 mmol), the title compound (1.28 g, quantitative) was obtained as an oil. The compound was used for Reference Example 64 without purification.

### Reference Example 64 tert-butyl 3-[(diethylamino)carbonyl]-3-methyl-1,4'-bipiperidine-1'-carboxylate

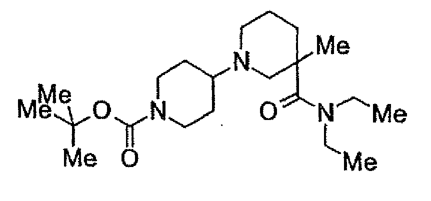

To a solution of N,N-diethyl-3-methylpiperidine-3-carboxamide hydrochloride obtained in Reference Example 63 (1.28 g, 5.45 mmol), tert-butyl 4-oxopiperidine-1-carboxylate (1.19 g, 6.00 mmol), triethylamine (0.76 mL, 5.45 mmol) and acetic acid (2 ml) in THF (20 ml) was added sodium triacetoxyborohydride (1.73 g, 8.18 mmol), and the mixture was stirred at room temperature for 3 days. Ethyl acetate was added to the reaction mixture, and the mixture was washed with aqueous potassium carbonate solution and saturated brine, and dried over anhydrous sodium sulfate. The solvent was evaporated under reduced pressure, and the obtained residue was purified by silica gel column chromatography (eluent; hexane-ethyl acetate:9:1 to 3:1) to give the title compound (1.37 g, yield 66%) as an oil.
EI(pos) 382.2 [M+H]⁺

### Reference Example 65 N,N-diethyl-3-methyl-1,4'-bipiperidine-3-carboxamide dihydrochloride

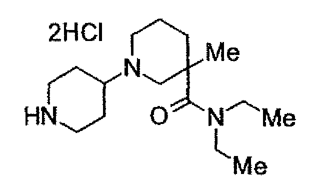

In the same manner as in Reference Example 6 and using tert-butyl 3-[(diethylamino)carbonyl]-3-methyl-1,4'-bipiperidine-1'-carboxylate obtained in Reference Example 64 (1.37 g, 3.59 mmol), the title compound (1.27 g, quantitative) was obtained as an oil. The compound was used for Reference Example 66 without purification.

### Reference Example 66 1'-[(2-amino-1-benzothien-3-yl}carbonyl]-N,N-diethyl-3-methyl-1,4'-bipiperidine-3-carboxamide

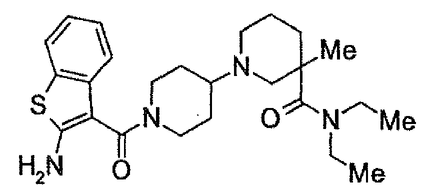

In the same manner as in Reference Example 1 and using ) N,N-diethyl-3-methyl-1,4'-bipiperidine-3-carboxamide dihydrochloride obtained in Reference Example 65 (700 mg, 1.98 mmol) and 2-amino-1-benzothiophene-3-carboxylic acid (382 mg, 1.98 mmol), the title compound (295 mg, yield 33%) was obtained as an oil.
EI(pos) 457.1 [M+H]⁺

### Example 1 N-ethyl-N'-[3-({4-[3-(morpholin-4-ylcarbonyl)-4-(trifluoroacetyl)piperazin-1-yl]piperidin-1-yl}carbonyl)-1-benzothiophen-2-yl]urea

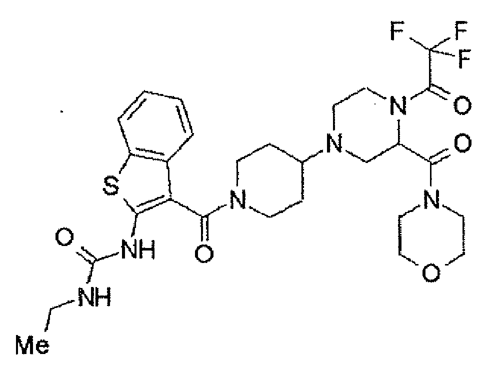

3-({4-[3-(Morpholin-4-ylcarbonyl)-4-(trifluoroacetyl)piperazin-1-yl]piperidin-1-yl}carbonyl)-1-benzothiophen-2-amine obtained in Reference Example 7 (200 mg, 0.361 mmol) was dissolved in pyridine (1 mL), ethyl isothiocyanate (0.057 mL, 0.723 mmol) was added, and the mixture was stirred at 60°C for 16 hr. The solvent was evaporated under reduced pressure, and the residue was purified by NH-silica gel column chromatography (eluent; hexane-ethyl acetate=1:1 to ethyl acetate) and triturating with diisopropyl ether to give the title compound (181 mg, yield 80.1%).
EI(pos) 625.0 [M+H]⁺

### Example 2 N-ethyl-N'-[3-({4-[3-(morpholin-4-ylcarbonyl)piperazin-1-yl]piperidin-1-yl}carbonyl)-1-benzothiophen-2-yl]urea

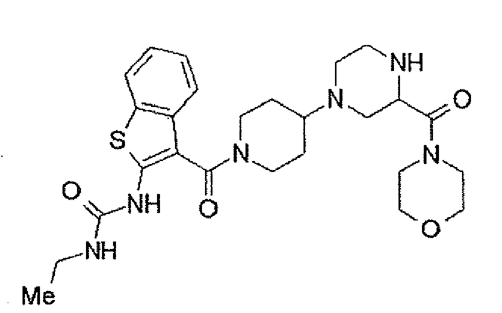

N-Ethyl-N'-[3-({4-[3-(morpholin-4-ylcarbonyl)-4-(trifluoroacetyl)piperazin-1-yl]piperidin-1-yl}carbonyl)-1-benzothiophen-2-yl]urea obtained in Example 1 (178 mg, 0.285 mmol) was dissolved in methanol (1.5 mL), an aqueous solution (0.5 mL) of potassium carbonate (118 mg, 0.855 mmol) was added, and the mixture was stirred at room temperature for 16 hr. The mixture was diluted with ethyl acetate, washed with saturated brine, and dried over anhydrous sodium sulfate. The solvent was evaporated under reduced pressure, and the obtained residue was purified by NH-silica gel column chromatography (eluent; ethyl acetate to ethyl acetate:methanol=10:1) and triturating with diisopropyl ether to give the title compound (121 mg, yield 80.3%) was obtained.
EI(pos) 529.0 [M+H]⁺

### Example 3 N-[3-({4-[3-(morpholin-4-ylcarbonyl)piperazin-1-yl]piperidin-1-yl}carbonyl)-1-benzothiophen-2-yl]urea

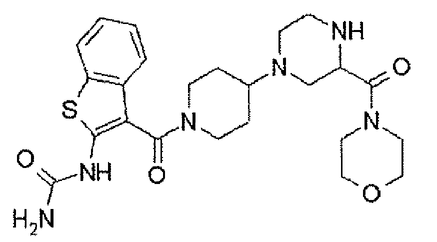

N-Ethyl-N'-[3-({4-[3-(morpholin-4-ylcarbonyl)-4-(trifluoroacetyl)piperazin-1-yl]piperidin-1-yl}carbonyl)-1-benzothiophen-2-yl]urea obtained in Example 1 (200 mg, 0.361 mmol) was dissolved in tetrahydrofuran (1 mL), trichloroacetyl isocyanate (0.086 mL, 0.723 mmol) was added under ice-cooling, and the mixture was stirred for 1 hr. 7N Ammonia-methanol (1 mL) was added, and the mixture was stirred at room temperature for 16 hr. The solvent was evaporated under reduced pressure, and the obtained residue was purified by NH-silica gel column chromatography (eluent; ethyl acetate to ethyl acetate:methanol=4:1) and triturating with diisopropyl ether to give the title compound (115 mg, yield 63.4%).
EI(pos) 501.0 [M+H]⁺

### Example 4 N,N-diethyl-4-{1-[(2-{[(ethylamino)carbonyl]amino}-1-benzothiophen-3-yl)carbonyl]piperidin-4-yl}morpholine-2-carboxamide

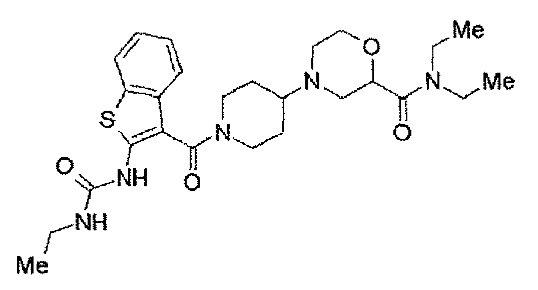

In the same manner as in Example 1 and using 4-{1-[(2-amino-1-benzothiophen-3-yl)carbonyl]piperidin-4-yl}-N,N-diethylmorpholine-2-carboxamide obtained in Reference Example 12 (150 mg, 0.337 mmol), the title compound (137 mg, yield 79.0%) was obtained.
EI(pos) 516.0 [M+H]⁺

### Example 5 4-[1-({2-[(aminocarbonyl)amino]-1-benzothiophen-3-yl)carbonyl)piperidin-4-yl]-N,N-diethylmorpholine-2-carboxamide

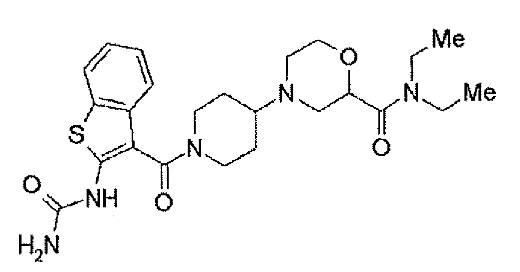

In the same manner as in Example 3 and using 4-{1-[(2-amino-1-benzothiophen-3-yl)carbonyl]piperidin-4-yl}-N,N-diethylmorpholine-2-carboxamide obtained in Reference Example 12 (232 mg, 0.522 mmol), the title compound (28.6 mg, yield 11.3%) was obtained.
EI(pos) 488.0 [M+H]⁺

### Example 6 N,N-diethyl-3-{1-[(2-{[(ethylamino)carbonyl]aminol-1-benzothiophen-3-yl)carbonyl]piperidin-4-yl}benzamide

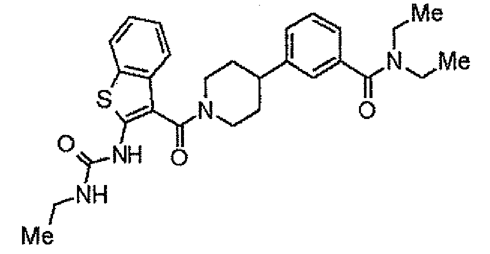

In the same manner as in Example 1 and using 3-{1-[(2-amino-1-benzothiophen-3-yl)carbonyl]piperidin-4-yl}-N,N-diethylbenzamide obtained in Reference Example 16 (100 mg, 0.230 mmol), the title compound (102 mg, yield 88.3%) was obtained. EI(pos) 507.0 [M+H]⁺

### Example 7 N,N-diethyl-1'-[(2-{[(ethylamino)carbonyl]amino}-1-benzothiophen-3-yl)carbonyl]-1,4'-bipiperidine-3-carboxamide

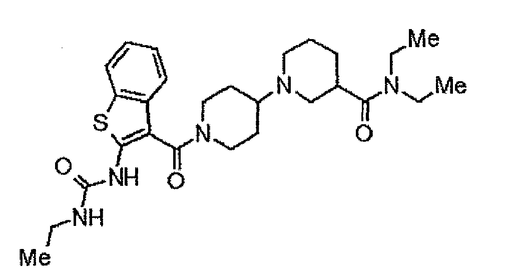

In the same manner as in Example 3 and using 1'-[(2-amino-1-benzothiophen-3-yl)carbonyl]-N,N-diethyl-1,4'-bipiperidine-3-carboxamide obtained in Reference Example 17 (172 mg, 0.389 mmol), the title compound (124 mg, yield 62.1%) was obtained. EI(pos) 514.1 [M+H]⁺

### Example 8 N,N-diethyl-1'-[(2-{[(ethylamino)carbonyl]amino}-5-phenyl-3-thienyl)carbonyl]-1,4'-bipiperidine-3-carboxamide

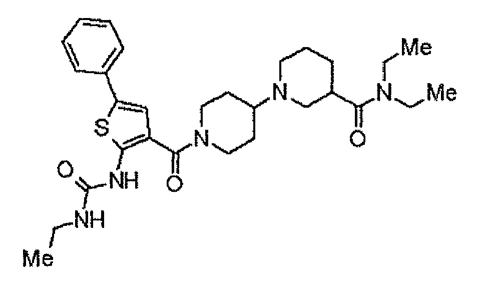

In the same manner as in Example 1 and using 1'-[(2-amino-5-phenyl-3-thienyl)carbonyl]-N,N-diethyl-1,4'-bipiperidine-3-carboxamide obtained in Reference Example 24 (55.0 mg, 0.117 mmol), the title compound (22.4 mg, yield 35.4%) was obtained. EI(pos) 540.1 [M+H]⁺

### Example 9 1'-({2-[(aminocarbonyl)amino]-5-phenyl-3-thienyl}carbonyl)-N,N-diethyl-1,4'-bipiperidine-3-carboxamide

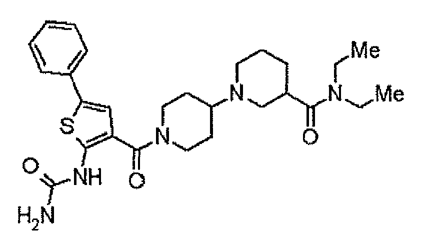

In the same manner as in Example 3 and using 1'-[(2-amino-5-phenyl-3-thienyl)carbonyl]-N,N-diethyl-1,4'-bipiperidine-3-carboxamide obtained in Reference Example 24 (165 mg, 0.352 mmol), the title compound (85.6 mg, yield 47.6%) was obtained. EI(pos) 512.0 [M+H]⁺

### Example 10 N-ethyl-N'-(3-{[3-(morpholin-4-ylcarbonyl)-1,4'-bipiperidin-1'-yl]carbonyl}-1-benzothiophen-2-yl)urea hydrochloride

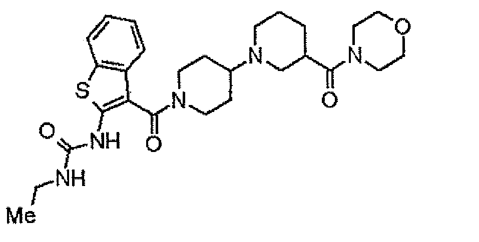

In the same manner as in Example 1, and using 3-{[3-(morpholin-4-ylcarbonyl)-1,4'-bipiperidin-1'-yl]carbonyl}-1-benzothiophen-2-amine obtained in Reference Example 18 (0.50 g, 1.10 mmol), the title compound was obtained as a free base. The free base was converted to a hydrochloride thereof with 4M hydrogen chloride-ethyl acetate solution, and the hydrochloride was recrystallized with ethanol/ethyl acetate to give the title compound (0.28 g, yield 45.1%).
EI(pos) 528.2 [M+H of the free base]⁺

Example 11 N-(3-{[3-(morpholin-4-ylcarbonyl)-1,4'-bipiperidin-1'-yl]carbonyl}-1-benzothiophen-2-yl)urea

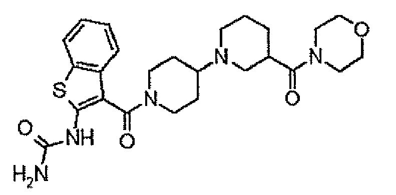

In the same manner as in Example 3 and using 3-{[3-(morpholin-4-ylcarbonyl)-1,4'-bipiperidin-1'-yl]carbonyl}-1-benzothiophen-2-amine obtained in Reference Example 18 (250 mg, 0.548 mmol), the title compound (102 mg, yield 37.2%) was obtained.
EI(pos) 500.0 [M+H]⁺

### Example 12 N-ethyl-N'-(3-{[3-(morpholin-4-ylcarbonyl)-1,4'-bipiperidin-1'-yl]carbonyl}-7-oxo-4,5,6,7-tetrahydro-1-benzothiophen-2-yl)urea

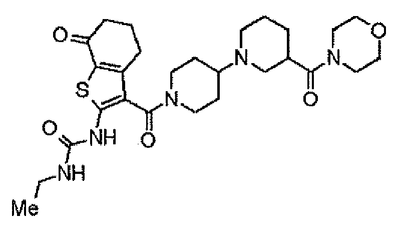

In the same manner as in Example 1 and using 2-amino-3-{[3-(morpholin-4-ylcarbonyl)-1,4'-bipiperidin-1'-yl]carbonyl}-5,6-dihydro-1-benzothiophen-7(4H)-one obtained in Reference Example 20 (20 mg, 0.042 mmol), the title compound (10 mg, yield 43.5%) was obtained.
EI(pos) 546.1 [M+H]⁺

Example 13 N-ethyl-N'-(3-{[3-(morpholin-4-ylcarbonyl)-1,4'-bipiperidin-1'-yl]carbonyl}-5-phenyl-2-thienyl)urea

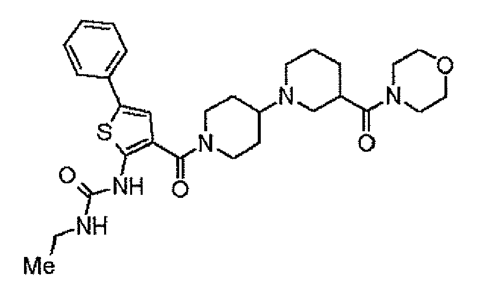

In the same manner as in Example 1 and using 3-{[3-(morpholin-4-ylcarbonyl)-1,4'-bipiperidin-1'-yl]carbonyl}-5-phenylthiophen-2-amine obtained in Reference Example 26 (163 mg, 0.338 mmol), the title compound (151 mg, yield 81.0%) was obtained.
EI(pos) 554.2 [M+H]⁺

### Example 14 N-(3-{[3-(morpholin-4-ylcarbonyl)-1,4'-bipiperidin-1'-yl] carbonyl}-5-phenyl-2-thienyl)urea

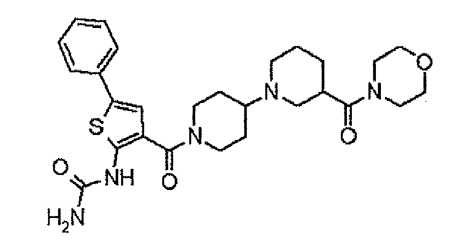

In the same manner as in Example 3 and using 3-{[3-(morpholin-4-ylcarbonyl)-1,4'-bipiperidin-1'-yl]carbonyl)-5-phenylthiophen-2-amine obtained in Reference Example 26 (289 mg, 0.599 mmol), the title compound (160 mg, yield 50.9%) was obtained.
EI(pos) 526.1 [M+H]⁺

Example 15 N-ethyl-N'-(4-{[3-(morpholin-4-ylcarbonyl)-1,4'-bipiperidin-1'-yl]carbonyl}-2-phenyl-1,3-thiazol-5-yl)urea

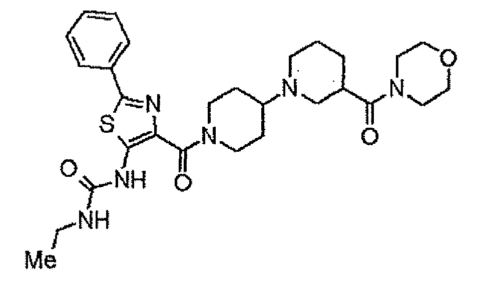

In the same manner as in Example 1 and using 4-{[3-(morpholin-4-ylcarbonyl)-1,4'-bipiperidin-1'-yl]carbonyl}-2-phenyl-1,3-thiazole-5-amine obtained in Reference Example 28 (150 mg, 0.310 mmol), the title compound (149 mg, yield 86.7%) was obtained.
EI(pos) 555.2 [M+H]⁺

### Example 16 N-(4-{[3-(morpholin-4-ylcarbonyl)-1,4'-bipiperidin-1'-yl]carbonyl}-2-phenyl-1,3-thiazol-5-yl)urea

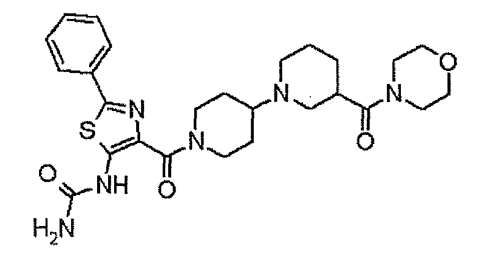

In the same manner as in Example 3 and using 4-{[3-(morpholin-4-ylcarbonyl)-1,4'-bipiperidin-1'-yl]carbonyl}-2-phenyl-1,3-thiazole-5-amine obtained in Reference Example 28 (184 mg, 0.380 mmol), the title compound (129 mg, yield 64.6%) was obtained.
EI(pos) 527.2 [M+H]⁺

Example 17 N-ethyl-N'-{3-{[(3R)-3-(morpholin-4-ylcarbonyl)-1,4'-bipiperidin-1'-yl]carbonyl}-5-[4-(trifluoromethyl)phenyl]-2-thienyl}urea

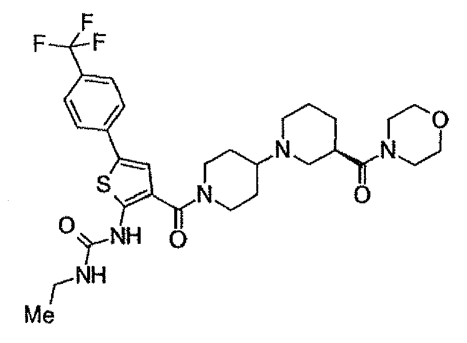

In the same manner as in Example 1 and using 3-{[(3R)-3-(morpholin-4-ylcarbonyl)-1,4'-bipiperidin-1'-yl]carbonyl}-5-[4-(trifluoromethyl)phenyl]thiophen-2-amine obtained in Reference Example 36 (94.6 mg, 0.172 mmol), the title compound (66.3 mg, yield 62.0%) was obtained.
EI(pos) 622.2 [M+H]⁺

### Example 18 N-ethyl-N'-(6-[4-(methylsulfonyl)phenyl]-4-{[3-(morpholin-4-ylcarbonyl)-1,4'-bipiperidin-1'-yl]carbonyl}pyridin-3-yl)urea

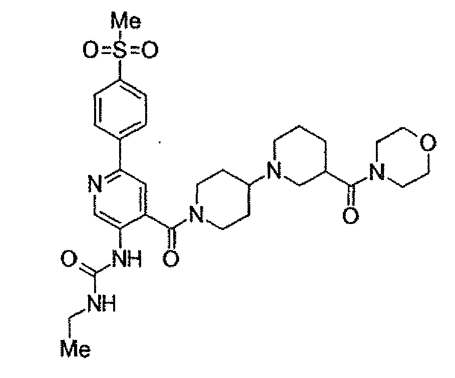

In the same manner as in Example 1 and using 6-[4-(methylsulfonyl)phenyl)-4-{[3-(morpholin-4-ylcarbonyl)-1,4'-bipiperidin-1'-yl]carbonyl}pyridine-3-amine obtained in Reference Example 36 (113 mg, 0.203 mmol), the title compound (36.9 mg, yield 29.1%) was obtained.
EI (pos) 627.1 [M+H]⁺

### Example 19 N-ethyl-N'-(3-{[3-(pyrrolidin-1-ylcarbonyl)-1,4'-bipiperidin-1'-yl]carbonyl)-1-benzothien-2-yl)urea

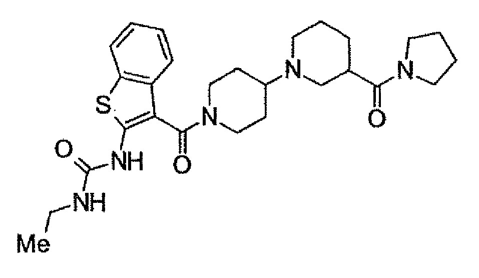

In the same manner as in Example 1 and using 3-{[3-(pyrrolidin-1-ylcarbonyl)-1,4'-bipiperidin-1'-yl]carbonyl}-1-benzothiophen-2-amine obtained in Reference Example 43 (100 mg, 0.227 mmol), the title compound (76.1 mg, yield 65.6%) was obtained.
EI(pos) 512.1 [M+H]⁺

### Example 20 N-ethyl-N'-(3-{[3-(piperidin-1-ylcarbonyl)-1,4'-bipiperidin-1'-yl]carbonyl}-1-benzothien-2-yl)urea

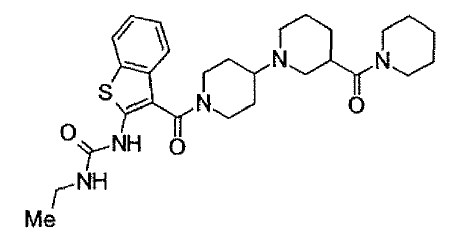

In the same manner as in Example 1 and using 3-{[3-(piperidin-1-ylcarbonyl)-1,4'-bipiperidin-1'-yl]carbonyl}-1-benzothiophen-2-amine obtained in Reference Example 46 (100 mg, 0.220 mmol), the title compound (70.7 mg, yield 60.9%) was obtained.
EI (pos) 526.1 [M+H]⁺

### Example 21 N-(7-methoxy-3-{[3-(piperidin-1-ylcarbnnyl)-1,4'-bipiperidin-1'-yl]carbonyl}-1-benzothien-2-yl)-N'-methylurea

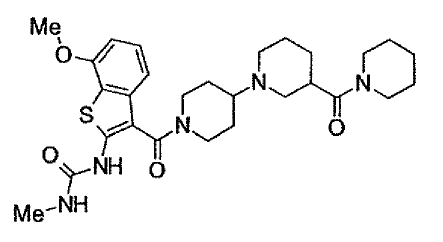

In the same manner as in Example 1 and using 7-methoxy-3-{[3-(piperidin-1-ylcarbonyl)-1,4'-bipiperidin-1'-yl]carbonyl}-1-benzothiophen-2-amine obtained in Reference Example 47 (115 mg, 0.237 mmol), the title compound (94.8 mg, yield 74%) was obtained.
EI(pos) 542.5 [M+H]⁺

### Example 22 N-ethyl-N'-(7-methoxy-3-{[3-(piperidin-1-ylcarbonyl)-1,4'-bipiperidin-1'-yl]carbonyl}-1-benzothien-2-yl)urea

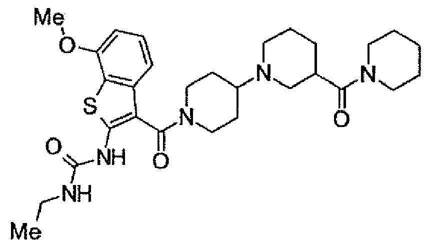

In the same manner as in Example 1 and using 7-methoxy-3-{[3-(piperidin-1-ylcarbonyl)-1,4'-bipiperidin-1'-yl]carbonyl}-1-benzothiophen-2-amine obtained in Reference Example 47 (200 mg, 0.413 mol), the title compound (200 mg, yield 87%) was obtained. EI(pos) 556.1 [M+H]⁺

### Example 23 N-ethyl-N-isopropyl-1'-[(7-methoxy-2-{[(methylamino)carbonyl]aminol-1-benzothien-3-yl)carbonyl]-1,4'-bipiperidine-3-carboxamide

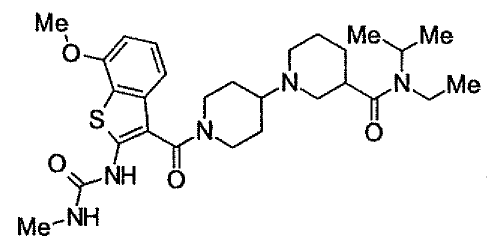

In the same manner as in Example 1 and using 1'-[(2-amino-7-methoxy-1-benzothien-3-yl)carbonyl]-N-ethyl-N-isopropyl-1,4'-bipiperidine-3-carboxamide obtained in Reference Example 51 (186 mg, 0.382 mmol), the title compound (128 mg, yield 62%) was obtained.
EI (pos) 544.5 [M+H]⁺

### Example 24 N-ethyl-1'-[(2-{[(ethylamino)carbonyl)amino}-7-methoxy-1-benzothien-3-yl)carbonyl]-N-isopropyl-1,4'-bipiperidine-3-carboxamide

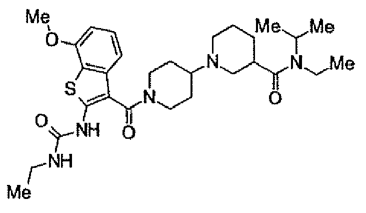

In the same manner as in Example 1 and using 1'-[(2-amino-7-methoxy-1-benzothien-3-yl)carbonyl]-N-ethyl-N-isopropyl-1,4'-bipiperidine-3-carboxamide obtained in Reference Example 51 (117 mg, 0.240 mmol), the title compound (76.3 mg, yield 57%) was obtained.
EI(pos) 558.5 [M+H]⁺

### Example 25 N-ethyl-1'-[(2-{[(ethylamino)carbonyl]amino}-6-methylthieno[2,3-b]pyridin-3-yl)carbonyl]-N-isopropyl-1,4'-bipiperidine-3-carboxamide

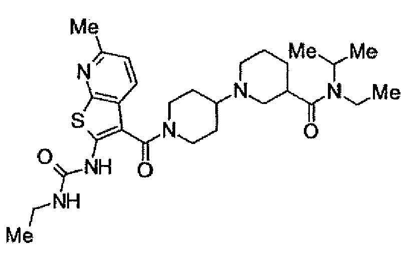

In the same manner as in Example 1 and using 1'-[(2-amino-6-methylthieno[2,3-b]pyridin-3-yl)carbonyl]-N-ethyl-N-isopropyl-1,4'-bipiperidine-3-carboxamide obtained in Reference Example 58 (158 mg, 0.335 mol), the title compound (139 mg, yield 77%) was obtained.
EI (pos) 543.1 [M+H]⁺

### Example 26 N-{3-[(3-{[(2R,6S)-2,6-dimethylpiperidin-1-yl]carbonyl}-1,4'-bipiperidin-1'-yl)carbonyl]-1-benzothien-2-yl}urea

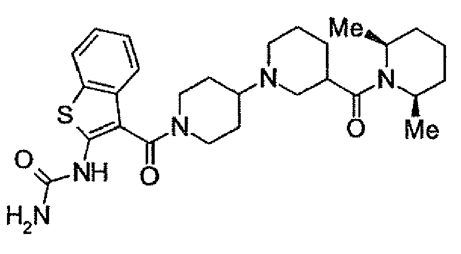

In the same manner as in Example 3 and using 3-[(3-{[(2R,6S)-2,6-dimethylpiperidin-1-yl]carbonyl}-1,4'-bipiperidin-1'-yl)carbonyl]-1-benzothiophen-2-amine obtained in Reference Example 61 (400 mg, 0.829 mmol), the title compound (222 mg, yield 51%) was obtained.
EI(pos) 526.2 [M+H]⁺

### Example 27 N-{3-[(3-{[(2R,6S)-2,6-dimethylpiperidin-1-yl]carbonyl}-1,4'-bipiperidin-1'-yl)carbonyl]-1-benzothien-2-yl}-N'-methylurea

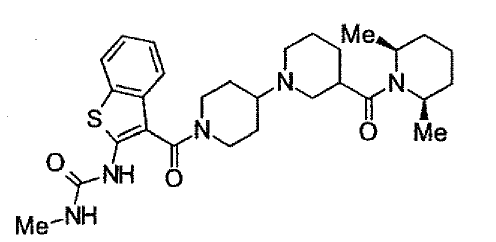

In the same manner as in Example 1 and using 3-[(3-{[(2R,6S)-2,6-dimethylpiperidin-1-yl]carbonyl}-1,4'-bipiperidin-1'-yl)carbonyl]-1-benzothiophen-2-amine obtained in Reference Example 61 (120 mg, 0.249 mol), the title compound (88.8 mg, yield 66%) was obtained.
EI (pos) 540.4 [M+H]⁺

### Example 28 N,N-diethyl-3-methyl-1'-[(2-{E(methylamino)carbonyl]amino}-1-benzothien-3-yl)carbonyl]-1,4-bipiperidine-3-carboxamide

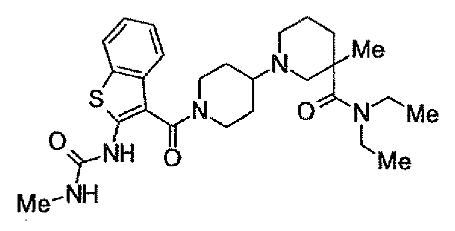

In the same manner as in Example 1 and using 1'-[(2-imino-1-benzothien-3-yl)carbonyl]-N,N-diethyl-3-methyl-1,4'-bipiperidine-3-carboxamide obtained in Reference Example 66 (147 mg, 0.32 mmol), the title compound (38.1 mg, yield 23%) was obtained.
EI(pos) 514.1 [M+H]⁺

### Example 29 1'-({2-[(aminocarbonyl)amino]-1-benzothien-3-yl}carbonyl)-N,N-diethyl-3-methyl-1,4'-bipiperidine-3-carboxamide

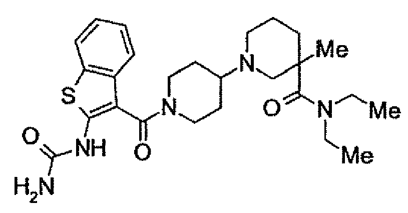

In the same manner as in Example 3 and using 1'-[(2-imino-1-benzothien-3-yl)carbonyl]-N,N-diethyl-3-methyl-1,4'-bipiperidine-3-carboxamide obtained in Reference Example 66 (147 mg, 0.32 mol), the title compound (17.3 mg, yield 11%) was obtained.
EI(pos) 500.1 [M+H]⁺

### Experimental Example 1

The ACC1 inhibitory action of the compound of the present invention was evaluated by the following method.
(1) Cloning of human ACC1 gene and preparation of recombinant Baculovirus
   Human ACC1 gene was cloned by PCR using a human liver cDNA library (Clontech) as a template and Primer 1 and Primer 2 shown below. Primer 1 and Primer 2 were prepared by adding SalI, NotI restriction enzyme recognition sequence based on the information of the base sequence (Genbank Accession U19822) of human ACC1 gene.
   Primer 1 5'AAAAGTCGACCCACCATGGATGAACCTTCTCCCTTGGCCC (SEQ ID NO:1)
   Primer 2 5'AAAAGCGGCCGCCTACGTAGAAGGGGAGTCCATAGTG (SEQ ID NO:2)
   PCR was performed using a Pyrobest DNA polymerase (TAKARA BIO INC.) The obtained PCR product was cloned to pT7 Blue vector (Novagen) and, after confirmation of the base sequence, digested with restriction enzymes SalI and NotI. The obtained DNA fragment was inserted to pFAST-BacHTc (Invitrogen) digested with restriction enzymes SalI and NotI to give expression plasmid ACC1/pFAST-BacHTc.
   Using the expression plasmid and BAC-TO-BAC Baculovirus Expression System (Invitrogen), virus stock BAC-ACC1 of recombinant Baculovirus was prepared.

### (2) Preparation of ACC1 protein

SF-9 cells (Invitrogen) were inoculated to a medium (1 L) for insect cells (Sf-900IISFM medium (Invitrogen) containing 10% fetal bovine serum (Trace), 50 mg/L Gentamicin (Invitrogen) and 0.1% Pluronic F-68 (Invitrogen)) at 1×10⁶ cells/mL, and cultured with shaking at 27°C, 100 rpm in a 2 L Erlenmeyer flask.
After 24 hr of the culture, recombinant Baculovirus BAC-ACC1 (10 mL) was added, and the cells were further cultured for 3 days. The culture medium was centrifuged at 1000xg for 5 min to give virus-infected cells. The cells were washed with phosphate buffered saline (Invitrogen), centrifuged under the same conditions, and cryopreserved at -80C.
The cryopreserved cells were thawed in ice and suspended in 100 mL of 25 mM HEPES buffer (pH 7.5) containing 10% Glycerol, 0.13 M NaCl, 1 mM EDTA, 25 mM Sodium β-Glycerophosphate and 1 mM Sodium Orthovanadate, and supplemented with Complete Protease Inhibitor (Nippon Boehringer Ingelheim Co., Ltd.). The obtained suspension was homogenized 3 times in a polytronhomogenizer (Kinematica) at 20,000 rpm, 30 sec. The obtained cell disruption solution was clarified by centrifugation at 185700g, 50 min, and filtered through a 0.45 µm filter. The filtrate was passed through a column packed with 12 mL of Ni-NTA Super Flow Gel (QUIAGEN) at a flow rate of about 5 mL/min. The column was washed with buffer A (50 mM HEPES (pH 7.5) containing 0.3 M NaCl), further washed with buffer A containing 20 mM Imidazole, and eluted with buffer A containing 100 mM Imidazole. The eluate was concentrated with Vivaspin 20 (Vivascience) with a molecular weight cut off of 30K. The obtained concentrate was dialyzed against Sephadex G-25 (Amersham Biosciences, 358 mL) equilibrated with 50 mM HEPES (pH 7.5) containing 10 mM MgCl₂, 2 mM Dithiothreitol, 10 mM Tripotassium Citrate and 0.3 M NaCl. The inner dialysate was concentrated with Vivaspin 20 (Vivascience) with a molecular weight cut off of 30K, and the concentrate was filtered through a 0.22 µm filter to give ACC1. The obtained ACC1 was cryopreserved at -80°C.

### (3) Measurement of ACC1 inhibitory activity

ACC1 (0.93 mg/ml) obtained in the above-mentioned (2) was diluted with an enzyme reaction buffer (50 mM HEPES (pH 7.5), 10 mM MgCl₂, 10 mM Tripottasium Citrate, 2 mM Dithiothreitol, 0.75 mg/ml Fatty acid free BSA) to concentration of 8 µg/ml, and added to each well of 384 well assay plate (Nunc 265196) by 10 µl.
Thereafter, ACC1 inhibitory rate (%) was measured in the same manner as in the below-mentioned Experimental Example 2-(3), and IC₅₀ value was calculated.
As a result, the compounds of Examples 4, 7 - 15, 17 and 19 - 27 showed IC₅₀ values of not more than 1 µM.

### Experimental Example 2

The ACC2 inhibitory action of the compound of the present invention was evaluated by the following method.
(1) Cloning of human ACC2 gene and preparation of recombinant Baculovirus
   Human ACC2 gene was cloned by PCR using a human skeletal muscle cDNA library (Clontech) as a template and Primer 1 and Primer 2 shown below. Primer 1 and Primer 2 were prepared by adding SalI, XbaI restriction enzyme recognition sequences based on the information of the base sequence (Genbank Accession U89344) of human ACC2 gene.
   Primer 1 5'AAAAGTCGACCCACCATGGTCTTGCTTCTTTGTCTATCTTG (SEQ ID NO:3)
   Primer 2 5'TTTTTCTAGATCAGGTAGAGGCCGGGCTGTCCATG (SEQ ID NO:4)
   PCR was performed using Pyrobest DNA polymerase (TAKARA BIO INC.). The obtained PCR product was cloned to pT7 Blue vector (Novagen) and, after confirmation of the base sequence, digested with restriction enzymes SalI and XbaI. The obtained DNA fragment was inserted into pFAST-BacHTa (Invitrogen) digested with restriction enzymes SalI and XbaI to give expression plasmid ACC2/pFAST-BacHTa.
   PCR was performed using the expression plasmid as a template and Primer 3 and Primer 4 shown below to prepare a plasmid to be used for expression of ACC2 free of mitochondrial targeting sequence.
   Primer 3 5'CCAGGTCGACCCGCCAACGGGACTGGGACACAAGG (SEQ ID NO:5)
   Primer 4 5'CGCACTCTCAGTTTCCCGGATTCCC (SEQ ID NO:6)
   PCR was performed using Pyrobest-DNA polymerase (TAKARA BIO INC.). The obtained PCR product was cloned to pT7 Blue vector (Novagen) and, after confirmation of the base sequence, digested with restriction enzymes SalI and AflII. The obtained DNA fragment was inserted into pFAST-BacHTa (Invitrogen) digested with restriction enzymes SalI and AflII to give expression plasmid ACC2mito7/pFAST-BacHTa.
   Using the expression plasmid and BAC-TO-BAC Baculovirus Expression System (Invitrogen), virus stock BAC-ACC2 (N terminal deleted (hereinafter Nd)) of recombinant Baculovirus was prepared.

### (2) Preparation of ACC2 (Nd) protein

SF-9 cells (Invitrogen) were inoculated to a medium (2 L) for insect cells (Sf-900IISFM medium (Invitrogen) containing 10% fetal bovine serum (Trace), 50 mg/L Gentamicin (Invitrogen), 0.1% Pluronic F-68 (Invitrogen)) at 0.5×10⁶ cells/mL, and cultured with shaking in Wave Bioreactor (Wave) at 27°C, 20 rpm, rocking angle 6°, oxygen concentration 30%.
On day 4 of the culture, 3 L of the medium for insect cells was added, the rocking angle was set to 8°, and the cells were cultured. On day 5 of the culture, 100 mL of recombinant Baculovirus BAC-ACC2 (Nd) was added, 5 L of the medium for insect cells was further added, the rocking angle was set to 11°, and the cells were cultured for 3 days. The culture medium was centrifuged at 1000×g for 10 min to give virus-infected cells. The cells were washed with phosphate buffered saline
(Invitrogen) and centrifuged under the same conditions. The obtained cells were cryopreserved at -80°C.
The cryopreserved cells were thawed in ice and suspended in 900 mL of 25 mM HEPES buffer (pH 7.5) containing 10% Glycerol, 0.13 M NaCl, 1 mM EDTA, 25 mM Sodium β-Glycerophosphate and 1 mM Sodium Orthovanadate, and supplemented with Complete Protease Inhibitor (Nippon Boehringer Ingelheim Co., Ltd.) The obtained suspension was homogenized 3 times in a polytron homogenizer (Kinematica) at 20,000 rpm, 30 sec. The obtained cell disruption solution was clarified by centrifugation at 31000×g, 60 min, and filtered through a 0.45 µm filter. The filtrate was passed through a column packed with 60 mL of Ni-NTA Super Flow Gel (QUIAGEN) at a flow rate of about 5 mL/min. The column was washed with buffer A (50 mM HEPES (pH 7.5) containing 0.3 M NaCl), further washed with buffer A containing 20 mM Imidazole, and eluted with buffer A containing 100 mM Imidazole. The eluate was concentrated with Vivaspin 20 (Vivascience) with a molecular weight cut off of 30K. The obtained concentrate was dialyzed against 50 mM HEPES (pH 7.5) containing 10 mM MgCl₂, 2 mM Dithiothreitol, 10 mM Tripotassium Citrate and 0.3 M NaCl. The inner dialysate was filtered through a 0.22 µm filter to give ACC2 (Nd). The obtained ACC2 (Nd) was cryopreserved at - 80°C.

### (3) Measurement of ACC2 inhibitory activity

ACC2 (Nd) (1.1 mg/ml) obtained in the above-mentioned (2) was diluted with an enzyme reaction buffer (50 mM HEPES (pH 7.5), 10 mM MgCl₂, 10 mM Tripottasium Citrate, 2 mM Dithiothreitol, 0.75 mg/ml Fatty acid free BSA) to a concentration of 6.4 µg/ml, and added to each well of a 384 well assay plate (Nunc 265196) by 10 µl. A test compound was dissolved in dimethyl sulfoxide (DMSO) and diluted with an enzyme reaction buffer and the resulting solution (5 µl) was added to each well. The mixture was incubated at 30°C for 60 min. Then, a substrate solution (50 mM KHCO₃, 200 uM ATP, 200 uM Acetyl-CoA, 5 µl) was added to each well, and the mixture was reacted at 30°C for 20 min (test compound addition group).
In addition, a reaction was performed in the same manner as above and without adding the test compound (test compound non-addition group). Furthermore, a reaction was performed in the same manner as above and without adding the test compound and Acetyl-CoA (control group).
The reaction was quenched by adding a malachite green solution to each of the obtained reaction mixtures by 5 µl and stirring the mixtures. The obtained reaction mixture was left standing at room temperature for 20 min, and absorbance (620 nm) was measured using wallac1420 (PerkinElmer Japan Co., Ltd.). The aforementioned malachite green solution was prepared by mixing Solution A (0.12% malachite green solution, prepared with 5N H₂SO₄, preserved at 4°C in shading), Solution B (7.5% aqueous ammoniummolybdate solution, prepared when in use) and Solution C (11% aqueous Tween 20 solution, preserved at room temperature) at a ratio of Solution A: Solution B: Solution C=100:25:2 (volume ratio).
Then, ACC2 inhibitory rate (%) was measured from the calculation formula:
(1-(absorbance of test compound addition group - absorbance of control group)÷(absorbance of test compound non-addition group - absorbance of control grnup))×100, and the IC₅₀ value was calculated.
   As a result, the compounds of Examples 4, 7 - 15, 17 and 19 - 27 showed IC₅₀ values of not more than 100 nM.

**Table 1**

| Formulation Example 1 (production of capsule) | | |
|---|---|---|
| 1) | compound of Example 1 | 30 mg |
| 2) | fine powdered cellulose | 10 mg |
| 3) | lactose | 19 mg |
| 4) | magnesium stearate | 1 mg |
| total | | 60 mg |

1), 2), 3) and 4) are mixed and filled in a gelatin capsule.

**Table 2**

| Formulation Example 2 (production of tablet) | | | |
|---|---|---|---|
| 1) | compound of Example 1 | | 30 g |
| 2) | lactose | | 50 g |
| 3) | cornstarch | | 15 g |
| 4) | calcium carboxymethylcellulose | | 44 g |
| 5) | magnesium stearate | | 1 g |
| | 1000 tablets | total | 140 g |

The total amount of 1), 2) and 3) and 4) (30 g) is kneaded with water, vacuum dried, and sieved.
The sieved powder is mixed with 4) (14 g) and 5) (1 g), and punched by a tableting machine, whereby 1000 tablets containing 30 mg of the compound of Example per tablet are obtained.

### Industrial Applicability

The compound of the present invention has ACC (an acetyl-CoA carboxylase) inhibitory action, and is useful for the prophylaxis or treatment of obesity, diabetes, hypertension, hyperlipidemia, cardiac failure, diabetic complications, metabolic syndrome, sarcopenia and the like.
This application is based on patent application No. 112769/2006 filed in Japan, the contents of which are hereby incorporated by reference. The contents disclosed in any publication cited in the present specification, including patents and patent applications, are hereby incorporated in their entireties by reference, to the extent that they have been disclosed herein.

## Claims

1. A compound represented by the following formula 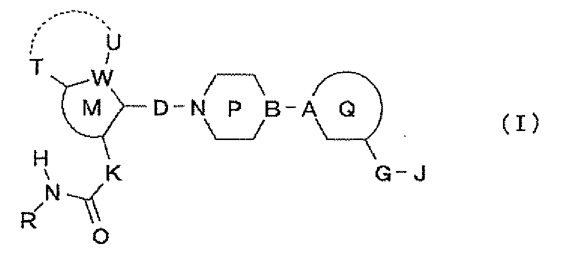 wherein
ring M is a 5- or 6-membered aromatic ring;
W is C or N;
K is an optionally substituted methylene group or an optionally substituted imino group;
R is a hydrogen atom, an optionally substituted hydrocarbon group, an optionally substituted hydroxy group or an optionally substituted heterocyclic group;
T and U are independently a hydrogen atom or a substituent or, T and U form, together with ring M, an optionally substituted bicyclic ring;
D and G are independently a carbonyl group or a sulfonyl group; ring P is an optionally substituted piperidine or an optionally substituted piperazine;
B is CH or N;
ring Q is an optionally substituted monocyclic ring;
A is C, CH or N; and
J is an optionally substituted hydrocarbon group, an optionally substituted heterocyclic group or an optionally substituted amino group,
provided that when the W moiety of ring M is =N- or -N=, then U should be absent,
or a salt thereof.

2. The compound of claim 1, wherein ring M is thiophene, thiazole or pyridine.

3. The compound of claim 1, wherein K is an imino group.

4. The compound of claim 1, wherein R is a hydrogen atom or an optionally substituted hydrocarbon group.

5. The compound of claim 1, wherein T is an optionally substituted phenyl group, and U is a hydrogen atom.

6. The compound of claim 1, wherein T and U form, together with ring M, an optionally substituted bicyclic ring.

7. The compound of claim 1, wherein D is a carbonyl group.

8. The compound of claim 1, wherein G is a carbonyl group.

9. The compound of claim 1, wherein ring P is piperidine.

10. The compound of claim 1, wherein ring Q is piperidine, piperazine, morpholine or benzene, each of which is optionally substituted.

11. The compound of claim 1, wherein A is C or N.

12. The compound of claim 1, wherein J is an optionally substituted heterocyclic group or an optionally substituted amino group.

13. The compound of claim 1, which is N,N-diethyl-1'-[(2-{[(ethylamino)carbonyl]amino}-1-benzothiophen-3-yl)carbonyl]-1,4'-bipiperidine-3-carboxamide;
N-ethyl-N'-(4-{[3-(morpholin-4-ylcarbonyl)-1,4'-bipiperidin-1'-yl]carbonyl}-2-phenyl-1,3-thiazol-5-yl)urea;
N-ethyl-N'-{3-{[(3R)-3-(morpholin-4-ylcarbonyl)-1,4'-bipiperidin-1'-yl]carbonyl}-5-[4-(trifluoromethyl)phenyl]-2-thienyl}urea;
N-ethyl-N'-(7-methoxy-3-{[3-(piperidin-1-ylcarbonyl)-1,4'-bipiperidin-1'-yl]carbonyl}-1-benzothien-2-yl)urea; or
N-ethyl-1'-[(2-{[(ethylamino)carbonyl]amino}-6-methylthieno[2,3-b]pyridin-3-yl)carbonyl]-N-isopropyl-1,4'-bipiperidine-3-carboxamide;
or a salt thereof.

14. A prodrug of the compound of claim 1.

15. An acetyl-CoA carboxylase) inhibitor comprising the compound of claim 1 or a prodrug thereof.

16. A pharmaceutical agent comprising the compound of claim 1 or a prodrug thereof.

17. The pharmaceutical agent of claim 16, which is an agent for the prophylaxis or treatment of obesity, diabetes, hypertension, hyperlipidemia, cardiac failure, diabetic complications, metabolic syndrome or sarcopenia.

18. Use of the compound of claim 1 or a prodrug thereof for the production of an acetyl-CoA carboxylase inhibitor.

19. Use of the compound of claim 1 or a prodrug thereof for the production of an agent for the prophylaxis or treatment of obesity, diabetes, hypertension, hyperlipidemia, cardiac failure, diabetic complications, metabolic syndrome or sarcopenia.

20. A method of inhibiting acetyl-CoA carboxylase in a mammal, which comprises administering the compound of claim 1 or a prodrug thereof to the mammal.

21. A method for the prophylaxis or treatment of obesity, diabetes, hypertension, hyperlipidemia, cardiac failure, diabetic complications, metabolic syndrome or sarcopenia in a mammal, which comprises administering the compound of claim 1 or a prodrug thereof to the mammal.
